# EUROPEAN PATENT APPLICATION

(11) **EP 1 950 211 A1**
(43) Date of publication of application: **30.07.2008**
(21) Application number: 06832861.6
(22) Date of filing: 17.11.2006
(51) Int. Cl.: C07D 401/10, A61K 31/4439, A61P 25/28, A61P 43/00, C07D 211/76, C07D 211/78, C07D 233/61

(54) **PROCESS FOR PRODUCTION OF CINNAMAMIDE DERIVATIVE**

(30) Priority: 18.11.2005 JP 2005334286
(71) Applicant: Eisai R&D Management Co., Ltd., Tokyo 112-8088 (JP)
(72) Inventor: SHIMOMURA, Naoyuki, Tsukuba-shi Ibaraki 300-2635 (JP); SATO, Nobuaki, Tsukuba-shi Ibaraki 300-2635 (JP); KANEKO, Toshihiko, Tsukuba-shi Ibaraki 300-2635 (JP); TAKAISHI, Mamoru, Tsukuba-shi Ibaraki 300-2635 (JP); WAKASUGI, Kazunori, Tsukuba-shi Ibaraki 300-2635 (JP); YOSHIKAWA, Seiji, Kamisu-shi Ibaraki 314-0255 (JP); NISHIKAWA, Yoshihiro, Kamisu-shi Ibaraki 314-0255 (JP); NAKAMURA, Taiju, misu-shi Ibaraki 314-0255 (JP); FUKUYAMA, Tohru, Tokyo 113-0033 (JP)
(74) Representative: HOFFMANN EITLE
(86) International application number: PCT/JP2006/322982
(87) International publication number: WO 2007/058305

(57) **Abstract**

A compound (8) represented by the formula: (8) 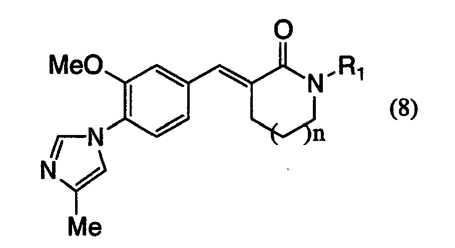 wherein R₁ represents a 6- to 14-membered aromatic hydrocarbon ring group which may have a substituent; and n represents 0 to 2, can be produced with good efficiency by reacting a compound (3) represented by the formula: (3) 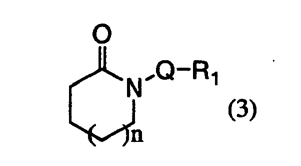 wherein R₁ and n are as defined above; and Q represents a single bond or -CH(Y)- where Y represents a hydrogen atom or a C1-6 alkyl group] with 3-methoxy-4-(4-methyl-1H-imidazol-1-yl)benzaldehyde in the presence of a base.

## Description

### TECHNICAL FIELD

The present invention relates to a process for producing a cinnamide derivative not yet described in any document, in particular, (3*E*)-1-[(1*S*)-1-(4-fluorophenyl)ethyl]-3-[3-methoxy-4-(4-methyl-1*H-*imidazol-1-yl)benzylidene]piperidin-2-one having an amyloid-β production reducing effect or the like and useful as a progression inhibitor or prophylactic for a disease involving amyloid-β such as Alzheimer's disease, and to a synthetic intermediate thereof.

### BACKGROUND ART

Alzheimer's disease is a disease characterized by degeneration and loss of neurons as well as formation of senile plaques and neurofibrillary degeneration. Currently, Alzheimer's disease is treated only with symptomatic treatment using a symptom improving agent typified by an acetylcholinesterase inhibitor, and a fundamental remedy to inhibit progression of the disease has not yet been developed. It is necessary to develop a method for controlling the cause of the onset of pathology in order to create a fundamental remedy for Alzheimer's disease.
It is assumed that Aβ-proteins as metabolites of amyloid precursor proteins (hereinafter referred to as APP) are highly involved in degeneration and loss of neurons and onset of symptoms of dementia (see Non-Patent Documents 1 and 2, for example). Accordingly, a compound that reduces production of Aβ40 and Aβ42 has been expected as a progression inhibitor or prophylactic agent for Alzheimer's disease.
[Non-Patent Document 1] Klein WL, and seven others, Alzheimer's disease-affected brain: Presence of oligomeric Aβ ligands (ADDLs) suggests a molecular basis for reversible memory loss, Proceding National Academy of Science USA 2003, Sep 2; 100(18), p.10417-10422.
[Non-Patent Document 2] Nitsch RM, and 16 others, Antibodies against β-amyloid slow cognitive decline in Alzheimer's disease, Neuron, 2003, May 22; 38, p.547-554.

### DISCLOSURE OF THE INVENTION

The present inventors have found that compounds typified by (3*E*)-1-[(1*S*)-1-(4-fluorophenyl)ethyl]-3-[3-methoxy-4-(4-methyl-1H-imidazol-1-yl)benzylidene]piperidin-2-one represented by the structural formula (I): 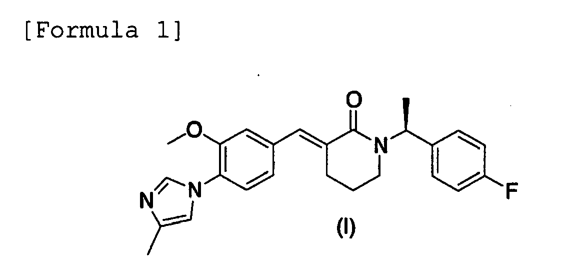 among cinnamide derivatives have an effect of reducing production of Aβ40 and 42 (PCT/JP2005/009537).
However, compounds having a cyclic amide moiety with a cyclic structure such as a pyrrolidone ring or piperidone ring among cinnamide derivatives typified by the structural formula (I) have not yet been known, and accordingly processes for producing such compounds have not been known.
An object of the present invention is to provide a process for producing a cinnamide derivative having an amyloid-β production reducing effect, in particular, (3*E*)-1-[(1*S*)-1-(4-fluorophenyl)ethyl]-3-[3-methoxy-4-(4-methyl-1H-imidazol-1-yl)benzylidene]piperidin-2-one, and a process for producing a synthetic intermediate thereof.

As a result of extensive studies to solve the above problem, the present inventors have discovered the following production processes and synthetic intermediates used for the processes. This has led to the completion of the present invention.
Specifically, the present invention relates to:
[1]. A process for producing a compound (8) represented by the formula: 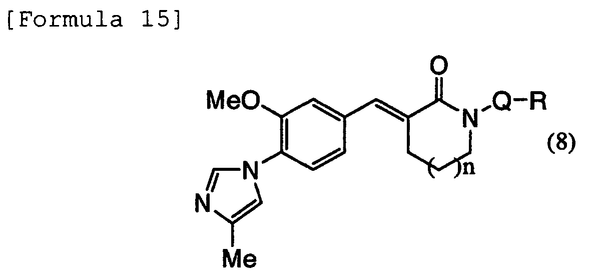 wherein Q represents a single bond or -CH(Y)- (wherein Y represents a hydrogen atom or a C1-6 alkyl group), R represents a 6- to 14-membered aromatic hydrocarbon ring group which may have a substituent or a 5- to 14-membered aromatic heterocyclic group which may have a substituent and n represents 0 to 2, the process comprising the steps of:
   reacting, in the presence of a base, a compound (1) represented by the formula (1):

   [Formula 2] R₁-Q-NH₂ (1)

   wherein Q is as defined above and R₁ represents a 6- to 14-membered aromatic hydrocarbon ring group which may have a substituent (wherein the substituent may have a protecting group) or a 5- to 14-membered aromatic heterocyclic group which may have a substituent (wherein the substituent may have a protecting group), with a compound (2) represented by the formula (2):

   [Formula 3] X₁ - (CH₂)₂- (CH₂)ₙ-CH₂-CO-X₂ (2)

   wherein X₁ and X₂ are the same or different and each represent a halogen atom and n is as defined above, to convert the compound (1) into a compound (3) represented by the formula (3): 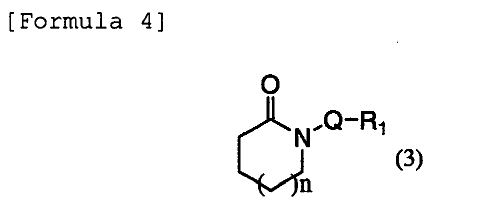 wherein Q, R₁ and n are as defined above;
   treating the compound (3) represented by the formula (3): 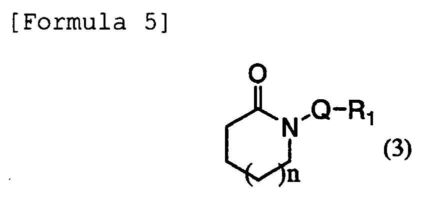 wherein Q, R₁ and n are as defined above, with a base and then reacting the treated compound with a halogenating reagent, or reacting the compound (3) with a halogenating reagent in the presence of a base to convert the compound (3) into a compound (4) represented by the formula (4): 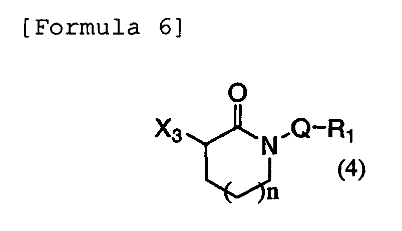 wherein X₃ represents a halogen atom and Q, R₁ and n are as defined above;
   reacting the compound (4) represented by the formula (4): 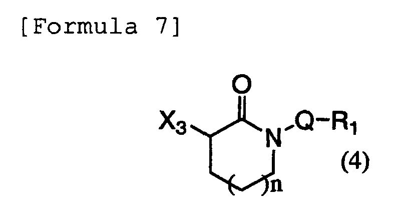 wherein Q, R₁, X₃ and n are as defined above, with a phosphorous acid compound (5-a) represented by the formula (5-a):

   [Formula 8] P (OR₂ₐ) ₃ (5-a)

   wherein R₂ₐ represents a C1-6 alkyl group which may have a substituent or a phenyl group which may have a substituent, or a phosphorus compound (5-b) represented by the formula (5-b):

   [Formula 9] P (R_{2b}) ₃ (5-b)

   wherein R_{2b} represents a C1-6 alkyl group which may have a substituent or a phenyl group which may have a substituent, to respectively convert the compound (4) into a compound (6-a) represented by the formula (6-a): 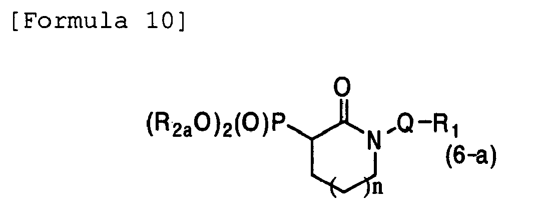 wherein Q, R₁, R₂ₐ and n are as defined above, or a compound (6-b) represented by the formula (6-b): 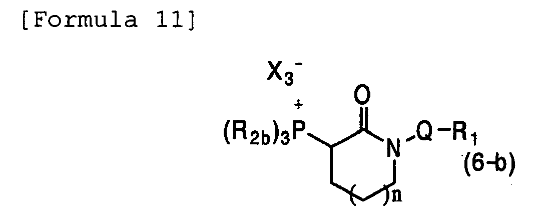 wherein Q, R₁, R_{2b}, X₃ and n are as defined above; and reacting, in the presence of a base, the compound (6-a) represented by the formula (6-a): 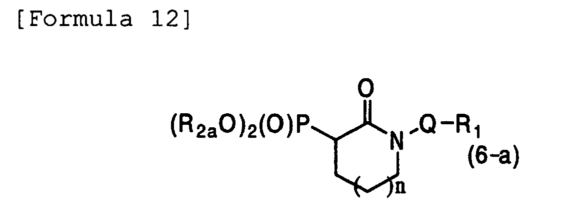 wherein Q, R₁, R₂ₐ and n are as defined above, or the compound (6-b) represented by the formula (6-b): 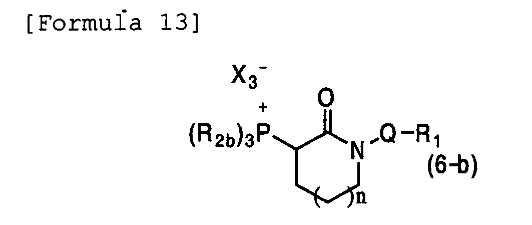 wherein Q, R₁, R_{2b}, X₃ and n are as defined above, with 3-methoxy-4-(4-methyl-1H-imidazol-1-yl)benzaldehyde represented by the formula (7): 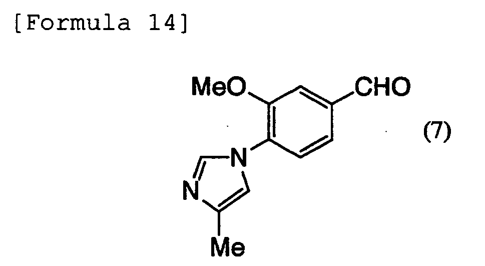 and removing a protecting group when R₁ has the protecting group;
[2]. The process for producing a compound (8) according to [1] above, comprising the step of reacting a compound (4) with a phosphorous acid compound (5-a) to convert the compound (4) into a compound (6-a);
[3]. A process for producing a compound (8) represented by the formula (8): 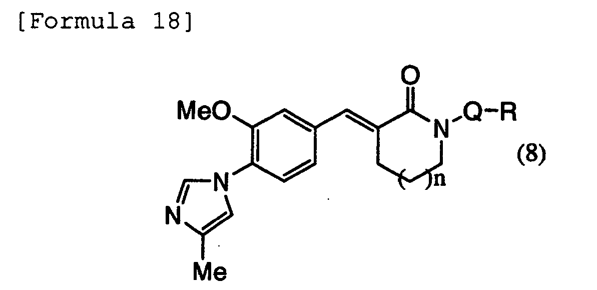 wherein Q, R and n are as defined in [1] above, the process comprising the steps of:
   reacting, in the presence of a base, a compound (6-a) represented by the formula (6-a): 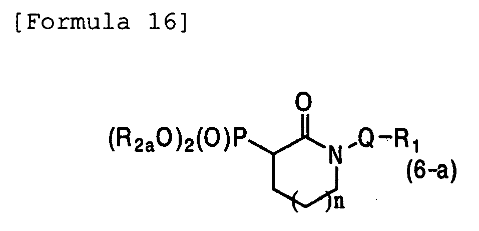 wherein R₁ and R₂ₐ are as defined in [1] above and Q and n are as defined above, with 3-methoxy-4-(4-methyl-1*H-*imidazol-1-yl)benzaldehyde represented by the formula (7) : 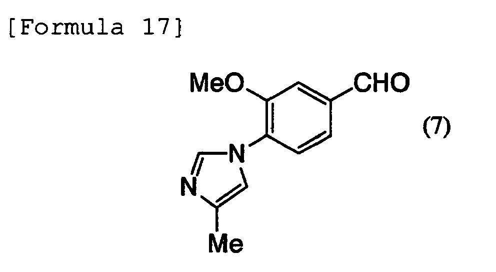 and removing a protecting group when R₁ has the protecting group;
[4]. A process for producing a compound (6-a) represented by the formula (6-a): 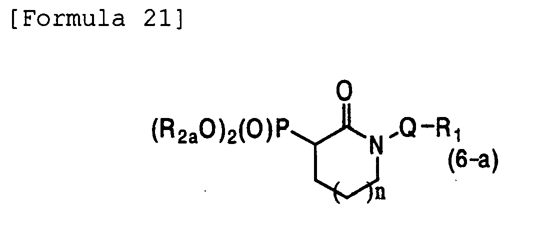 wherein Q, R₁, R₂ₐ and n are as defined in [1] above, the process comprising:
   reacting a compound (4) represented by the formula (4): 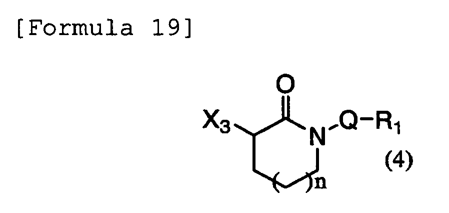 wherein X₃ is as defined in [1] above and Q, R₁ and n are as defined above, with a phosphorous acid compound (5-a) represented by the formula (5-a):

   [Formula 20] P (OR₂ₐ)₃ (5-a)

   wherein R₂ₐ is as defined above;
[5]. A process for producing a compound (4) represented by the formula (4): 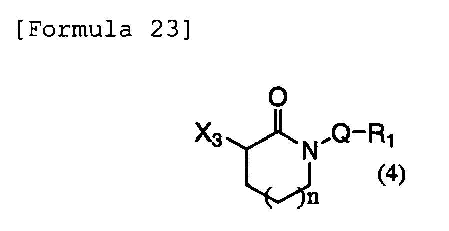 wherein Q, R₁, X₃ and n are as defined in [1] above, the process comprising:
   treating a compound (3) represented by the formula (3): 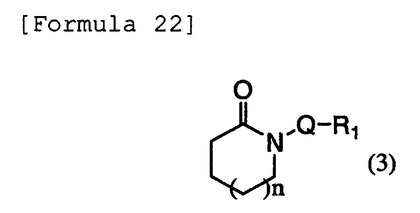 wherein Q, R₁ and n are as defined above, with a base and reacting the treated compound with a halogenating reagent;
[6]. A process for producing a compound (3) represented by the formula (3): 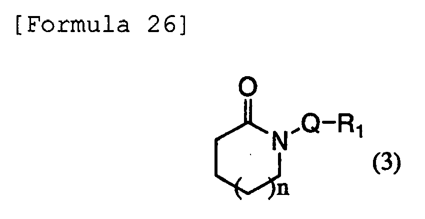 wherein Q represents a single bond or -CH(Y)- (wherein Y represents a hydrogen atom or a C1-6 alkyl group), R₁ represents a 6- to 14-membered aromatic hydrocarbon ring group which may have a substituent (wherein the substituent may have a protecting group) or a 5- to 14-membered aromatic heterocyclic group which may have a substituent (wherein the substituent may have a protecting group) and n represents 0 to 2, the process comprising:
   reacting, in the presence of a base, a compound (1) represented by the formula (1):

   [Formula 24] R₁-Q-NH₂ (1)

   wherein Q and R₁ are as defined above, with a compound (2) represented by the formula (2):

   [Formula 25] X₁- (CH₂) ₂- (CH₂) ₙ-CH₂-CO-X₂ (2)

   wherein X₁ and X₂ are the same or different and each represent a halogen atom and n is as defined above;
[7]. A compound (15) represented by the formula (15) : 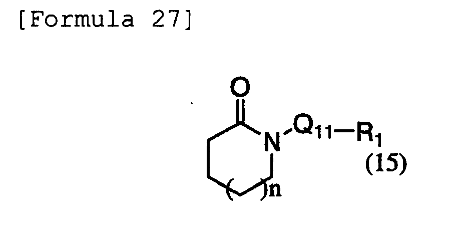 wherein Q₁₁ represents -CH (Y₁₁) - (wherein Y₁₁ represents a C1-6 alkyl group) and R₁ and n are as defined in [1] above, or a salt thereof, or a hydrate thereof;
[8]. A compound (16) represented by the formula (16): 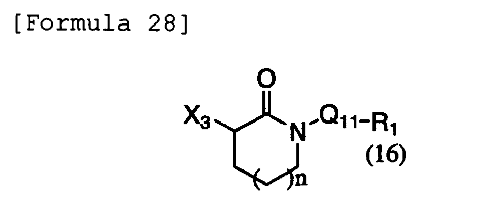 wherein R₁, X₃ and n are as defined in [1] above and Q₁₁ is as defined in [7] above, or a salt thereof, or a hydrate thereof;
[9]. A compound (17) represented by the formula (17): 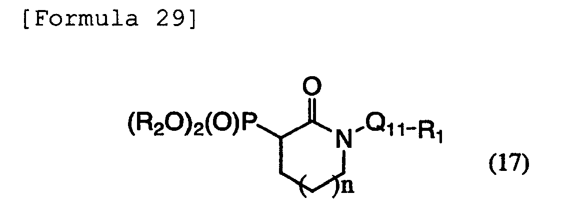 wherein R₁, R₂ and n are as defined in [1] above and Q₁₁ is as defined in [7] above, or a salt thereof, or a hydrate thereof;
[10]. A process for producing a compound (8) represented by the formula (8): 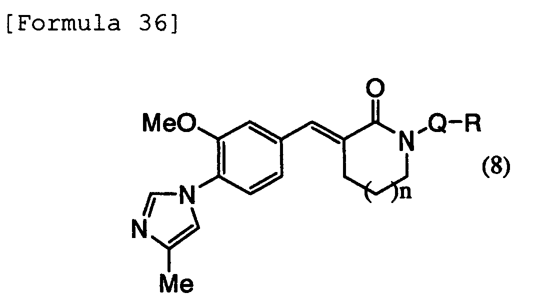 wherein Q, R and n are as defined in [1] above, the process comprising the steps of:
   reacting, in the presence of a base, a compound (3) represented by the formula (3): 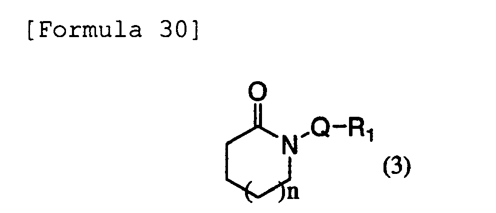 wherein R₁ is as defined in [1] above and Q and n are as defined above, with 3-methoxy-4-(4-methyl-1H-imidazol-1-yl)benzaldehyde represented by the formula (7): 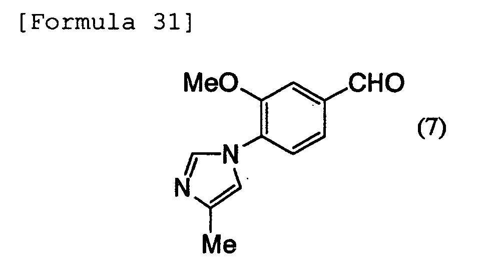 to convert the compound (3) into a compound (9) represented by the formula (9): 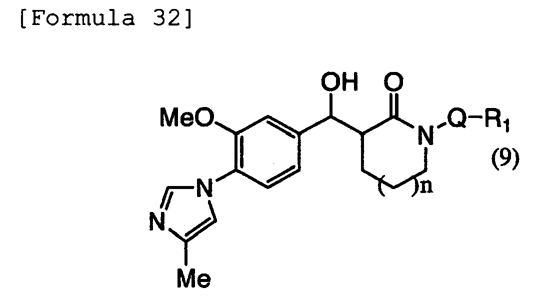 wherein Q, R₁ and n are as defined above;
   reacting the compound (9) represented by the formula (9): 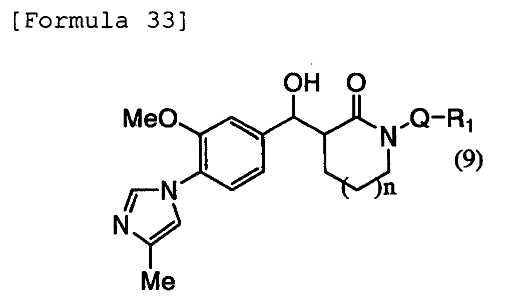 wherein Q, R₁ and n are as defined above, with a leaving group introduction reagent in the presence of a base if necessary to convert the compound (9) into a compound (10) represented by the formula (10): 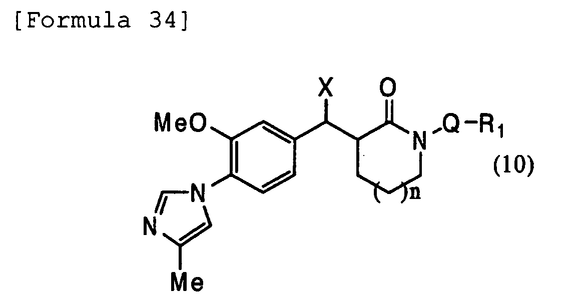 wherein X represents a leaving group and Q, R₁ and n are as defined above; and
   treating the compound (10) represented by the formula (10): 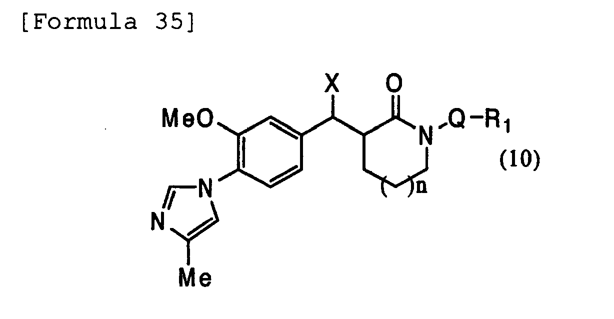 wherein Q, R₁, X and n are as defined above, with a base and removing a protecting group when R₁ has the protecting group;
[11]. The process for producing a compound (8) according to [10] above, comprising the steps of:
   reacting a compound (9) with a halogenating reagent or a sulfonylating reagent in the presence of a base if necessary to convert the compound (9) into a compound (10) represented by the formula (10-a): 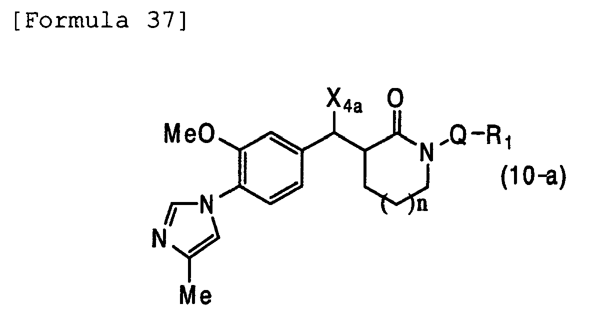 wherein X₄ₐ represents a halogen atom or a sulfonyloxy group and Q, R₁ and n are as defined above; and
   treating the compound represented by the formula (10-a): 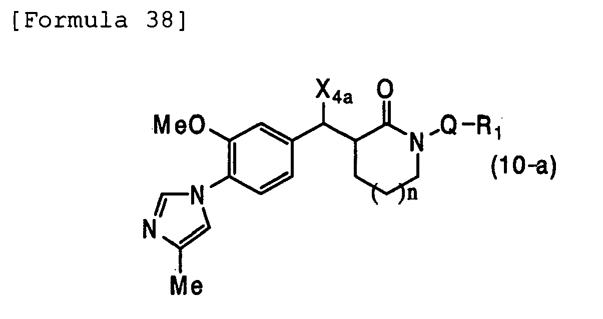 wherein Q, R₁, X₄ₐ and n are as defined above, with a base and removing a protecting group when R₁ has the protecting group;
[12]. A process for producing a compound (8) represented by the formula (8): 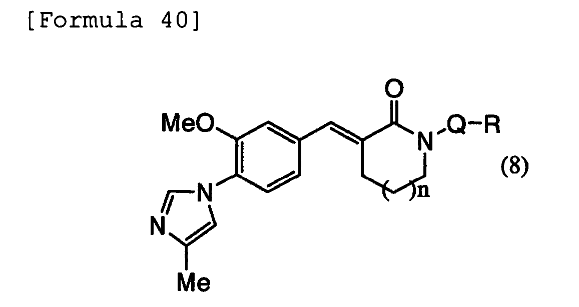 wherein Q, R and n are as defined in [1] above, the process comprising the steps of:
   treating a compound represented by the formula (10-a): 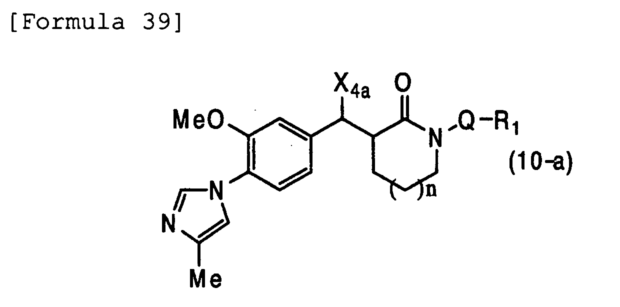 wherein R₁ is as defined in [1] above, X₄ₐ is as defined in [11] above and Q and n are as defined above, with a base and removing a protecting group when R₁ has the protecting group;
[13]. A process for producing a compound (10) represented by the formula (10-a): 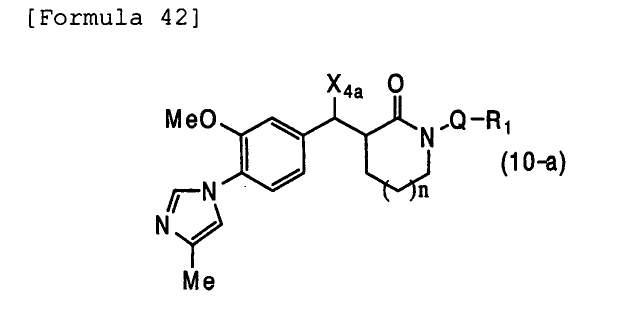 wherein Q, R₁ and n are as defined in [1] above and X₄ₐ is as defined in [11] above, the process comprising:
   reacting a compound (9) represented by the formula (9): 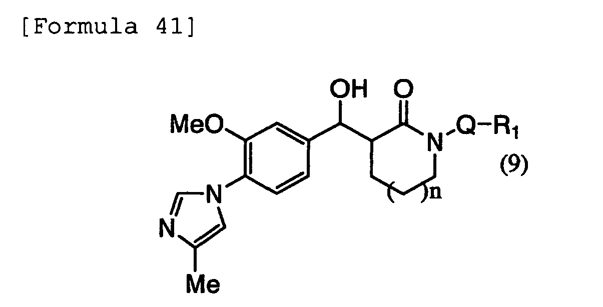 wherein Q, R₁ and n are as defined in [1] above, with a halogenating reagent or a sulfonylating reagent in the presence of a base if necessary;
[14]. A process for producing a compound (9) represented by the formula (9): 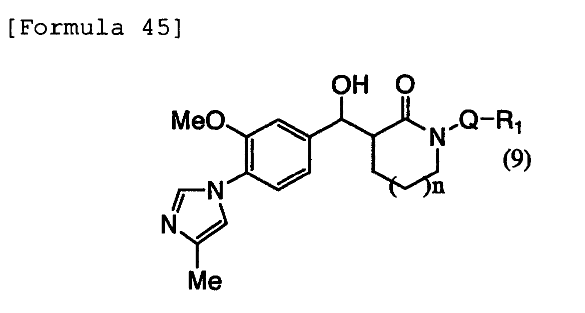 wherein Q, R₁ and n are as defined in [1] above, the process comprising:
   reacting, in the presence of a base, a compound (3) represented by the formula (3): 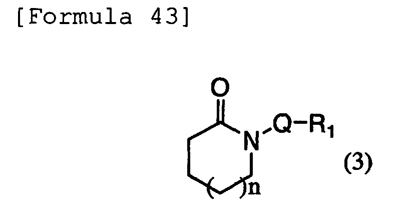 wherein Q, R₁ and n are as defined above, with 3-methoxy-4-(4-methyl-1H-imidazol-1-yl)benzaldehyde represented by the formula (7): 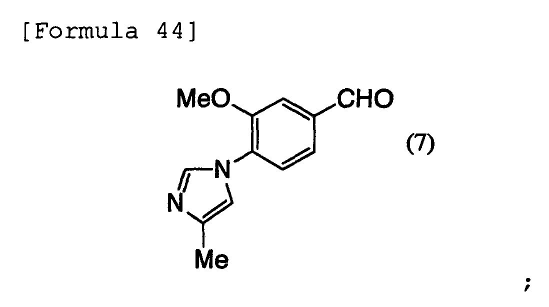
[15]. The process for producing a compound (8) according to [10] above, comprising the steps of:
   reacting a compound (9) with an acylating reagent in the presence of a base if necessary to convert the compound (9) into a compound (10-b) represented by the formula (10-b): 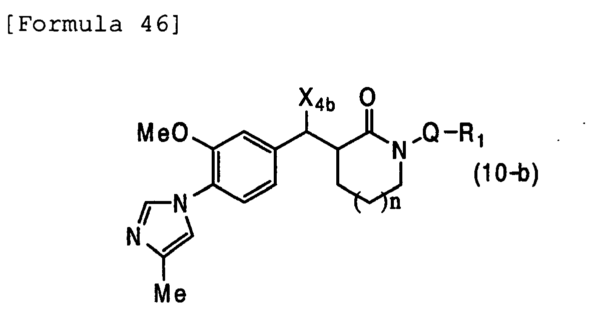 wherein X_{4b} represents an acyloxy group and Q, R₁ and n are as defined above; and
   treating the compound represented by the formula (10-b): 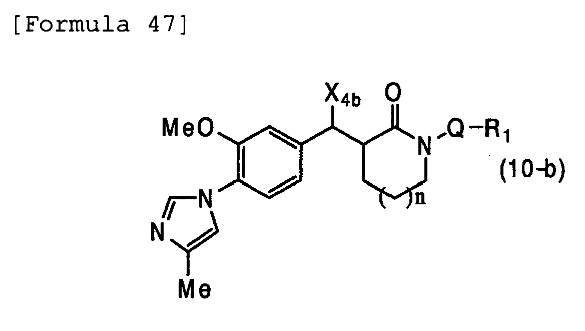 wherein Q, R₁, X_{4b} and n are as defined above, with a base and removing a protecting group when R₁ has the protecting group;
[16]. A process for producing a compound (8) represented by the formula (8): 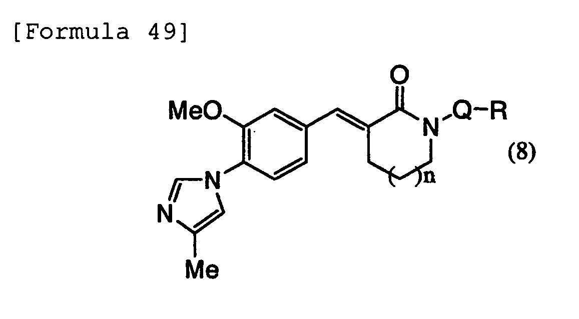 wherein Q, R and n are as defined in [1] above, the process comprising the steps of:
   treating a compound represented by the formula (10-b): 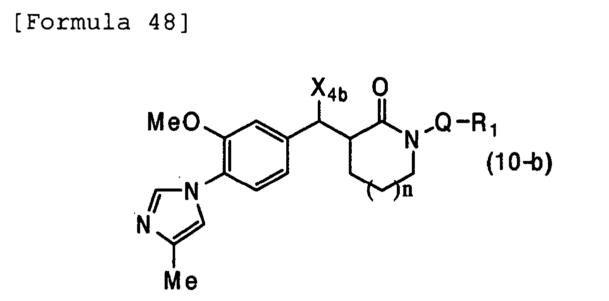 wherein R₁ is as defined in [1] above, X_{4b} is as defined in [16] above and Q and n are as defined above, with a base and removing a protecting group when R₁ has the protecting group;
[17]. A process for producing a compound (10-b) represented by the formula (10-b): 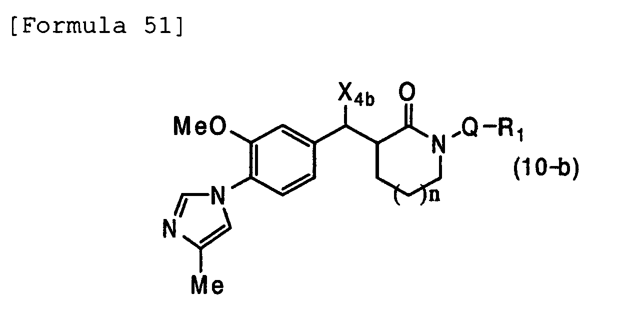 wherein Q, R₁ and n are as defined in [1] above and X_{4b} is as defined in [16] above, the process comprising:
   reacting a compound (9) represented by the formula (9): 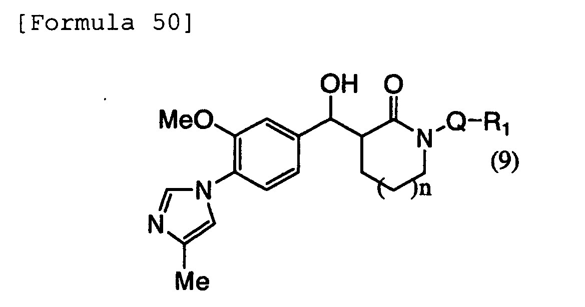 wherein Q, R₁ and n are as defined in [1] above, with an acylating reagent in the presence of a base if necessary;
[18]. A compound (9) represented by the formula (9) : 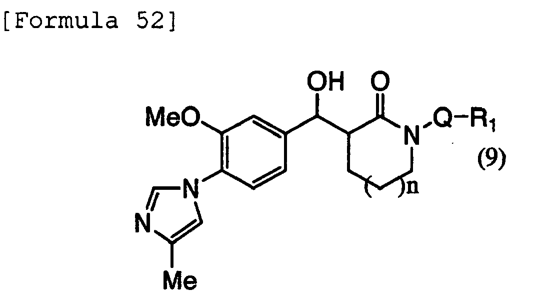 wherein Q, R₁ and n are as defined in [1] above, or a salt thereof, or a hydrate thereof;
[19]. A compound (10-a) represented by the formula (10-a): 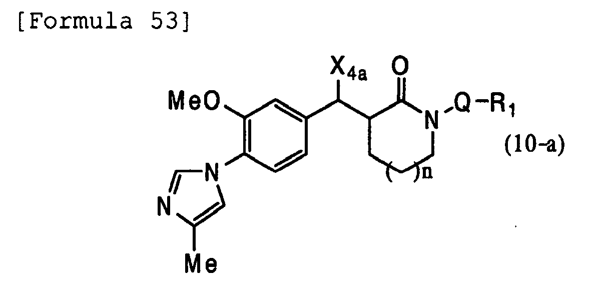 wherein Q, R₁ and n are as defined in [1] above and X₄ₐ is as defined in [11] above, or a salt thereof, or a hydrate thereof;
[20]. A compound (10-b) represented by the formula (10-b) : 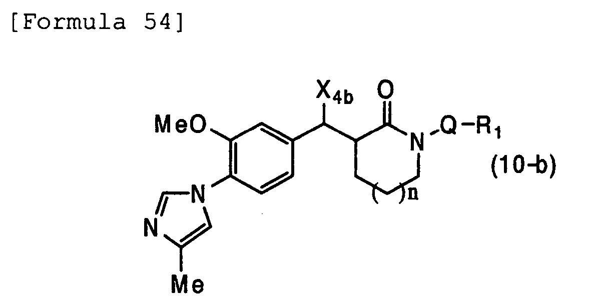 wherein Q, R₁ and n are as defined in [1] above and X_{4b} is as defined in [16] above, or a salt thereof, or a hydrate thereof;
[21]. A process for producing a compound (8) represented by the formula (8): 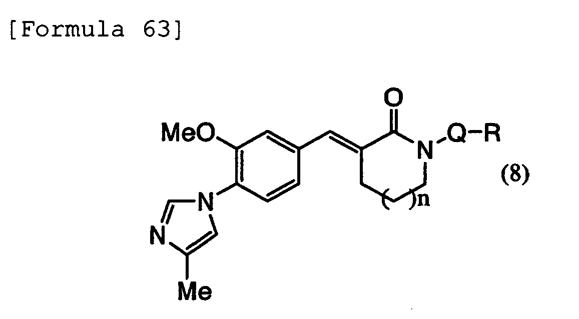 wherein Q, R and n are as defined in [1] above, the process comprising the steps of:
   reacting, in the presence of a base, a compound (3) represented by the formula (3): 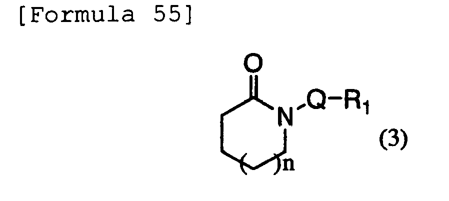 wherein R₁ is as defined in [1] above and Q and n are as defined above, with a compound (20) represented by the formula (20): 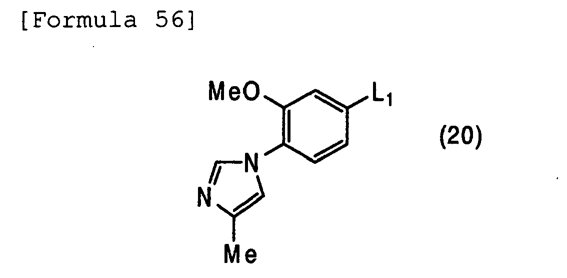 wherein L₁ represents an ester group or -CO-NR₂₀ₐ(-OR₂₀ₐ) (wherein R₂₀ₐ represents a C1-6 alkyl group), to convert the compound (3) into a compound (21) represented by the formula (21): 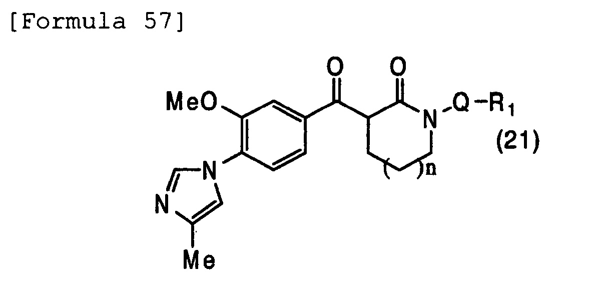 wherein Q, R₁ and n are as defined above;
   treating the compound (21) represented by the formula (21): 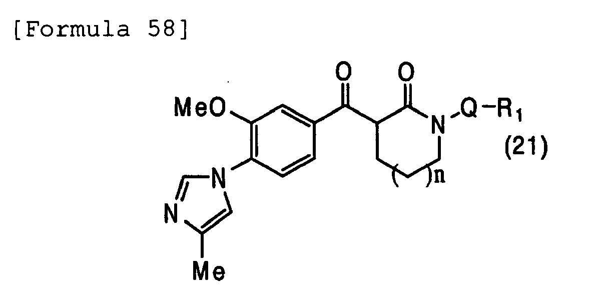 wherein Q, R₁ and n are as defined above, with a reducing agent to convert the compound (21) into a compound (9) represented by the formula (9): 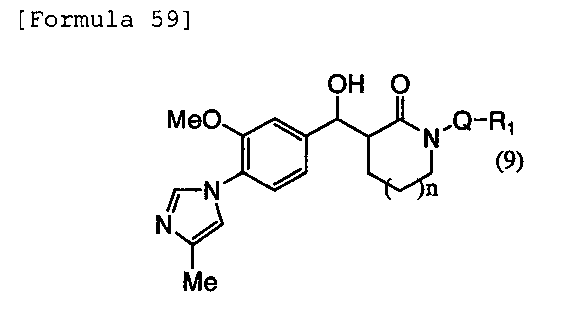 wherein Q, R₁ and n are as defined above;
   reacting the compound (9) represented by the formula (9): 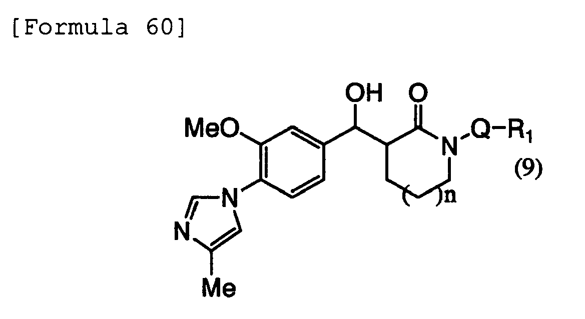 wherein Q, R₁ and n are as defined above, with a leaving group introduction reagent in the presence of a base if necessary to convert the compound (9) into a compound (10) represented by the formula (10): 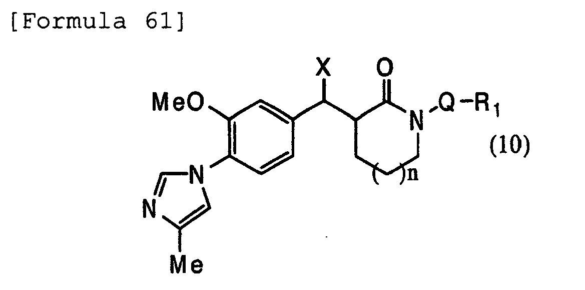 wherein X represents a leaving group and Q, R₁ and n are as defined above; and
   treating the compound represented by the formula (10): 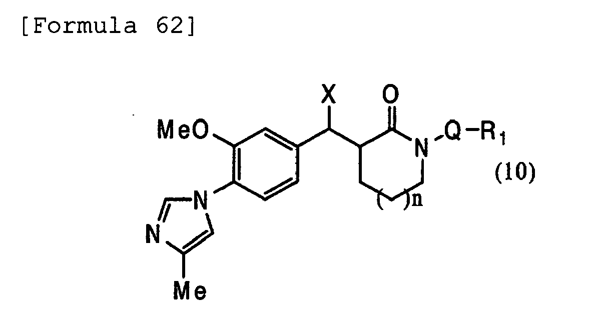 wherein Q, R₁, X and n are as defined above, with a base and removing a protecting group when R₁ has the protecting group;
[22]. A compound (21) represented by the formula (21): 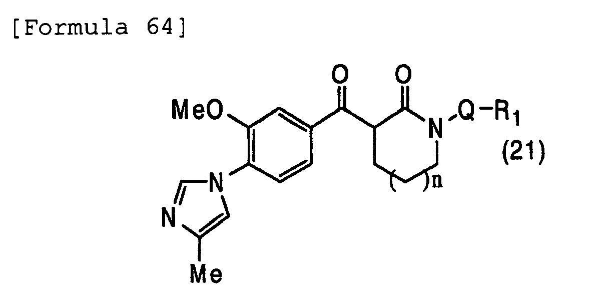 wherein Q, R₁ and n are as defined above, or a salt thereof, or a hydrate thereof;
[23]. A compound (20-a) represented by the formula (20-a): 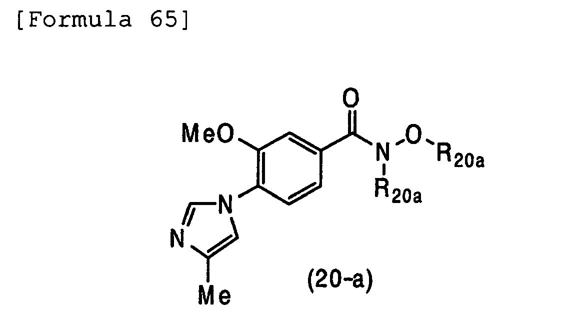 wherein R₂₀ₐ represents a C1-6 alkyl group, or a salt thereof, or a hydrate thereof;
[24]. A process for producing a compound (8) represented by the formula (8): 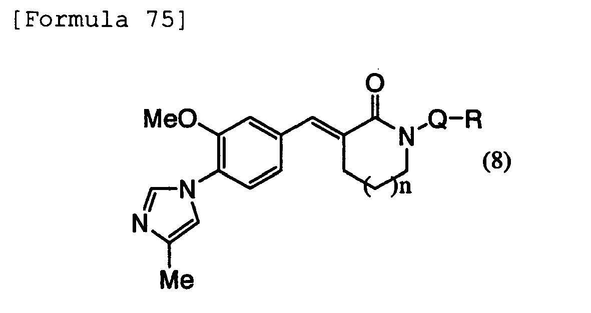 wherein Q, R and n are as defined in [1] above, the process comprising the steps of:
   reacting, in the presence of a reducing agent, a compound (11) represented by the formula (11): 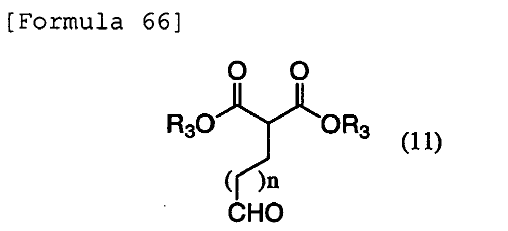 wherein R₃ is a protecting group for a carboxyl group and n is as defined above, with a compound (1) represented by the formula (1):

   [Formula 67] R₁-Q-NH₂ (1)

   wherein R₁ is as defined in [1] above and Q is as defined above, to convert the compound (11) into a compound (12) represented by the formula (12): 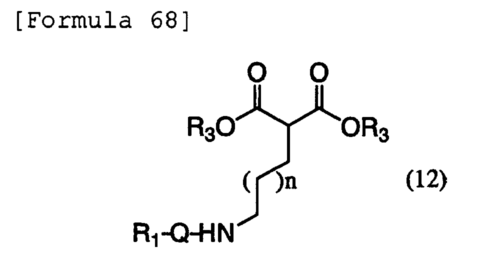 wherein Q, R₁, R₃ and n are as defined above;
   heating, in the coexistence of an acid, the compound (12) represented by the formula (12): 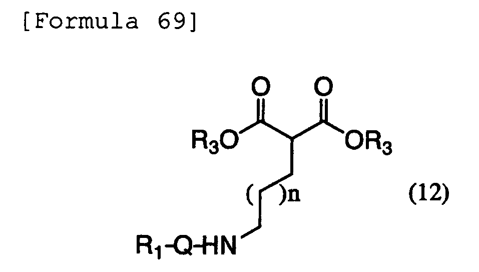 wherein Q, R₁, R₃ and n are as defined above, to convert the compound (12) into a compound (13) represented by the formula (13): 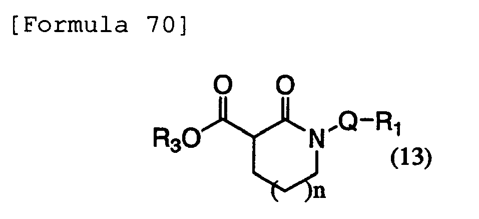 wherein Q, R₁, R₃ and n are as defined above;
   hydrolyzing, in the presence of a base, the compound (13) represented by the formula (13): 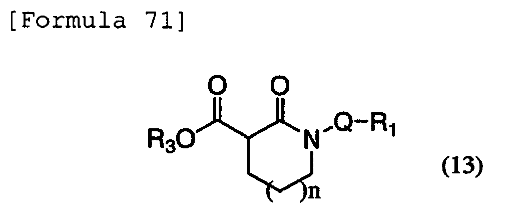 wherein Q, R₁, R₃ and n are as defined above, to convert the compound (13) into a compound (14) represented by the formula (14): 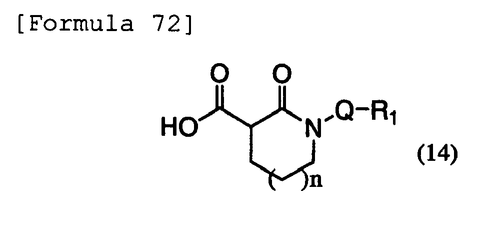 wherein Q, R₁ and n are as defined above; and reacting, in the presence of a base, the compound (14) represented by the formula (14): 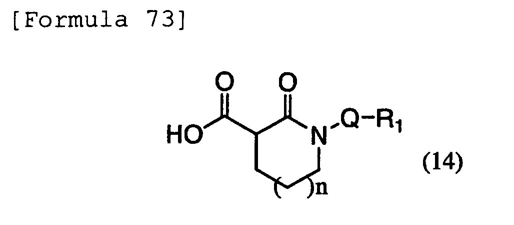 wherein Q, R₁ and n are as defined above, with 3-methoxy-4-(4-methyl-1H-imidazol-1-yl)benzaldehyde represented by the formula (7): 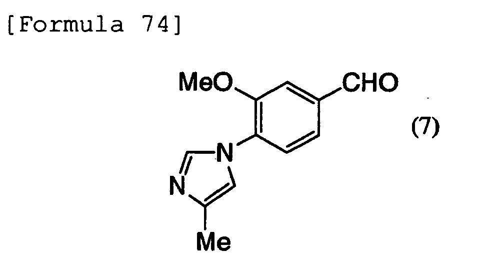 and removing a protecting group when R₁ has the protecting group;
[25]. A process for producing a compound (8) represented by the formula (8): 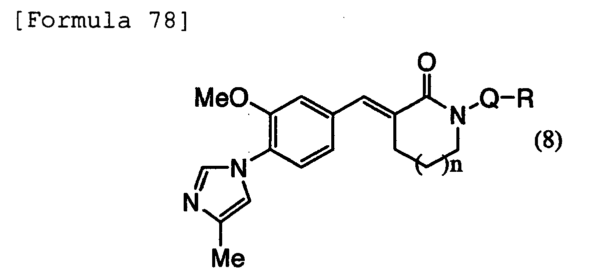 wherein Q, R and n are as defined in [1] above, the process comprising the steps of:
   reacting, in the presence of a base, a compound (14) represented by the formula (14): 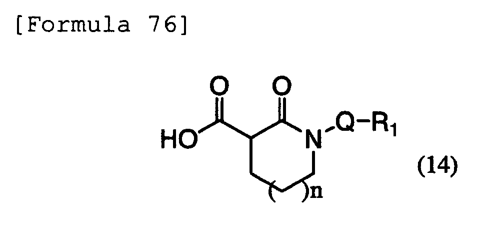 wherein R₁ is as defined in [1] above and Q and n are as defined above, with 3-methoxy-4-(4-methyl-1H-imidazol-1-yl)benzaldehyde represented by the formula (7): 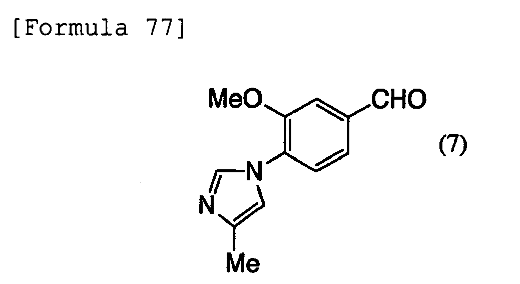 and removing a protecting group when R₁ has the protecting group;
[26]. A process for producing a compound (14) represented by the formula (14): 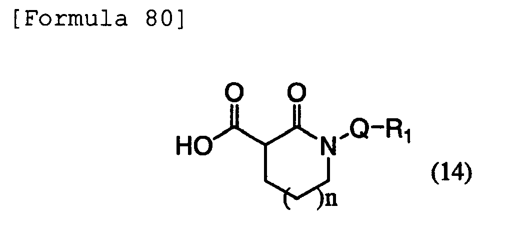 wherein Q, R₁ and n are as defined in [1] above, the process comprising:
   hydrolyzing, in the presence of a base, a compound (13) represented by the formula (13): 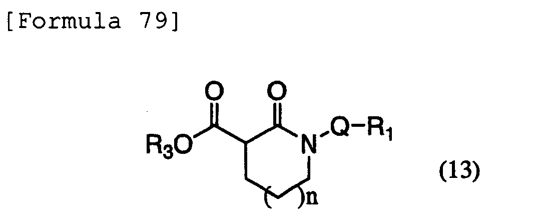 wherein R₃ is as defined in [1] above and Q, R₁ and n are as defined above;
[27]. A process for producing a compound (13) represented by the formula (13): 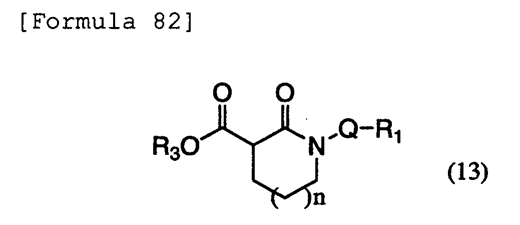 wherein Q, R₁ and n are as defined in [1] above and R₃ is as defined in [24] above, the process comprising:
   heating, in the coexistence of an acid, a compound (12) represented by the formula (12): 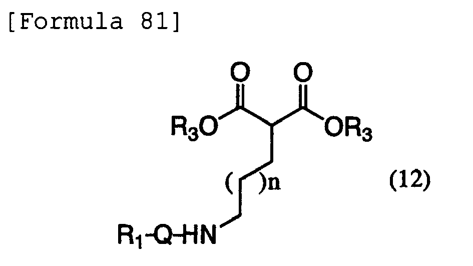 wherein Q, R₁, R₃ and n are as defined above;
[28]. A process for producing a compound (12) represented by the formula (12): 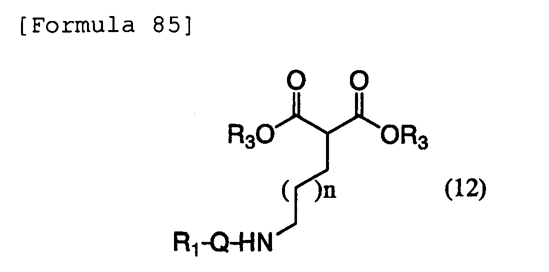 wherein Q, R₁ and n are as defined in [1] above and R₃ is as defined in [24] above, the process comprising:
   reacting, in the presence of a reducing agent, a compound (11) represented by the formula (11): 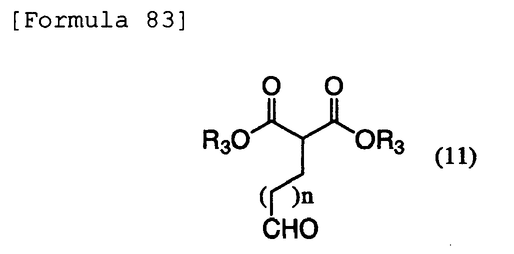 wherein R₃ and n are as defined above, with a compound (1) represented by the formula (1):

   [Formula 84] R₁-Q-NH₂ (1)

   wherein Q and R₁ are as defined above;
[29]. A compound (18) represented by the formula (18): 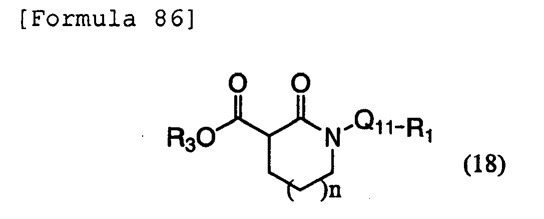 wherein R₁ and n are as defined in [1] above, Q₁₁ is as defined in [7] above and R₃ is as defined in [24] above, or a salt thereof, or a hydrate thereof; and
[30]. A compound (19) represented by the formula (19): 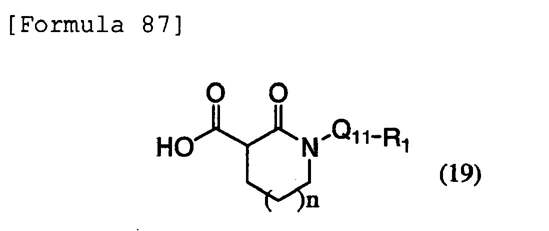
wherein R₁ and n are as defined in [1] above and Q₁₁ is as defined in [7] above, or a salt thereof, or a hydrate thereof.

Cinnamide derivatives, in particular, compounds typified by the structural formula (I) having a cyclic amide moiety with a cyclic structure such as a pyrrolidone ring or piperidone ring have not yet been known. Accordingly, the processes for producing such compounds according to these inventions and the synthetic intermediates used for the processes are novel.

Meanings of symbols, terms and the like used in the specification will be explained.

In the present specification, a structural formula of a compound may represent a certain isomer for convenience. However, the present invention includes all isomers and isomer mixtures such as geometric isomers which can be generated from the structure of a compound, optical isomers based on asymmetric carbon, stereoisomers and tautomers. The present invention is not limited to the description of a chemical formula for convenience and may include any one of the isomers or mixtures thereof. Accordingly, the compound of the present invention may have an asymmetric carbon atom in the molecule and exist as an optically active compound or racemate, and the present invention includes each of the optically active compound and the racemate without limitations. Although crystal polymorphs of the compound may be present, the compound is not limited thereto as well and may be present as a single crystal form or a mixture of single crystal forms. The compound may be an anhydride or a solvate such as a hydrate.

Any compound used in the production process of the present invention or any target compound produced by the process may be a salt. Specific examples of the salt include inorganic acid salts (such as sulfates, nitrates, perchlorates, phosphates, carbonates, bicarbonates, hydrofluorides, hydrochlorides, hydrobromides and hydroiodides), organic carboxylates (such as acetates, oxalates, maleates, tartrates, fumarates and citrates), organic sulfonates (such as methanesulfonates, trifluoromethanesulfonates, ethanesulfonates, benzenesulfonates, toluenesulfonates and camphorsulfonates), amino acid salts (such as aspartates and glutamates), quaternary amine salts, alkali metal salts (such as sodium salts and potassium salts) and alkali earth metal salts (such as magnesium salts and calcium salts).

The "diseases involving amyloid-β" refer to various diseases involving amyloid-β such as Alzheimer's disease.

In the present invention, the "6- to 14-membered cyclic aromatic hydrocarbon ring group" and the "5- to 14-membered aromatic heterocyclic group" in the "6- to 14-membered aromatic hydrocarbon ring group which may have a substituent" and the "5- to 14-membered aromatic heterocyclic group which may have a substituent" have the following meanings.

The "6- to 14-membered cyclic aromatic hydrocarbon ring group" refers to a monocyclic, bicyclic or tricyclic aromatic hydrocarbon ring group having 6 to 14 carbon atoms. Preferable examples of the group include 6- to 14-membered monocyclic, bicyclic or tricyclic aromatic hydrocarbon ring groups such as a phenyl group, indenyl group, naphthyl group, azulenyl group, heptalenyl group, biphenyl group, fluorenyl group, phenalenyl group, phenanthrenyl group and anthracenyl group.

The "5- to 14-membered aromatic heterocyclic group" refers to a monocyclic, bicyclic or tricyclic aromatic heterocyclic group having 5 to 14 carbon atoms. Preferable examples of the group include (1) nitrogen-containing aromatic heterocyclic groups such as a pyrrolyl group, pyridyl group, pyridazinyl group, pyrimidinyl group, pyrazinyl group, pyrazolinyl group, imidazolyl group, indolyl group, isoindolyl group, indolizinyl group, purinyl group, indazolyl group, quinolyl group, isoquinolyl group, quinolizinyl group, phthalazinyl group, naphthyridinyl group, quinoxalinyl group, quinazolinyl group, cinnolinyl group, pteridinyl group, imidazotriazinyl group, pyrazinopyridazinyl group, acridinyl group, phenanthridinyl group, carbazolyl group, perimidinyl group, phenanthrolinyl group and phenacyl group, (2) sulfur-containing aromatic heterocyclic groups such as a thienyl group and benzothienyl group, (3) oxygen-containing aromatic heterocyclic groups such as a furyl group, pyranyl group, cyclopentapyranyl group, benzofuranyl group and isobenzofuranyl group and (4) aromatic heterocyclic groups containing two or more hetero atoms selected from the group consisting of a nitrogen atom, sulfur atom and oxygen atom such as a thiazolyl group, isothiazolyl group, benzothiazolinyl group, benzothiadiazolyl group, phenothiazinyl group, isoxazolyl group, furazanyl group, phenoxazinyl group, pyrazoloxazolyl group, imidazothiazolyl group, thienofuryl group, furopyrrolyl group and pyridooxazinyl group.

The "substituent" in the "6- to 14-membered cyclic aromatic hydrocarbon ring group which may have a substituent" or the "5- to 14-membered aromatic heterocyclic group which may have a substituent" refers to 1 to 3 substituents selected from Substituent Group A1.
Substituent Group A1 refers to (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A2, (19) a C1-6 alkoxy group which may be substituted with 1 to 3 substituents selected from Substituent Group A2, (20) an amino group which may be substituted with 1 to 2 substituents selected from Substituent Group A2, (21) a carbamoyl group which may be substituted with 1 to 2 substituents selected from Substituent Group A2, (22) a 6- to 14-membered aromatic hydrocarbon ring group which may be substituted with 1 to 5 substituents selected from Substituent Group A2, (23) a 5- to 14-membered aromatic heterocyclic group which may be substituted with 1 to 3 substituents selected from Substituent Group A2, (24) a 6- to 14-membered non-aromatic hydrocarbon ring group which may be substituted with 1 to 3 substituents selected from Substituent Group A2, (25) a 5- to 14-membered non-aromatic heterocyclic group which may be substituted with 1 to 3 substituents selected from Substituent Group A2, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or-S- and A represents a 6- to 14-membered aromatic hydrocarbon ring group or 5-to 14-membered aromatic heterocyclic group which may be substituted with 1 to 3 substituents selected from Substituent Group A2), (31) -CO-A (wherein A is as defined above) and (32) =CH-A (wherein A is as defined above). Here, Substituent Group A2 refers to (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group and (17) a C1-6 alkoxyimino group.

The "substituent" in the "C1-6 alkyl group which may have a substituent" or the "phenyl group which may have a substituent" refers to a halogen atom, hydroxyl group, cyano group or nitro group.

The "halogen atom" refers to a fluorine atom, chlorine atom, bromine atom or iodine atom and is preferably a fluorine atom, chlorine atom or bromine atom, for example.

The "C3-8 cycloalkyl group" refers to a cyclic alkyl group having 3 to 8 carbon atoms. Preferable examples of the group include a cyclopropyl group, cyclobutyl group, cyclopentyl group, cyclohexyl group, cycloheptyl group and cyclooctyl group.

The "C2-6 alkenyl group" refers to an alkenyl group having 2 to 6 carbon atoms. Preferable examples of the group include linear or branched alkenyl groups such as a vinyl group, allyl group, 1-propenyl group, isopropenyl group, 1-buten-1-yl group, 1-buten-2-yl group, 1-buten-3-yl group, 2-buten-1-yl group and 2-buten-2-yl group.

The "C2-6 alkynyl group" refers to an alkynyl group having 2 to 6 carbon atoms. Preferable examples of the group include linear or branched alkynyl groups such as an ethynyl group, 1-propynyl group, 2-propynyl group, butynyl group, pentynyl group and hexynyl group.

The "C3-8 cycloalkoxy group" refers to a cyclic alkyl group having 3 to 8 carbon atoms in which one hydrogen atom is replaced by an oxygen atom. Preferable examples of the group include a cyclopropoxy group, cyclobutoxy group, cyclopentoxy group, cyclohexoxy group, cycloheptyloxy group and cyclooctyloxy group.

The "C3-8 cycloalkylthio group" refers to a cyclic alkyl group having 3 to 8 carbon atoms in which one hydrogen atom is replaced by a sulfur atom. Preferable examples of the group include a cyclopropylthio group, cyclobutylthio group, cyclopentylthio group, cyclohexylthio group, cycloheptylthio group and cyclooctylthio group.

The "C1-6 alkylcarbonyl group" refers to an alkyl group having 1 to 6 carbon atoms in which one hydrogen atom is replaced by a carbonyl group. Preferable examples of the group include an acetyl group, propionyl group and butyryl group.

The "C1-6 alkylthio group" refers to an alkyl group having 1 to 6 carbon atoms in which one hydrogen atom is replaced by a sulfur atom. Preferable examples of the group include a methylthio group, ethylthio group, *n*-propylthio group, *i*-propylthio group, n-butylthio group, *i*-butylthio group, *tert*-butylthio group, *n*-pentylthio group, *i*-pentylthio group, neopentylthio group, *n*-hexylthio group and 1-methylpropylthio group.

The "C1-6 alkylsulfinyl group" refers to an alkyl group having 1 to 6 carbon atoms in which one hydrogen atom is replaced by a sulfinyl group. Preferable examples of the group include a methanesulfinyl group and ethanesulfinyl group.

The "C1-6 alkylsulfonyl group" refers to an alkyl group having 1 to 6 carbon atoms in which one hydrogen atom is replaced by a sulfonyl group. Preferable examples of the group include a methanesulfonyl group and ethanesulfonyl group.

The "C1-6 alkoxyimino group" refers to an imino group in which a hydrogen atom is replaced by a C1-6 alkoxy group. Preferable examples of the group include a methoxyimino group and ethoxyimino group.

The "C1-6 alkyl group" refers to an alkyl group having 1 to 6 carbon atoms. Preferable examples of the group include linear or branched alkyl groups such as a methyl group, ethyl group, *n*-propyl group, i-propyl group, *n*-butyl group, *i*-butyl group, *tert*-butyl group, *n*-pentyl group, *i*-pentyl group, neopentyl group, *n*-hexyl group, 1-methylpropyl group, 1,2-dimethylpropyl group, 1-ethylpropyl group, 1-methyl-2-ethylpropyl group, 1-ethyl-2-methylpropyl group, 1,1,2-trimethylpropyl group, 1-methylbutyl group, 2-methylbutyl group, 1,1-dimethylbutyl group, 2,2-dimethylbutyl group, 2-ethylbutyl group, 1,3-dimethylbutyl group, 2-methylpentyl group and 3-methylpentyl group.

The "C1-6 alkoxy group" refers to an alkyl group having 1 to 6 carbon atoms in which a hydrogen atom is replaced by an oxygen atom. Preferable examples of the group include a methoxy group, ethoxy group, *n*-propoxy group, *i*-propoxy group, *n*-butoxy group, *i*-butoxy group, *sec*-butoxy group, *tert*-butoxy group, *n*-pentoxy group, *i*-pentoxy group, sec-pentoxy group, *tert*-pentoxy group, *n*-hexoxy group, *i*-hexoxy group, 1,2-dimethylpropoxy group, 2-ethylpropoxy group, 1-methyl-2-ethylpropoxy group, 1-ethyl-2-methylpropoxy group, 1,1,2-trimethylpropoxy group, 1,1-dimethylbutoxy group, 2,2-dimethylbutoxy group, 2-ethylbutoxy group, 1,3-dimethylbutoxy group, 2-methylpentoxy group, 3-methylpentoxy group and hexyloxy group.

The "amino group which may be substituted with a C1-6 alkyl group" refers to an amino group which may be substituted with an alkyl group having 1 to 6 carbon atoms. Preferable examples of the group include an amino group, methylamino group, ethylamino group, propylamino group and dimethylamino group.

The "6- to 14-membered non-aromatic hydrocarbon ring group" refers to a cyclic aliphatic hydrocarbon group having 6 to 14 carbon atoms.
Examples of the group include cyclic aliphatic hydrocarbon groups having 6 to 14 carbon atoms such as a cyclopropyl group, cyclobutyl group, cyclopentyl group, cyclohexyl group, cycloheptyl group, cyclooctyl group, spiro[3.4]octanyl group, decanyl group, indanyl group, 1-acenaphthenyl group, cyclopentacyclooctenyl group, benzocyclooctenyl group, indenyl group, tetrahydronaphthyl group, 6,7,8,9-tetrahydro-5H-benzocycloheptenyl group and 1,4-dihydronaphthalenyl group.

The "5- to 14-membered non-aromatic heterocyclic group" 1) has 5 to 14 ring-forming atoms, 2) contains 1 to 5 hetero atoms such as a nitrogen atom, -O- or -S- in the ring-forming atoms, and 3) may contain one or more carbonyl groups, double bonds or triple bonds in the ring, and refers not only to a 5-to 14-membered non-aromatic monocyclic heterocyclic group but also to a saturated heterocyclic group condensed with an aromatic hydrocarbon ring group or a saturated hydrocarbon ring group or saturated heterocyclic group condensed with an aromatic heterocyclic group. Specific examples of the 5- to 14-membered non-aromatic heterocyclic group include an azetidinyl group, pyrrolidinyl group, piperidinyl group, azepanyl group, azocanyl group, tetrahydrofuranyl group, tetrahydropyranyl group, morpholinyl group, thiomorpholinyl group, piperazinyl group, thiazolidinyl group, dioxanyl group, imidazolinyl group, thiazolinyl group, 1,2-benzopyranyl group, isochromanyl group, chromanyl group, indolinyl group, isoindolinyl group, azaindanyl group, azatetrahydronaphthyl group, azachromanyl group, tetrahydrobenzofuranyl group, tetrahydrobenzothienyl group, 2,3,4,5-tetrahydrobenzo[b]thienyl group, 3,4-dihydro-2 X-benzo[b][1,4]dioxepinyl group, indan-1-onyl group, 6,7-dihydro-5H-cyclopentapyrazinyl group, 6,7-dihydro-5H-[1]pyridinyl group, 6,7-dihydro-5H-[1]pyridinyl group, 5,6-dihydro-4H-cyclopenta[b]thienyl group, 4,5,6,7-tetrahydrobenzo[b]thienyl group, 3,4-dihydro-2H-naphthalen-1-onyl group, 2,3-dihydroisoindol-1-onyl group, 3,4-dihydro-2H-isoquinolin-1-onyl group and 3,4-dihydro-2H-benzo[1,4]oxapinyl group.

The "C2-6 alkenyloxy group" refers to an alkenyl group having 2 to 6 carbon atoms in which one hydrogen atom is replaced by an oxygen atom.
Preferable examples of the group include linear or branched alkenyloxy groups such as a vinyloxy group, allyloxy group, 1-propenyloxy group, isopropenyloxy group, 1-buten-1-yloxy group, 1-buten-2-yloxy group, 1-buten-3-yloxy group, 2-buten-1-yloxy group and 2-buten-2-yloxy group.

The "C2-6 alkynyloxy group" refers to an alkynyl group having 2 to 6 carbon atoms in which one hydrogen atom is replaced by an oxygen atom.
Preferable examples of the group include linear or branched alkynyloxy groups such as an ethynyloxy group, 1-propynyloxy group, 2-propynyloxy group, butynyloxy group, pentynyloxy group and hexynyloxy group.

The "C3-8 cycloalkylsulfinyl group" refers to a cyclic alkyl group having 3 to 8 carbon atoms in which one hydrogen atom is replaced by a sulfinyl group. Preferable examples of the group include a cyclopropylsulfinyl group, cyclobutylsulfinyl group, cyclopentylsulfinyl group, cyclohexylsulfinyl group, cycloheptylsulfinyl group and cyclooctylsulfinyl group.

The "C3-8 cycloalkylsulfonyl group" refers to a cyclic alkyl group having 3 to 8 carbon atoms in which one hydrogen atom is replaced by a sulfonyl group. Preferable examples of the group include a cyclopropylsulfonyl group, cyclobutylsulfonyl group, cyclopentylsulfonyl group, cyclohexylsulfonyl group, cycloheptylsulfonyl group and cyclooctylsulfonyl group.

Examples of the protecting group in the "6-to 14-membered aromatic hydrocarbon ring group which may have a substituent (wherein the substituent may have a protecting group) or 5- to 14-membered aromatic heterocyclic group which may have a substituent (wherein the substituent may have a protecting group" include, but are not limited to, protecting groups described in "Protective Groups In Organic Synthesis Second Edition by T.W. Greene and P.G.M. Wuts John Wiley & Sons, Inc.".

Examples of the protecting group for a hydroxyl group include a methoxymethyl group, methylthiomethyl group, tetrahydrofuranyl group, 1-ethoxyethyl group, *tert*-butyldimethylsilyl group, benzyl group, *tert*-butyl group, allyl group and triphenylmethyl group.
Examples of the protecting group for a carboxyl group include a methyl group, ethyl group, benzyl group, 2,2,2-trichloroethyl group, o-nitrobenzyl group, *p*-nitrobenzyl group, β-*p*-toluenesulfonylethyl group and *p*-methoxybenzyl group.
Examples of the protecting group for an amino group include an N-formyl group, N-acetyl group, N-chloroacetyl group, N-benzoyl group, *tert*-butyl group, N-phthalimide group, diphenylmethyl group and benzyl group.

The "ester group" refers to a C1-6 alkoxycarbonyl group. Examples of the group include carbonyl groups such as a methoxycarbonyl group, ethoxycarbonyl group, *n*-propoxycarbonyl group, i-propoxycarbonyl group, *n*-butoxycarbonyl group, i-butoxycarbonyl group, *sec*-butoxycarbonyl group, *tert-*butoxycarbonyl group, *n*-pentoxycarbonyl group, *i-*pentoxycarbonyl group, *sec*-pentoxycarbonyl group, *tert-*pentoxycarbonyl group, *n*-hexoxycarbonyl group, i-hexoxycarbonyl group, 1,2-dimethylpropoxycarbonyl group, 2-ethylpropoxycarbonyl group, 1-methyl-2-ethylpropoxycarbonyl group, 1-ethyl-2-methylpropoxycarbonyl group, 1,1,2-trimethylpropoxycarbonyl group, 1,1-dimethylbutoxycarbonyl group, 2,2-dimethylbutoxycarbonyl group, 2-ethylbutoxycarbonyl group, 1,3-dimethylbutoxycarbonyl group, 2-methylpentoxycarbonyl group and 3-methylpentoxycarbonyl group.

Examples of the "halogenating reagent" include chlorine, bromine, iodine, NCS (N-chlorosuccinimide), NBS (N-bromosuccinimide), sulfuryl chloride, thionyl chloride, sulfuryl bromide and thionyl bromide.

Examples of the "leaving group introduction reagent" include a halogenating reagent, a sulfonylating reagent and an acylating reagent.

The "sulfonylating reagent" refers to a reagent converting a hydroxyl group into a sulfonyloxy group. Examples of the sulfonylating reagent include methanesulfonyl chloride, *p*-toluenesulfonyl chloride, trifluoromethanesulfonyl chloride, methanesulfonic anhydride, p-toluenesulfonic anhydride and trifluoromethanesulfonic anhydride.

The "acylating reagent" refers to a reagent converting a hydroxyl group into an acyloxy group. Examples of the acylating reagent include acetyl chloride, acetyl bromide and acetic anhydride.

Preferable examples of the "protecting group" for a hydrolyzable carboxyl group include an ethyl ester group, methyl ester group, β,β,β-trichloroethyl ester group, β-*p*-toluenesulfonylethyl ester group, benzyl ester group, *p*-methoxybenzyl ester group and p-nitrobenzyl ester group.

The present invention will be described in detail below.

The production processes of the present invention are roughly classified into the following Production Processes 1, 2, 3 and 4.

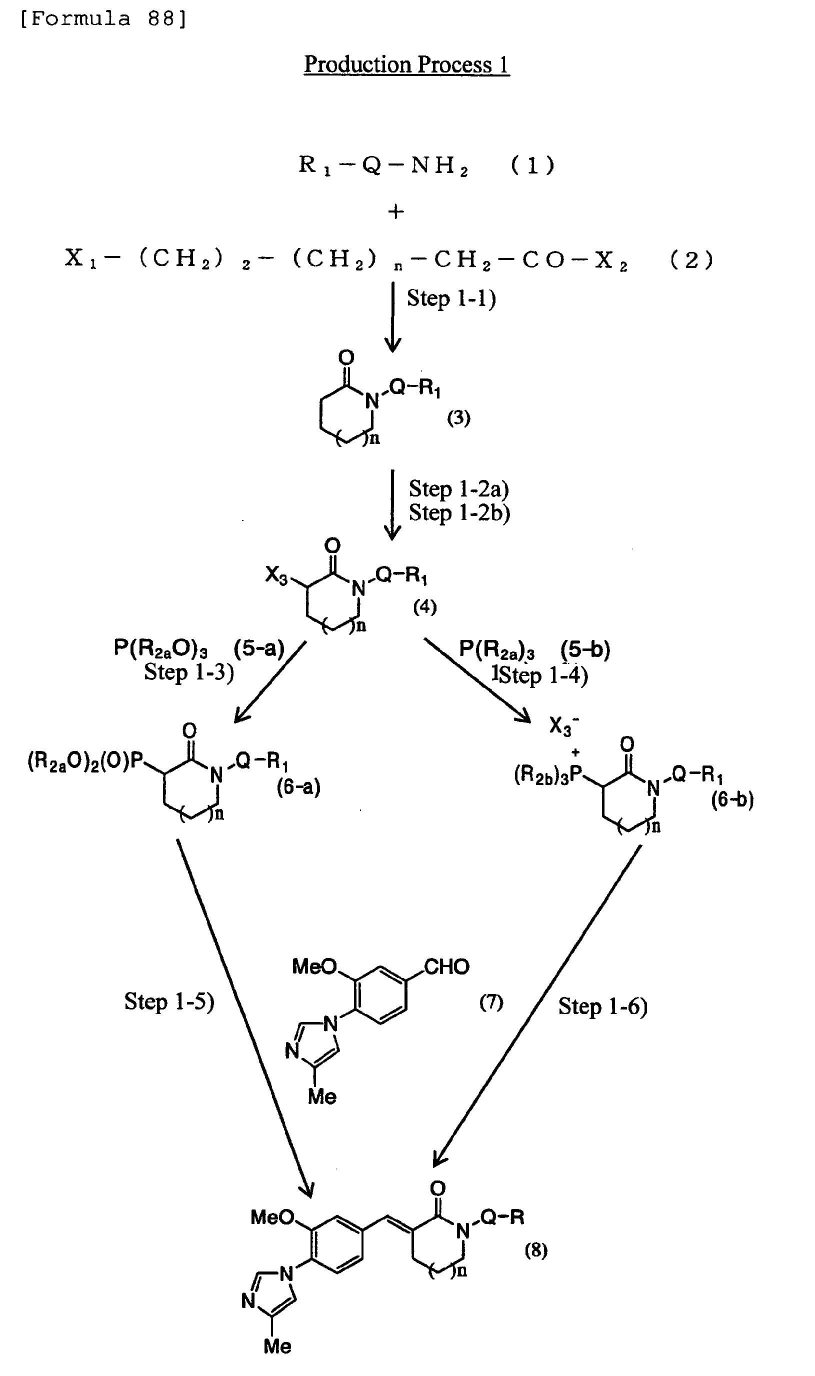

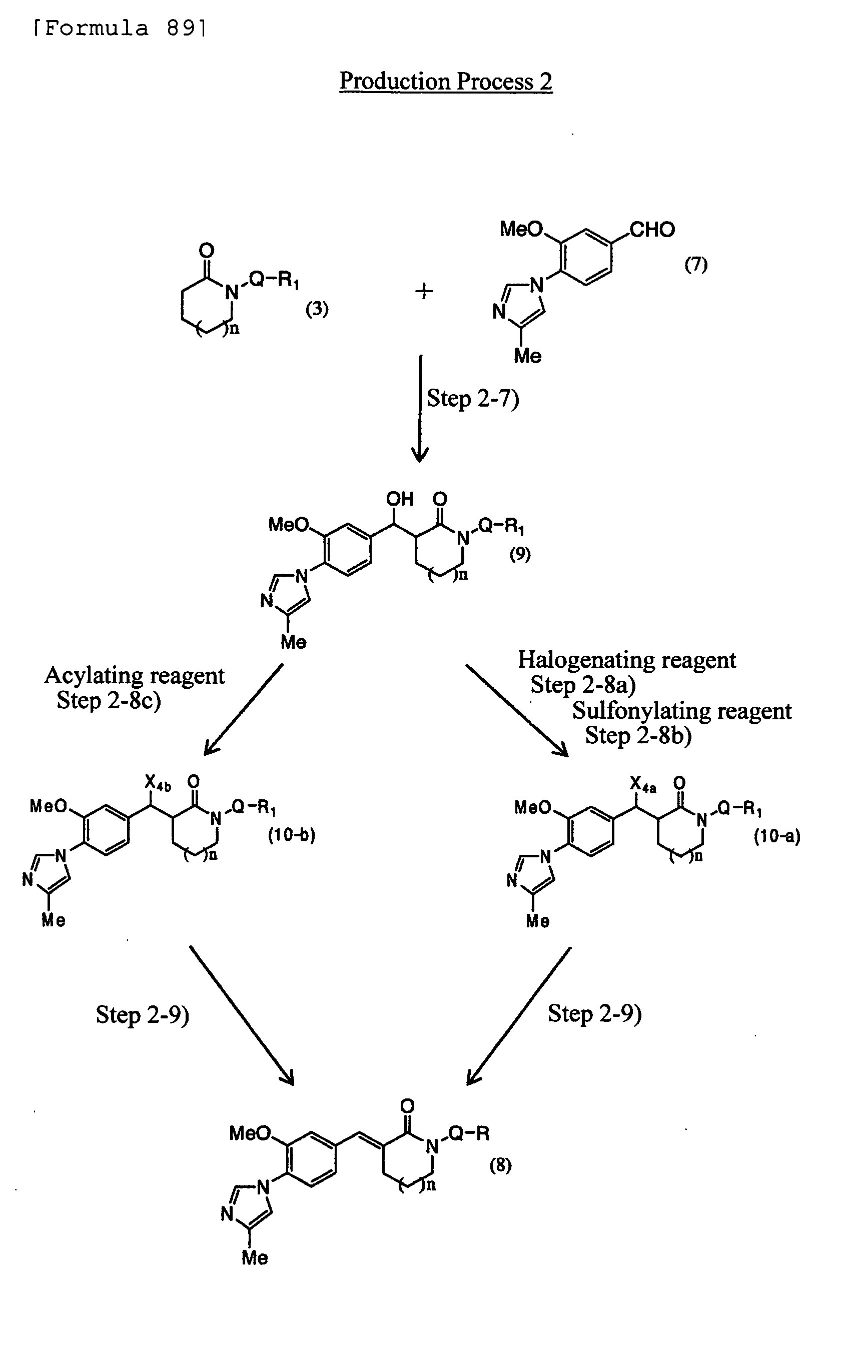

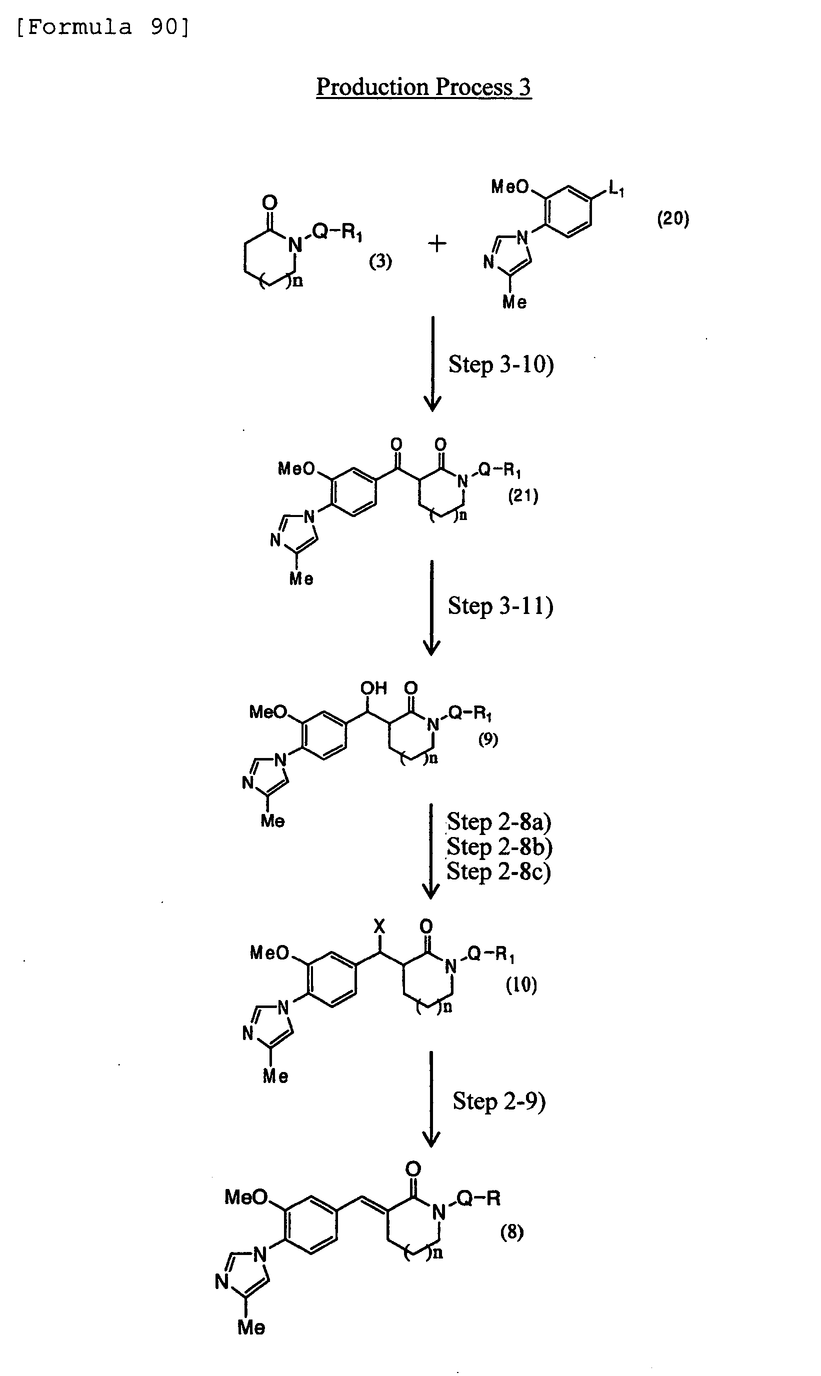

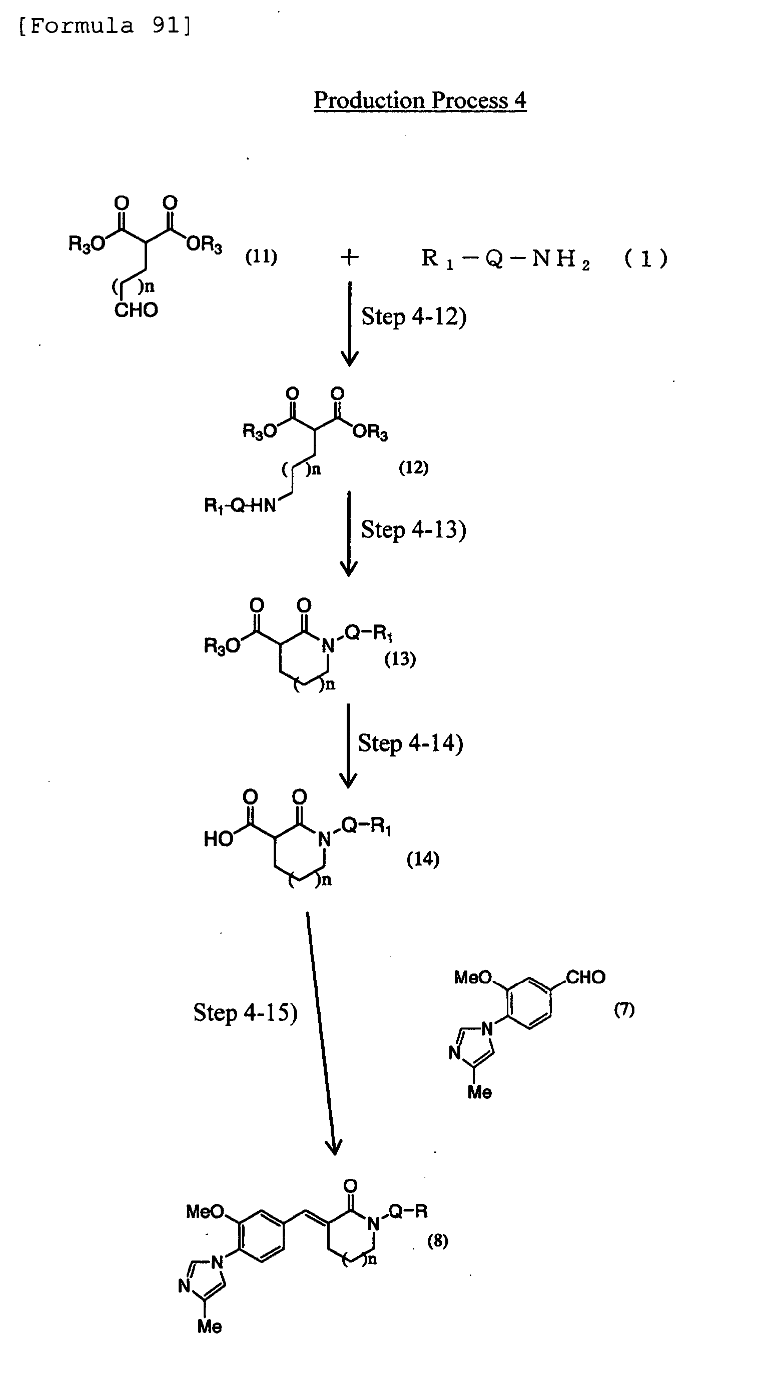

Next, the production processes 1, 2, 3 and 4 will be described in detail.

1. Production Process 1

### 1) Process for producing compound represented by formula (3) (Step 1-1))

The production process is a step of reacting a compound (1) with a compound (2) in the presence of a base to convert the compound (1) into a compound represented by the formula (3) (hereinafter referred to as Step 1-1)).

Step 1-1) can be carried out by a generally used method described in Shin Jikken Kagaku Koza (New Experimental Chemistry Course) 14, Yuki Kagobutsu no Gosei to Hanno (Synthesis and reaction of organic compounds) [II] (pages 1134-1220) or the like. More specifically, this step can be carried out with reference to the reaction conditions, the operation after the reaction, the purification method and the like described in the later-described Example 5.

1-1-1) The production process in an aqueous solvent in the presence of a base and a phase transfer catalyst will be described in detail.

As the compound represented by the formula (1), it is possible to use a known compound, a commercially available compound, or a compound that can be readily produced from a commercially available compound by a method usually carried out by a person skilled in the art.
As the compound represented by the formula (2), it is possible to use a known compound, a commercially available compound, or a compound that can be readily produced from a commercially available compound by a method usually carried out by a person skilled in the art.

The solvent used in this reaction is not particularly limited insofar as the solvent allows the starting material to be dissolved therein to a certain extent and does not inhibit the reaction. Preferable examples of the solvent include mixed solvents of solvents such as benzene, toluene, xylene, isopropanol, tetrahydrofuran, *tert*-butyl methyl ether, cyclopentyl methyl ether, acetonitrile, N,N-dimethylformamide, 1,4-dioxane, 1,2-dimethoxyethane, 1-methyl-2-pyrrolidone and 1,3-dimethyl-2-imidazolidinone and water. More preferable examples of the solvent include mixed solvents of solvents such as toluene and *tert*-butyl methyl ether and water.

The amount of the compound (2) used may be appropriately increased and reduced and may be preferably 1 to 3 mole, for example, and more preferably 1.0 to 1.3 mole, for example, per mole of the compound (1).

Preferable examples of the base used in this reaction include lithium hydroxide, sodium hydroxide, potassium hydroxide, lithium carbonate, sodium carbonate, potassium carbonate, cesium carbonate, sodium bicarbonate and potassium bicarbonate. More preferable examples of the base include bases such as sodium hydroxide, potassium hydroxide, sodium carbonate and potassium carbonate.

The amount of the base used may be appropriately increased and reduced and is preferably 1.0 to 50.0 mole, for example, and more preferably 1.0 to 30.0 mole, for example, per mole of the compound (1).

Preferable examples of the phase transfer catalyst used in this reaction include benzyltriethylammonium chloride, benzyltriethylammonium bromide, *tetra-n*-butylammonium fluoride, tetra-n-butylammonium chloride and tetra-n-butylammonium bromide. The phase transfer catalyst is more preferably benzyltriethylammonium chloride, for example.

The amount of the phase transfer catalyst used may be appropriately increased and reduced and is preferably 0.05 to 0.5 mole, for example, and more preferably 0.05 to 0.15 mole, for example, per mole of the compound (1).

The reaction temperature usually varies according to the starting material, the solvent and other reagents used in the reaction and the setting may be appropriately changed. The reaction temperature is preferably -5°C to 50°C, for example, and more preferably 0°C to 30°C, for example.

Usually, the reaction time may be appropriately increased and reduced according to the starting material, the solvent, other reagents used in the reaction, the reaction temperature and the degree of progress of the reaction. The reaction time at the aforementioned reaction temperature is preferably 0.5 to 200 hours, for example, and more preferably about 96 hours, for example.

1-1-2) The production process including reacting the compound represented by the formula (2) with the compound represented by the formula (1) in the presence of a base and subjecting the resulting ring-opening amide to cyclization reaction in the presence of a base will be described below.

The solvent used in the reaction of providing the ring-opening amide is not particularly limited insofar as the solvent allows the starting material to be dissolved therein to a certain extent and does not inhibit the reaction. Preferable examples of the solvent include mixed solvents of solvents such as benzene, toluene, xylene, isopropanol, tetrahydrofuran, *tert*-butyl methyl ether, cyclopentyl methyl ether, acetonitrile, N,N-dimethylformamide, 1,4-dioxane, 1,2-dimethoxyethane, 1-methyl-2-pyrrolidone and 1,3-dimethyl-2-imidazolidinone and water. More preferable examples of the solvent include mixed solvents of solvents such as toluene, cyclopentyl methyl ether and *tert*-butyl methyl ether and water.

The amount of the compound (2) used may be appropriately increased and reduced and may be preferably 1 to 3 mole, for example, and more preferably 1.0 to 1.3 mole, for example, per mole of the compound (1).

Preferable examples of the base used in the reaction of providing the ring-opening amide include lithium hydroxide, sodium hydroxide, potassium hydroxide, lithium carbonate, sodium carbonate, potassium carbonate, cesium carbonate, sodium bicarbonate and potassium bicarbonate. More preferable examples of the base include bases such as sodium hydroxide, potassium hydroxide, sodium carbonate and potassium carbonate.

The amount of the base used in the reaction of providing the ring-opening amide may be appropriately increased and reduced and is preferably 1.0 to 10.0 mole, for example, and more preferably 1.0 to 3.0 mole, for example, per mole of the compound (1).

The reaction temperature in the reaction of providing the ring-opening amide usually varies according to the starting material, the solvent and other reagents used in the reaction and the setting may be appropriately changed. The reaction temperature is preferably -5°C to 50°C, for example, and more preferably 0°C to 30°C, for example.

Usually, the reaction time in the reaction of providing the ring-opening amide may be appropriately increased and reduced according to the starting material, the solvent, other reagents used in the reaction, the reaction temperature and the degree of progress of the reaction. The reaction time at the aforementioned reaction temperature is preferably 0.5 to 200 hours, for example, and more preferably about 1 to 2 hours, for example.

The solvent used in the reaction of cyclizing the ring-opening amide to provide the compound represented by the formula (3) is not particularly limited insofar as the solvent allows the starting material to be dissolved therein to a certain extent and does not inhibit the reaction. Preferable examples of the solvent include mixed solvents of solvents such as benzene, toluene, xylene, isopropanol, tetrahydrofuran, *tert*-butyl methyl ether, cyclopentyl methyl ether, acetonitrile, N,N-dimethylformamide, 1,4-dioxane, 1,2-dimethoxyethane, 1-methyl-2-pyrrolidone and 1,3-dimethyl-2-imidazolidinone and water. More preferable examples of the solvent include solvents such as tetrahydrofuran, toluene, cyclopentyl methyl ether and *tert*-butyl methyl ether and mixed solvents thereof.
The cyclization reaction of the ring-opening amide without a solvent can also provide the compound represented by the formula (3).

Preferable examples of the base used in the reaction of cyclizing the ring-opening amide to provide the compound represented by the formula (3) include sodium ethoxide, sodium methoxide, sodium tert-butoxide, potassium tert-butoxide, triethylamine, pyridine, diisopropylethylamine, lithium hydroxide, sodium hydroxide, potassium hydroxide, lithium carbonate, sodium carbonate, potassium carbonate, cesium carbonate, sodium bicarbonate and potassium bicarbonate. More preferable examples of the base include bases such as sodium ethoxide, sodium methoxide, potassium tert-butoxide and sodium tert-butoxide.

The amount of the base used in the reaction of cyclizing the ring-opening amide to provide the compound represented by the formula (3) may be appropriately increased and reduced and is preferably 1.0 to 10.0 mole, for example, and more preferably 1.0 to 3.0 mole, for example, per mole of the compound (1).

The reaction temperature in the reaction of cyclizing the ring-opening amide to provide the compound represented by the formula (3) usually varies according to the starting material, the solvent and other reagents used in the reaction and the setting may be appropriately changed. The reaction temperature is preferably -5°C to 50°C, for example, and more preferably 0°C to 30°C, for example.

Usually, the reaction time in the reaction of cyclizing the ring-opening amide to provide the compound represented by the formula (3) may be appropriately increased and reduced according to the starting material, the solvent, other reagents used in the reaction, the reaction temperature and the degree of progress of the reaction. The reaction time at the aforementioned reaction temperature is preferably 0.5 to 10 hours, for example, and more preferably about 0.5 to 1 hours, for example.

Among the compounds (3) obtained in this manner, a compound (15) represented by the formula (15) is a novel compound.

### 2) Process for producing compound represented by formula (4) (Step 1-2a))

The production process is a step of treating the compound (3) with a base and then reacting the treated compound with a halogenating reagent to convert the compound (3) into a compound represented by the formula (4) (hereinafter referred to as Step 1-2a)).
Step 1-2a) can be carried out by a method described in Shin Jikken Kagaku Koza (New Experimental Chemistry Course) 14, Yuki Kagobutsu no Gosei to Hanno (Synthesis and reaction of organic compounds) [I] (pages 307-450) or the like. More specifically, this step can be carried out with reference to the reaction conditions, the operation after the reaction, the purification method and the like described in the later-described Example 6.

As the compound (3), it is possible to use a compound that can be produced by a method described in the later-described Example 3 or the like, for example.

This reaction is preferably carried out in a stream or atmosphere of an inert gas such as nitrogen or argon.

As the compound represented by the formula (3), it is possible to use a compound that can be produced by a method described in the later-described Example 5 or the like, a known compound, a commercially available compound, or a compound that can be readily produced from a commercially available compound by a method usually carried out by a person skilled in the art.

The solvent used in this reaction is not particularly limited insofar as the solvent allows the starting material to be dissolved therein to a certain extent and does not inhibit the reaction. Preferable examples of the solvent that may be used include tetrahydrofuran, 1,2-dimethoxyethane, *tert*-butyl methyl ether, cyclopentyl methyl ether, diethyl ether, diisopropyl ether, dibutyl ether, dicyclopentyl ether, benzene, toluene, heptane, hexane, cyclohexane and mixed solvents thereof. More preferable examples of the solvent include tetrahydrofuran, toluene, hexane, cyclohexane and mixed solvents thereof.

Preferable examples of the base that may be used in this reaction include *n*-butyllithium, sec-butyllithium, *tert*-butyllithium and lithium diisopropylamide. More preferable examples of the base include *sec*-butyllithium and *tert*-butyllithium.

The amount of the base used may be appropriately increased and reduced and is preferably 1.0 to 3.0 mole, for example, and more preferably 1.1 to 1.4 mole, for example, per mole of the compound (3).

The temperature for reacting with the base usually varies according to the starting material, the solvent and other reagents used in the reaction and the setting may be appropriately changed. The reaction temperature is preferably -78°C to 10°C, for example, and more preferably -78°C to -50°C, for example.

The time for reaction with the base usually varies according to the starting material, the solvent, other reagents used in the reaction, the reaction temperature and the degree of progress of the reaction and may be appropriately increased and reduced. The reaction time at the aforementioned reaction temperature after addition of the base is preferably 10 minutes to 2 hours, for example, and more preferably about 20 minutes, for example.

The halogenating reagent used in this reaction is preferably chlorine, bromine or iodine, for example, and more preferably chlorine or bromine, for example.

The amount of the halogenating reagent used may be appropriately increased and reduced and is preferably 1 to 3 mole, for example, and more preferably 1.05 to 1.2 mole, for example, per mole of the compound (3).

The temperature for reacting with the halogenating reagent usually varies according to the starting material, the solvent and other reagents used in the reaction and the setting may be appropriately changed. The reaction temperature is preferably -78°C to -20°C, for example, and more preferably -78°C to - 40°C, for example.

The time for reaction with the halogenating reagent usually varies according to the starting material, the solvent, other reagents used in the reaction, the reaction temperature and the degree of progress of the reaction and may be appropriately increased and reduced. The reaction time at the aforementioned reaction temperature after addition of the base is preferably 10 minutes to 2 hours, for example, and more preferably 20 minutes to 1 hour, for example.

### 3) Process for producing compound represented by formula (4) (Step 1-2b))

The production process is a step of reacting the compound (3) with a halogenating reagent in the presence of a base to convert the compound (3) into a compound represented by the formula (4) (hereinafter referred to as Step 1-2b)).
Step 1-2b) can be carried out by a method described in J. of Organic Chemistry., (58), 3384-3386 (1993) A.O. King et. al., or the like. More specifically, this step can be carried out with reference to the reaction conditions, the operation after the reaction, the purification method and the like described in the later-described Example 8.

As the compound (3), it is possible to use a compound that can be produced by a method described in the later-described Example 3 or the like, for example.

This reaction is preferably carried out in a stream or atmosphere of an inert gas such as nitrogen or argon.

The solvent used in this reaction is not particularly limited insofar as the solvent allows the starting material to be dissolved therein to a certain extent and does not inhibit the reaction. Preferable examples of the solvent that may be used include tetrahydrofuran, 1,2-dimethoxyethane, *tert*-butyl methyl ether, cyclopentyl methyl ether, diethyl ether, diisopropyl ether, dibutyl ether, dicyclopentyl ether, benzene, toluene, heptane, hexane, acetonitrile, methylene chloride and mixed solvents thereof. More preferable examples of the solvent include acetonitrile, methylene chloride, toluene, hexane and cyclohexane.

Preferable examples of the base used in this reaction include triethylamine, diethylmethylamine, diisopropylethylamine and *N,N,N',N'-*tetramethylethylenediamine.

The amount of the base used may be appropriately increased and reduced and is preferably 2.0 to 10.0 mole, for example, and more preferably 3.0 to 5.0 mole, for example, per mole of the compound (3).

Preferable examples of the halogenating reagent used in this reaction include iodotrimethylsilane-iodine and chlorotrimethylsilane-iodine.

The amount of chlorotrimethylsilane used may be appropriately increased and reduced and is preferably 1.0 to 10.0 mole, for example, and more preferably 1.0 to 5.0 mole, for example, per mole of the compound (3).

The amount of iodine or chlorine used may be appropriately increased and reduced and is preferably 1.0 to 10.0 mole, for example, and more preferably 1.5 to 3.0 mole, for example, per mole of the compound (3).

The reaction temperature usually varies according to the starting material, the solvent and other reagents used in the reaction and the setting may be appropriately changed. The reaction temperature is preferably -78°C to 20°C, for example, and more preferably -40°C to 10°C, for example.

The reaction time usually varies according to the starting material, the solvent, other reagents used in the reaction, the reaction temperature and the degree of progress of the reaction and may be appropriately increased and reduced. The reaction time at the aforementioned reaction temperature is preferably 30 minutes to 3 hours, for example, and more preferably about 1 hour, for example.

Among the compounds (4) obtained in this manner, a compound (16) represented by the formula (16) is a novel compound.

### 4) Process for producing compound represented by formula (6-a) (Step 1-3))

The production process is a step of reacting the compound represented by the formula (4) with a phosphorous acid compound represented by the formula (5-a) to convert the compound represented by the formula (4) into a compound represented by the formula (6-a) (hereinafter referred to as Step 1-3)).

Step 1-3) can be carried out by a generally used method described in Tetrahedron Letters, 37(9), 1433-1434 (1996) D. Kim et al., Organophosphorus Reagents in Organic Synthesis, Edited by J.I.G. Cadogan Academic Press (1979) or the like. More specifically, this step can be carried out with reference to the reaction conditions, the operation after the reaction, the purification method and the like described in the later-described Example 7 and Example 9.

As the compound represented by the formula (4), it is possible to use a compound that can be produced by a method described in the later-described Example 6 or Example 8 or the like, for example.

As the phosphorous acid compound represented by the formula (5-a), it is possible to use a known compound, a commercially available compound such as trimethyl phosphite, triethyl phosphite, triphenyl phosphite or 2,2,2-trifluoroethoxy phosphite, or a compound that can be readily produced from a commercially available compound by a method usually carried out by a person skilled in the art.

The solvent used in this reaction is not particularly limited insofar as the solvent allows the starting material to be dissolved therein to a certain extent and does not inhibit the reaction. Preferable examples of the solvent include benzene, toluene, xylene, *N,N*-dimethylformamide, N-methylpyrrolidone, acetonitrile, dimethyl sulfoxide and no solvent. More preferably, the solvent is no solvent, for example.

The amount of the used compound represented by the formula (5-a) may be appropriately increased and reduced and is preferably 1 to 15 mole, for example, and more preferably 1.0 to 4.0 mole, for example, per mole of the compound (4).

The reaction temperature usually varies according to the starting material, the solvent and other reagents used in the reaction and the setting may be appropriately changed. The reaction temperature is preferably 50°C to 150°C, for example, and more preferably 60°C to 100°C, for example.

The reaction time usually varies according to the starting material, the solvent, other reagents used in the reaction, the reaction temperature and the degree of progress of the reaction and may be appropriately increased and reduced. The reaction time at the aforementioned reaction temperature after addition of the base is preferably 1 to 20 hours, for example, and more preferably 5 to 10 hours, for example.

Among the compounds (6-a) obtained in this manner, a compound represented by the formula (17) is a novel compound.

### 5) Process for producing compound represented by formula (6-b) (Step 1-4))

The production process is a step of reacting the compound represented by the formula (4) with a phosphorus compound represented by the formula (5-b) to convert the compound represented by the formula (4) into a compound represented by the formula (6-b) (hereinafter referred to as Step 1-4)).

Step 1-4) can be carried out by a generally used method described in Organophosphorus Reagents in Organic Synthesis, Edited by J.I.G. Cadogan Academic Press (1979) or the like. More specifically, this step can be carried out with reference to the reaction conditions, the operation after the reaction, the purification method and the like described in the later-described Example 17.

As the compound represented by the formula (4), it is possible to use a compound that can be produced by a method described in the later-described Example 6 or Example 8 or the like, for example.

As the phosphorus compound represented by the formula (5-b), it is possible to use a known compound, a commercially available compound such as triphenylphosphine or tri-*n*-butylphosphine, or a compound that can be readily produced from a commercially available compound by a method usually carried out by a person skilled in the art.

The solvent used in this reaction is not particularly limited insofar as the solvent allows the starting material to be dissolved therein to a certain extent and does not inhibit the reaction. Preferable examples of the solvent include benzene, toluene, xylene, N,N-dimethylformamide, N-methylpyrrolidone, acetonitrile, dimethyl sulfoxide, tetrahydrofuran, diethyl ether, methanol, ethanol and no solvent. More preferably, the solvent is tetrahydrofuran, for example.

The amount of the compound (5-b) used may be appropriately increased and reduced and is preferably 1 to 15 mole, for example, and more preferably 1.0 to 4.0 mole, for example, per mole of the compound (4).

The reaction temperature usually varies according to the starting material, the solvent and other reagents used in the reaction and the setting may be appropriately changed. The reaction temperature is preferably 50°C to 150°C, for example, and more preferably 60°C to 100°C, for example.

The reaction time usually varies according to the starting material, the solvent, other reagents used in the reaction, the reaction temperature and the degree of progress of the reaction and may be appropriately increased and reduced. The reaction time at the aforementioned reaction temperature after addition of the base is preferably 1 to 20 hours, for example, and more preferably 2 to 10 hours, for example.

### 6) Process for producing compound represented by formula (8) (Step 1-5))

The production process is a step of reacting the compound represented by the formula (6-a) with a compound represented by the formula (7) in the presence of a base to convert the compound represented by the formula (6-a) into a compound represented by the formula (8) (hereinafter referred to as Step 1-5)).

Step 1-5) can be carried out by a generally used method described in Organophosphorus Reagents in Organic Synthesis, Edited by J.I.G. Cadogan Academic Press (1979) or the like. More specifically, this step can be carried out with reference to the reaction conditions, the operation after the reaction, the purification method and the like described in the later-described Example 10.

As the compound represented by the formula (6-a), it is possible to use a compound that can be produced by a method described in the later-described Example 7 or Example 9 or the like, a known compound, a commercially available compound, or a compound that can be readily produced from a commercially available compound by a method usually carried out by a person skilled in the art.

As the compound represented by the formula (7), it is possible to use a compound that can be produced by a method described in Reference Example 1, for example.

The solvent used in this reaction is not particularly limited insofar as the solvent allows the starting material to be dissolved therein to a certain extent and does not inhibit the reaction. Preferable examples of the solvent that may be used include 1,4-dioxane, tetrahydrofuran, 1,2-dimethoxyethane, *tert-*butyl methyl ether, cyclopentyl methyl ether, diethyl ether, diisopropyl ether, dibutyl ether, dicyclopentyl ether, N,N-dimethylformamide, N-methylpyrrolidone, acetonitrile, dimethyl sulfoxide, methanol, ethanol, propanol, butanol and mixed solvents thereof. More preferably, a mixed solvent of tetrahydrofuran and ethanol may be used, for example.

The amount of the compound (6-a) used may be appropriately increased and reduced and is preferably 1.0 to 3.0 mole, for example, and more preferably 1.0 to 2.0 mole, for example, per mole of the compound (7).

Preferable examples of the base that may be used in this reaction include lithium hydroxide, sodium hydroxide, potassium hydroxide, lithium carbonate, sodium carbonate, potassium carbonate, cesium carbonate, sodium bicarbonate and potassium bicarbonate. More preferable examples of the base include sodium hydroxide and lithium hydroxide.

The amount of the base used may be appropriately increased and reduced and is preferably 1.0 to 5.0 mole, for example, and more preferably 1.5 to 2.5 mole, for example, per mole of the compound (6-a) .

The reaction temperature usually varies according to the starting material, the solvent and other reagents used in the reaction and the setting may be appropriately changed. The reaction temperature is preferably -10°C to 50°C, for example, and more preferably 15°C to 30°C, for example.

The reaction time usually varies according to the starting material, the solvent, other reagents used in the reaction, the reaction temperature and the degree of progress of the reaction and may be appropriately increased and reduced. The reaction time at the aforementioned reaction temperature after addition of the base is preferably 6 to 24 hours, for example, and more preferably about 18 hours, for example.

When the compound obtained in this manner employs, as a starting material, the compound (3) wherein R₁ has a protecting group, it is necessary to further remove the protecting group. In order to remove the protecting group, a typical reaction of removing a protecting group may be used. As such a removing reaction, it is possible to use a deprotection reaction described in many known documents (see T.W. Green, "Protective Groups in Organic Synthesis", John Wiley & Sons, Inc., 1981, for example).

### 7) Process for producing compound represented by formula (8) (Step 1-6))

The production process is a step of reacting the compound represented by the formula (6-b) with a compound represented by the formula (7) in the presence of a base to convert the compound represented by the formula (6-b) into a compound represented by the formula (8) (hereinafter referred to as Step 1-6)).

Step 1-6) can be carried out by a generally used method described in Organophosphorus Reagents in Organic Synthesis, Edited by J.I.G. Cadogan Academic Press (1979) or the like. More specifically, this step can be carried out with reference to the reaction conditions, the operation after the reaction, the purification method and the like described in the later-described Example 17.

As the compound represented by the formula (6-b), it is possible to use a compound that can be produced by a method described in the later-described Example 17 or the like, a known compound, a commercially available compound, or a compound that can be readily produced from a commercially available compound by a method usually carried out by a person skilled in the art.

The solvent used in this reaction is not particularly limited insofar as the solvent allows the starting material to be dissolved therein to a certain extent and does not inhibit the reaction. Preferable examples of the solvent that may be used include 1,4-dioxane, tetrahydrofuran, 1,2-dimethoxyethane, *tert-*butyl methyl ether, cyclopentyl methyl ether, diethyl ether, diisopropyl ether, dibutyl ether, dicyclopentyl ether, N,N-dimethylformamide, N-methylpyrrolidone, acetonitrile, dimethyl sulfoxide, methanol, ethanol, propanol, butanol and mixed solvents thereof. More preferably, solvents such as ethanol may be used, for example.

The amount of the compound (6-b) used may be appropriately increased and reduced and is preferably 1.0 to 3.0 mole, for example, and more preferably 1.0 to 2.0 mole, for example, per mole of the compound (7).

Preferable examples of the base that may be used in this reaction include lithium hydroxide, sodium hydroxide, potassium hydroxide, lithium carbonate, sodium carbonate, potassium carbonate, cesium carbonate, sodium bicarbonate, potassium bicarbonate, triethylamine, sodium hydride, sodium ethoxide, sodium methoxide, potassium t-butoxide, sodium t-butoxide and *n*-butyllithium. The base is more preferably triethylamine, for example.

The amount of the base used may be appropriately increased and reduced and is preferably 1.0 to 5.0 mole, for example, and more preferably 2.0 to 4.0 mole, for example, per mole of the compound (6-b).

The reaction temperature usually varies according to the starting material, the solvent and other reagents used in the reaction and the setting may be appropriately changed. The reaction temperature is preferably -10°C to 100°C, for example, and more preferably 40°C to 80°C, for example.

The reaction time usually varies according to the starting material, the solvent, other reagents used in the reaction, the reaction temperature and the degree of progress of the reaction and may be appropriately increased and reduced. The reaction time at the aforementioned reaction temperature after addition of the base is preferably 6 to 24 hours, for example, and more preferably about 18 hours, for example.

When the compound obtained in this manner employs, as a starting material, the compound (3) wherein R₁ has a protecting group, it is necessary to further remove the protecting group as described above.

### 2. Production Process 2

### 1) Process for producing compound represented by formula (9) (Step 2-7))

The production process is a step of reacting a compound represented by the formula (3) with a compound represented by the formula (7) in the presence of a base to convert the compound represented by the formula (3) into a compound represented by the formula (9) (hereinafter referred to as Step 2-7)).

Step 2-7) can be carried out by a generally used method described in Shin Jikken Kagaku Koza (New Experimental Chemistry Course) 14, Yuki Kagobutsu no Gosei to Hanno (Synthesis and reaction of organic compounds) [I] (pages 511-534) or the like. More specifically, this step can be carried out with reference to the reaction conditions, the operation after the reaction, the purification method and the like described in the later-described Example 1 and Example 22.

The reaction in this step may also be carried out in a stream of an inert gas such as nitrogen or argon, for example (or in an atmosphere of nitrogen or argon, for example).

The solvent used in this reaction is not particularly limited insofar as the solvent allows the starting material to be dissolved therein to a certain extent and does not inhibit the reaction. Preferable examples of the solvent that may be used include 1,4-dioxane, tetrahydrofuran, 1,2-dimethoxyethane, tert-butyl methyl ether, cyclopentyl methyl ether, diethyl ether, diisopropyl ether, dibutyl ether, dicyclopentyl ether, benzene, toluene, xylene, *n*-hexane, *c*-hexane and mixed solvents thereof. The solvent is more preferably a mixed solvent of tetrahydrofuran and toluene, for example.

Preferable examples of the base include *n-*butyllithium, sec-butyllithium, *tert*-butyllithium and lithium amides such as lithium diisopropylamide and 2,2,6,6-tetramethylpiperidine lithium amide. The base is more preferably lithium diisopropylamide.

The amount of the compound (7) used may be appropriately increased and reduced and is preferably 0.9 to 3.0 mole, for example, and more preferably 0.9 to 1.5 mole, for example, per mole of the compound (3).

Typically, as a method of reacting the compound (3) with the compound (7) in the presence of the base, a method of treating the compound (3) with the base and then reacting the treated compound with the compound (7) is preferably used. The temperature for the reaction of the compound (3) with the base usually varies according to the starting material, the solvent and other reagents used in the reaction and the setting may be appropriately changed. The reaction temperature is preferably -100°C to 0°C, for example, and more preferably -80°C to 0°C, for example.

The time for reaction of the compound (3) with the base usually varies according to the starting material, the solvent, other reagents used in the reaction, the reaction temperature and the reaction rate and may be appropriately increased and reduced. The reaction time at the aforementioned reaction temperature after addition of the base is preferably 10 minutes to 3 hours, for example, and more preferably 30 minutes to 1 hour, for example.

The temperature for the reaction of the base-treated compound represented by the formula (3) with the compound represented by the formula (7) usually varies according to the starting material, the solvent and other reagents used in the reaction and the setting may be appropriately changed. The reaction temperature is preferably -100°C to 0°C, for example, and more preferably -80°C to 0°C, for example.

The time for reaction of the base-treated compound represented by the formula (3) with the compound represented by the formula (7) usually varies according to the starting material, the solvent, other reagents used in the reaction and the reaction temperature and the setting may be appropriately changed. The reaction time at the aforementioned temperature after addition of the base is preferably 10 minutes to 5 hours, for example, and more preferably 20 minutes to 2 hours, for example.

The compound (9) obtained in this manner is a novel compound.

### 2) Process for producing compound represented by formula (10) (Step 2-8a), Step 2-8b) and Step 2-8c))

The production process is a step of reacting the compound represented by the formula (9) having a hydroxyl group with a leaving group introduction reagent in the presence of a base if necessary to convert the compound represented by the formula (9) into a compound (10) having a leaving group X. (Hereinafter, a step of converting the compound (9) into a compound (10-a) is referred to as Step 2-8a) where X₄ₐ is a halogen atom; a step of converting the compound (9) into a compound (10-a) is referred to as Step 2-8b) where X₄ₐ is a sulfonyloxy group; and a step of converting the compound (9) into a compound (10-b) is referred to as Step 2-8c) where X_{4b} is an acyloxy group.)

Step 2-8a), Step 2-8b) and Step 2-8c) can be carried out by a generally used method described in Shin Jikken Kagaku Koza (New Experimental Chemistry Course) 14, Yuki Kagobutsu no Gosei to Hanno (Synthesis and reaction of organic compounds) [I] (pages 307-450) or the like or a generally used method described in Shin Jikken Kagaku Koza (New Experimental Chemistry Course) 14, Yuki Kagobutsu no Gosei to Hanno (Synthesis and reaction of organic compounds) [III] (pages 1793-1798), Shin Jikken Kagaku Koza (New Experimental Chemistry Course) 14, Yuki Kagobutsu no Gosei to Hanno (Synthesis and reaction of organic compounds) [II] (pages 1000-1062) or the like. More specifically, this step can be carried out with reference to the reaction conditions, the operation after the reaction, the purification method and the like described in the later-described Example 2, Example 4 and Example 20.

The case where X₄ₐ is a halogen atom (Step 2-8a)) will be described below.

As the compound represented by the formula (9), it is possible to use a compound that can be produced by a method described in the later-described Example 1 or Example 22 or the like.

The solvent used in this reaction is not particularly limited insofar as the solvent allows the starting material to be dissolved therein to a certain extent and does not inhibit the reaction. Preferable examples of the solvent include 1,4-dioxane, tetrahydrofuran, 1,2-dimethoxyethane, *tert*-butyl methyl ether, cyclopentyl methyl ether, diethyl ether, diisopropyl ether, dibutyl ether, dicyclopentyl ether, benzene, toluene, xylene and mixed solvents thereof. The solvent is more preferably 1,2-dimethoxyethane or toluene, for example.

The halogenating reagent used in this reaction is preferably chlorine, bromine, iodine, thionyl chloride or thionyl bromide, for example, and more preferably thionyl chloride, for example.

The amount of the halogenating reagent used may be appropriately increased and reduced and is preferably 1.0 to 10.0 mole, for example, and more preferably 1.0 to 3.0 mole, for example, per mole of the compound (9).

The reaction temperature usually varies according to the starting material, the solvent and other reagents used in the reaction and the setting may be appropriately changed. The reaction temperature is preferably 50°C to 0°C, for example, and more preferably 10°C to 30°C, for example.

The reaction time usually varies according to the starting material, the solvent, other reagents used in the reaction, the reaction temperature and the degree of progress of the reaction and may be appropriately increased and reduced. The reaction time at the aforementioned reaction temperature after addition of the base is preferably 1 to 10 hours, for example, and more preferably 1 to 3 hours, for example.

The case where X₄ₐ is a sulfonyloxy group (Step 2-8b)) will be described below.

The sulfonylating reagent used in this reaction is preferably methanesulfonyl chloride, p-toluenesulfonyl chloride, trifluoromethanesulfonyl, methanesulfonic anhydride, p-toluenesulfonic anhydride or trifluoromethanesulfonic anhydride, for example, and more preferably methanesulfonyl chloride, for example.

The amount of the sulfonylating reagent used may be appropriately increased and reduced and is preferably 1.0 to 10.0 mole, for example, and more preferably 1.0 to 3.0 mole, for example, per mole of the compound (9).

Preferable examples of the base used in this reaction include pyridine, triethylamine and diisopropylethylamine. The base is more preferably triethylamine, for example.

The amount of the base used may be appropriately increased and reduced and is preferably 1.0 to 3.0 mole, for example, and more preferably 1.2 to 1.6 mole, for example, per mole of the compound (6).

The reaction temperature usually varies according to the starting material, the solvent and other reagents used in the reaction and the setting may be appropriately changed. The reaction temperature is preferably 50°C to 0°C, for example, and more preferably 10°C to 30°C, for example.

The reaction time usually varies according to the starting material, the solvent, other reagents used in the reaction, the reaction temperature and the degree of progress of the reaction and may be appropriately increased and reduced. The reaction time at the aforementioned reaction temperature after addition of the base is preferably 1 to 10 hours, for example, and more preferably 1 to 5 hours, for example.

The case where X_{4b} is an acyloxy group (Step 2-8c)) will be described below.

The solvent used in this reaction is not particularly limited insofar as the solvent allows the starting material to be dissolved therein to a certain extent and does not inhibit the reaction. Preferable examples of the solvent include 1,4-dioxane, tetrahydrofuran, 1,2-dimethoxyethane, *tert*-butyl methyl ether, cyclopentyl methyl ether, diethyl ether, diisopropyl ether, dibutyl ether, dicyclopentyl ether, benzene, toluene, xylene and mixed solvents thereof. The solvent is more preferably tetrahydrofuran, toluene or a mixed solvent thereof, for example.

The acylating reagent used in this reaction is preferably acetyl chloride, acetic anhydride, propyl chloride, or acetyl bromide, for example, and more preferably acetic anhydride or acetyl chloride, for example.

The amount of the acylating reagent used may be appropriately increased and reduced and is preferably 1.0 to 10.0 mole, for example, and more preferably 1.0 to 3.0 mole, for example, per mole of the compound (9).

Preferable examples of the base used in this reaction include 4-dimethylaminopyridine, pyridine, triethylamine and diisopropylethylamine. The base is more preferably 4-dimethylaminopyridine or triethylamine, for example.

The amount of the base used may be appropriately increased and reduced and is preferably 1.0 to 3.0 mole, for example, and more preferably 1.2 to 1.6 mole, for example, per mole of the compound (6). However, the amount of 4-dimethylaminopyridine used may be appropriately increased and reduced and is preferably 0.02 to 1.0 mole, for example, and more preferably 0.05 mole, for example, per mole of the compound (9).

The reaction temperature usually varies according to the starting material, the solvent and other reagents used in the reaction and the setting may be appropriately changed. The reaction temperature is preferably 0°C to 100°C, for example, and more preferably 10°C to 50°C, for example.

The reaction time usually varies according to the starting material, the solvent, other reagents used in the reaction, the reaction temperature and the degree of progress of the reaction and may be appropriately increased and reduced. The reaction time at the aforementioned reaction temperature after addition of the base is preferably 1 to 20 hours, for example, and more preferably 1 to 5 hours, for example.

The production process is a step of converting the compound represented by the formula (9) having a hydroxyl group into a compound (10) having a leaving group X. The case where X₄ₐ is a halogen atom (Step 2-8a)), the case where X₄ₐ is a sulfonyloxy group (Step 2-8b)) and the case where X_{4b} is an acyloxy group (Step 2-8c)) are described as examples; however, the leaving group X is not limited to a halogen atom, a sulfonyloxy group and an acyloxy group. The hydroxyl group can be converted into various leaving groups using the reaction reagents and the reaction conditions described in Yuki Kagobutsu no Gosei to Hanno (Synthesis and reaction of organic compounds) [1] (pages 114-157) or the like.

Among the compounds (10) obtained in this manner, the compound (10-a) represented by the formula (10-a), the compound (10-b) represented by the formula (10-b) and the compound (10-c) represented by the formula (10-c) are novel compounds.

### 3) Process for producing compound represented by formula (8) (Step 2-9))

The production process is a step of treating the compound represented by the formula (10) (which refers to the compound (10-a) where X₄ₐ is a halogen atom or a sulfonyloxy group and refers to the compound (10-b) where X_{4b} is an acyloxy group, for example) with a base to convert the compound represented by the formula (10) into a compound (8) (hereinafter referred to as Step 2-9)).

Step 2-9) can be carried out by a generally used method described in Shin Jikken Kagaku Koza (New Experimental Chemistry Course) 14, Yuki Kagobutsu no Gosei to Hanno (Synthesis and reaction of organic compounds) [I] (pages 114-157) or the like. More specifically, this step can be carried out with reference to the reaction conditions, the operation after the reaction, the purification method and the like described in the later-described Example 3, Example 4 or Example 20.

The compound represented by the formula (10) (which refers to the compound (10-a) where X₄ₐ is a halogen atom or a sulfonyloxy group and refers to the compound (10-b) where X_{4b} is an acyloxy group, for example) can be produced by a method described in the later-described Example 2, Example 4 or Example 20 or the like.

The solvent used in this reaction is not particularly limited insofar as the solvent allows the starting material to be dissolved therein to a certain extent and does not inhibit the reaction. Preferable examples of the solvent include 1,4-dioxane, tetrahydrofuran, tetrahydropyran, 1,2-dimethoxyethane, *tert*-butyl methyl ether, cyclopentyl methyl ether, diethyl ether, diisopropyl ether, dibutyl ether, dicyclopentyl ether, benzene, toluene, xylene and mixed solvents thereof. The solvent is more preferably tetrahydrofuran or toluene, for example.

Preferable examples of the base used in this reaction include lithium hydroxide, sodium hydroxide, potassium hydroxide, lithium carbonate, sodium carbonate, potassium carbonate, cesium carbonate, sodium bicarbonate, potassium bicarbonate, cesium carbonate chlorine, imidazole, pyridine, 4-dimethylaminopyridine, 1,4-diazabicyclo[2.2.2]octane, triethylamine, N,N-diisopropylethylamine, sodium methoxide, sodium ethoxide, potassium methoxide, potassium ethoxide, sodium tert-butoxide, potassium tert-butoxide and 1,8-diazabicyclo[5.4.0]unde-7-cene. More preferable examples of the base include sodium methoxide, sodium ethoxide, potassium methoxide, potassium ethoxide, sodium tert-butoxide, potassium tert-butoxide and 1,8-diazabicyclo[5.4.0]unde-7-cene.

The amount of the base used may be appropriately increased and reduced and is preferably 1.0 to 10.0 mole, for example, and more preferably 1.0 to 4.0 mole, for example, per mole of the compound (10).

The reaction temperature usually varies according to the starting material, the solvent and other reagents used in the reaction and the setting may be appropriately changed. The reaction temperature is preferably -40°C to 100°C, for example, and more preferably -30°C to 80°C, for example.

The reaction time usually varies according to the starting material, the solvent, other reagents used in the reaction, the reaction temperature and the degree of progress of the reaction and may be appropriately increased and reduced. The reaction time at the aforementioned reaction temperature after addition of the base is preferably 10 minutes to 5 hours, for example, and more preferably 10 minutes to 3 hours, for example.

The compound represented by the formula (8) can also be derived from the compound represented by the formula (9) by reacting the compound represented by the formula (9) with a leaving group introduction reagent such as a halogenating reagent, a sulfonylating reagent or an acylating reagent in the presence of a base if necessary, and treating the resulting compound represented by the formula (10) (which refers to the compound (10-a) where X₄ₐ is a halogen atom or a sulfonyloxy group and refers to the compound (10-b) where X_{4b} is an acyloxy group, for example) with a base without isolation.

When the compound obtained in this manner employs, as a starting material, the compound (3) wherein R₁ has a protecting group, it is necessary to further remove the protecting group as described in Step 1-5) of Production Process 1.

### 3. Production Process 3

### 1) Process for producing compound represented by formula (21) (Step 3-10))

The production process is a step of reacting a compound represented by the formula (3) with a compound represented by the formula (20) in the presence of a base to convert the compound represented by the formula (3) into a compound represented by the formula (21) (hereinafter referred to as Step 3-10)).

Step 3-10) can be carried out by a generally used method described in Shin Jikken Kagaku Koza (New Experimental Chemistry Course) 14, Yuki Kagobutsu no Gosei to Hanno (Synthesis and reaction of organic compounds) [I] (pages 511-534) or the like. More specifically, this step can be carried out with reference to the reaction conditions, the operation after the reaction, the purification method and the like described in the later-described Examples 18 and 19.

As the compound represented by the formula (20), it is possible to use a compound that can be produced by a method described in Reference Example 1 or Example 19, for example. Among the compounds (20), a compound (10-a) represented by the formula (20-a) is a novel compound.

The solvent used in this reaction is not particularly limited insofar as the solvent allows the starting material to be dissolved therein to a certain extent and does not inhibit the reaction. Preferable examples of the solvent include 1,4-dioxane, tetrahydrofuran, 1,2-dimethoxyethane, *tert*-butyl methyl ether, cyclopentyl methyl ether, diethyl ether, diisopropyl ether, dibutyl ether, dicyclopentyl ether, benzene, toluene, xylene, *n*-hexane, *c*-hexane and mixed solvents thereof. The solvent is more preferably a mixed solvent of tetrahydrofuran and toluene, for example.

Preferable examples of the base include *n-*butyllithium, sec-butyllithium, *tert*-butyllithium and lithium amides such as lithium diisopropylamide and 2,2,6,6-tetramethylpiperidine lithium amide. The base is more preferably lithium diisopropylamide.

The amount of the compound (20) used may be appropriately increased and reduced and is preferably 0.9 to 3.0 mole, for example, and more preferably 0.9 to 1.5 mole, for example, per mole of the compound (3).

Typically, as a method of reacting the compound (3) with the compound (20) in the presence of the base, a method of treating the compound (3) with the base and then reacting the treated compound with the compound (20) is preferably used. The temperature for the reaction of the compound (3) with the base usually varies according to the starting material, the solvent and other reagents used in the reaction and the setting may be appropriately changed. The reaction temperature is preferably -80°C to 0°C, for example, and more preferably -70°C to -30°C, for example.

The time for reaction of the compound (3) with the base usually varies according to the starting material, the solvent, other reagents used in the reaction, the reaction temperature and the reaction rate and may be appropriately increased and reduced. The reaction time at the aforementioned reaction temperature after addition of the base is preferably 10 minutes to 3 hours, for example, and more preferably 20 minutes to 1 hour, for example.

The temperature for the reaction of the base-treated compound represented by the formula (3) with the compound represented by the formula (20) usually varies according to the starting material, the solvent and other reagents used in the reaction and the setting may be appropriately changed. The reaction temperature is preferably -100°C to 0°C, for example, and more preferably -80°C to -30°C, for example.

The time for reaction of the base-treated compound represented by the formula (3) with the compound represented by the formula (20) usually varies according to the starting material, the solvent, other reagents used in the reaction and the reaction temperature and the setting may be appropriately changed. The reaction time at the aforementioned temperature after addition of the base is preferably 10 minutes to 5 hours, for example, and more preferably 20 minutes to 2 hours, for example.

The compound (21) obtained in this manner is a novel compound.

### 2) Process for producing compound represented by formula (9) (Step 3-11))

The production process is a step of treating the compound represented by the formula (21) with a reducing agent to convert the compound represented by the formula (21) into a compound represented by the formula (9) (hereinafter referred to as Step 3-11)).

Step 3-11) can be carried out by a generally used method described in Shin Jikken Kagaku Koza (New Experimental Chemistry Course) 15, Sanka to Kangen (Oxidation and reduction) [II] (pages 29-332), Shin Jikken Kagaku Koza (New Experimental Chemistry Course) 14, Yuki Kagobutsu no Gosei to Hanno (Synthesis and reaction of organic compounds) [I] (pages 461-484) or the like. More specifically, this step can be carried out with reference to the reaction conditions, the operation after the reaction, the purification method and the like described in the later-described Example 18.
The reaction in this step is preferably carried out in the presence of a solvent. The reaction may also be carried out in a stream of an inert gas such as nitrogen or argon, for example (or in an atmosphere of nitrogen or argon, for example).

The compound represented by the formula (21) can be produced by a method described in the later-described Example 18 or Example 19 or the like.

The solvent used in this reaction is not particularly limited insofar as the solvent allows the starting material to be dissolved therein to a certain extent and does not inhibit the reaction. Preferable examples of the solvent that may be used include alcohol solvents such as isopropyl alcohol, ethanol and methanol; ether solvents such as tetrahydrofuran, 1,2-dimethoxyethane, diethyl ether and dioxane; hydrocarbon solvents such as benzene, toluene and hexane; and mixed solvents thereof. The solvent is more preferably tetrahydrofuran, isopropyl alcohol, benzene or toluene, for example.

The reducing reagent refers to a metal hydride such as dialkylaluminum hydride, boron hydride alkali metal salt or aluminum hydride alkal metal salt; or a boron compound such as borane or alkylborane, for example. Preferable examples of the reducing reagent may include diisobutylaluminum hydride, sodium borohydride, lithium aluminum hydride and diborane.
The reducing agent is more preferably sodium borohydride, for example.

The amount of the reducing agent used may be appropriately increased and reduced and is preferably 0.5 to 10.0 mole, for example, and more preferably 0.5 to 1.5 mole, for example, per mole of the compound (21).

The temperature for the reaction of the compound (21) with the reducing agent usually varies according to the starting material, the solvent and other reagents used in the reaction and the setting may be appropriately changed. The reaction temperature is preferably -40°C to 40°C, for example, and more preferably 0°C to 30°C, for example.

The time for reaction of the compound (21) with the reducing agent usually varies according to the starting material, the solvent, other reagents used in the reaction, the reaction temperature and the reaction rate and may be appropriately increased and reduced. The reaction time at the aforementioned reaction temperature after addition of the reducing agent is preferably 10 minutes to 24 hours, for example, and more preferably 20 minutes to 13 hours, for example.

The compound represented by the formula (9) which is obtained in the above step can be converted into a compound (8) by converting the compound represented by the formula (9) into a compound represented by the formula (10) (which refers to a compound (10-a) where X₄ₐ is a halogen atom or a sulfonyloxy group and refers to a compound (10-b) where X_{4b} is an acyloxy group, for example) in Step 2-8a), Step 2-8b) or Step 2-8c) described in the aforementioned Production Process 2, and then treating the compound represented by the formula (10) with a base according to the aforementioned Step 2-9).

When the compound obtained in this manner employs, as a starting material, the compound (3)
wherein R₁ has a protecting group, it is necessary to further remove the protecting group as described in Step 1-5) of Production Process 1.

### 4. Production Process 4

### 1) Process for producing compound represented by formula (12) (Step 4-12))

The production process is a step of reacting a compound represented by the formula (11) with a compound represented by the formula (1) in the presence of a reducing agent to convert the compound represented by the formula (11) into a compound represented by the formula (12) (hereinafter referred to as Step 4-12)).

Step 4-12) can be carried out by a generally used method described in Shin Jikken Kagaku Koza (New Experimental Chemistry Course) 14, Yuki Kagobutsu no Gosei to Hanno (Synthesis and reaction of organic compounds) [III] (pages 1380-1387) or the like. More specifically, this step can be carried out with reference to the reaction conditions, the operation after the reaction, the purification method and the like described in the later-described Example 11.

As the compound represented by the formula (11), it is possible to use a known compound, a commercially available compound, or a compound that can be readily produced from a commercially available compound by a method usually carried out by a person skilled in the art.

The solvent used in the reaction is not particularly limited insofar as the solvent allows the starting material to be dissolved therein to a certain extent and does not inhibit the reaction. Preferable specific examples of the solvent include ether solvents such as tetrahydrofuran, 1,2-dimethoxyethane diethyl ether and dioxane; hydrocarbon solvents such as benzene, toluene and hexane; ethyl acetate, methanol and mixed solvents thereof. The solvent is more preferably tetrahydrofuran, benzene or toluene, for example.

The amount of the compound (1) is preferably 1.0 to 5.0 mole, for example, and more preferably 1.0 to 1.5 mole, for example, per mole of the compound (11).

Preferable examples of the reducing agent may include diisobutylaluminum hydride, cyanoborohydride, α-picolineborane, sodium borohydride, sodium triacetoxyborohydride and lithium aluminum hydride.
The reducing agent is more preferably cyanoborohydride or sodium triacetoxyborohydride, for example.

The amount of the reducing agent used may be appropriately increased and reduced and is preferably 1.0 to 10.0 mole, for example, and more preferably 1.0 to 3.0 mole, for example, per mole of the compound (11) .

The reaction temperature is not particularly limited; however, the temperature usually varies according to the starting material, the solvent and other reagents used in the reaction and the setting may be appropriately changed. The reaction temperature is preferably 0°C to 80°C, for example, and more preferably 10°C to 30°C, for example.

The reaction time is not particularly limited but usually varies according to the starting material, the solvent, other reagents used in the reaction, the reaction temperature and the degree of progress of the reaction and may be appropriately increased and reduced. The reaction time at the aforementioned reaction temperature after addition of the reagent is preferably 1 to 10 hours, for example, and more preferably about 2 hours, for example.

The reductive amination reaction can be carried out in a buffer in the presence of a catalyst such as acetic acid or ammonium acetate if necessary.

### 2) Process for producing compound represented by formula (13) (Step 4-13))

The production process is characterized by a step of heating the compound represented by the formula (12) in the coexistence of an acid to convert the compound represented by the formula (12) into a compound (13) (hereinafter referred to as Step 4-13)).

Step 4-13) can be carried out by a generally used method described in Shin Jikken Kagaku Koza (New Experimental Chemistry Course) 14, Yuki Kagobutsu no Gosei to Hanno (Synthesis and reaction of organic compounds) [II] (pages 1134-1220) or the like. More specifically, this step can be carried out with reference to the reaction conditions, the operation after the reaction, the purification method and the like described in the later-described Example 12.

As the compound represented by the formula (12), it is possible to use a compound that can be produced by a method described in the later-described Example 11 or the like, a known compound, a commercially available compound, or a compound that can be readily produced from a commercially available compound by a method usually carried out by a person skilled in the art.

The solvent used in the heating reaction is not particularly limited insofar as the solvent allows the starting material to be dissolved therein to a certain extent and does not inhibit the reaction. Preferable specific examples of the solvent include 1,4-dioxane, tetrahydrofuran, 1,2-dimethoxyethane, *tert*-butyl methyl ether, cyclopentyl methyl ether, diethyl ether, diisopropyl ether, dibutyl ether, dicyclopentyl ether, *N,N*-dimethylformamide, *N-*methylpyrrolidone, acetonitrile, dimethyl sulfoxide, benzene, toluene, xylene and acetic acid. The solvent is more preferably acetic acid, for example.

The reaction temperature is not particularly limited; however, the temperature usually varies according to the starting material, the solvent and other reagents used in the reaction and the setting may be appropriately changed. The reaction temperature is preferably 50°C to 200°C, for example, and more preferably 80°C to 150°C, for example.

The reaction time is not particularly limited but usually varies according to the starting material, the solvent, other reagents used in the reaction, the reaction temperature and the degree of progress of the reaction and may be appropriately increased and reduced. More preferably, the reaction time at the aforementioned reaction temperature after addition of the reagent is preferably 1 to 24 hours, for example, and more preferably about 15 hours, for example.

Among the compounds (13) obtained in this manner, a compound (18) represented by the formula (18) is a novel compound.

### 3) Process for producing compound represented by formula (14) (Step 4-14))

The production process is a step of hydrolyzing the compound represented by the formula (13) in the presence of a base to convert the compound represented by the formula (13) into a compound (14) (hereinafter referred to as Step 4-14)).

Step 4-14) can be carried out by a generally used method described in Shin Jikken Kagaku Koza (New Experimental Chemistry Course) 14, Yuki Kagobutsu no Gosei to Hanno (Synthesis and reaction of organic compounds) [II] (pages 921-1000) or the like. More specifically, this step can be carried out with reference to the reaction conditions, the operation after the reaction, the purification method and the like described in the later-described Example 12.

The compound represented by the formula (13) can be produced by a method described in the later-described Example 11 or the like.

The solvent used in the reaction is not particularly limited insofar as the solvent allows the starting material to be dissolved therein to a certain extent and does not inhibit the reaction. Preferable specific examples of the solvent include methanol, ethanol, propanol, 1,4-dioxane, tetrahydrofuran, 1,2-dimethoxyethane, *tert*-butyl methyl ether, cyclopentyl methyl ether, diethyl ether, diisopropyl ether, dibutyl ether, dicyclopentyl ether, acetonitrile, water and mixed solvents thereof. The solvent is more preferably methanol, ethanol or a mixed solvent of water and methanol or ethanol, for example.

Preferable examples of the base that may be used in this reaction include lithium hydroxide, sodium hydroxide, potassium hydroxide, lithium carbonate, sodium carbonate, potassium carbonate, cesium carbonate, sodium bicarbonate, potassium bicarbonate, cesium carbonate chlorine, imidazole, pyridine, 4-dimethylaminopyridine, triethylamine, N,N-diisopropylethylamine, sodium methoxide, sodium ethoxide, potassium methoxide and potassium ethoxide. The base is more preferably sodium hydroxide or potassium hydroxide, for example.

The amount of the base is preferably 1.0 to 10.0 mole, for example, and more preferably 1.0 to 5.0 mole, for example, per mole of the compound (13).

The reaction temperature is not particularly limited; however, the temperature usually varies according to the starting material, the solvent and other reagents used in the reaction and the setting may be appropriately changed. The reaction temperature is preferably 10°C to 50°C, for example, and more preferably 10°C to 30°C, for example.

The reaction time is not particularly limited but usually varies according to the starting material, the solvent, other reagents used in the reaction, the reaction temperature and the degree of progress of the reaction and may be appropriately increased and reduced. The reaction time at the aforementioned reaction temperature after addition of the reagent is preferably 1 to 24 hours, for example, and more preferably about 2 hours, for example.

Among the compounds (14) obtained in this manner, a compound (19) represented by the formula (19) is a novel compound.

### 4) Process for producing compound represented by formula (8) (Step 4-15))

The production process is a step of reacting the compound represented by the formula (14) with a compound represented by the formula (7) in the presence of a base to convert the compound represented by the formula (14) into a compound represented by the formula (8) (hereinafter referred to as Step 4-15)).

Step 4-15) can be carried out by a generally used method described in Org. React. 1, 210 (1492) J.R. Johnson, Modern Synthetic Reactions H.O. House (W.A. Benjamin, Menlo Park, California, 2nd ed., 1972) or the like. More specifically, this step can be carried out with reference to the reaction conditions, the operation after the reaction, the purification method and the like described in the later-described Example 13.

The compound represented by the formula (14) can be produced by a method described in the later-described Example 12 or the like.

The compound represented by the formula (7) can be produced by a method described in Reference Example 1, for example.

The solvent used in the reaction is not particularly limited insofar as the solvent allows the starting material to be dissolved therein to a certain extent and does not inhibit the reaction. Preferable specific examples of the solvent that can be used include methanol, ethanol, propanol, 1,4-dioxane, tetrahydrofuran, 1,2-dimethoxyethane, *tert*-butyl methyl ether, cyclopentyl methyl ether, diethyl ether, diisopropyl ether, dibutyl ether, dicyclopentyl ether, N,N-dimethylformamide, N-methylpyrrolidone, acetonitrile, dimethyl sulfoxide and pyridine. The solvent is more preferably pyridine, for example.

The base used in this reaction may be preferably pyrrolidine, piperidine or pyridine, for example.

The amount of the base is preferably 1.0 to 10.0 mole, for example, and more preferably 1.0 to 3.0 mole, for example, per mole of the compound (14).

The reaction temperature usually varies according to the starting material, the solvent and other reagents used in the reaction and the setting may be appropriately changed. The reaction temperature is preferably 50°C to 150°C, for example, and more preferably 50°C to 80°C, for example.

The reaction time is not particularly limited but usually varies according to the starting material, the solvent, other reagents used in the reaction, the reaction temperature and the degree of progress of the reaction and may be appropriately increased and reduced. The reaction time at the aforementioned reaction temperature after addition of the reagent is preferably 1 to 48 hours, for example, and more preferably 1 to 24 hours, for example.

When the compound obtained in this manner employs, as a starting material, the compound (12) wherein R₁ has a protecting group, it is necessary to further remove the protecting group as described in Step 1-5) of Production Process 1.

According to the present invention, a compound represented by the formula (8): 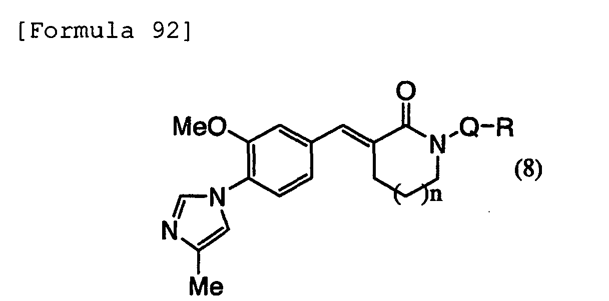 wherein R represents a 0 to14 membered aromatic hydrocarbon ring group which may have a substituent or a 5- to 14-membered aromatic heterocyclic group which may have a substituent, Q represents a single bond or - CH(Y)- (wherein Y represents a hydrogen atom or a C1-6 alkyl group) and n represents 0 to 2, or a solvate thereof can be produced in a high yield. The present invention also provides synthetic intermediates for producing the compound of the formula (8) in a high yield and a process for producing the same.

### BEST MODE FOR CARRYING OUT THE INVENTION

The present invention will now be described in detail with reference to examples; however, the examples are provided only for illustration purposes. The production process of the present invention is not limited to the following specific examples in any cases. A person skilled in the art can fully implement the present invention by making various modifications to not only the following examples but also the claims of the present specification, and such modifications are within the scope of the claims of the present specification.

### Reference Example 1

### Synthesis of 3-methoxy-4-(4-methyl-1H-imidazol-1-yl)benzaldehyde

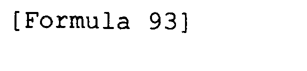

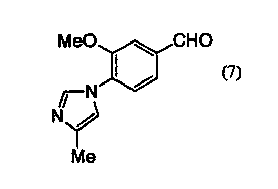

### 1) Synthesis of 3-methoxy-4-nitrobenzoic acid methyl ester

Methyl iodide (463 g) was added dropwise to a mixture of 3-hydroxy-4-nitrobenzoic acid (199 g) and potassium carbonate (450 g) in DMF (1 L) at room temperature. The reaction solution was stirred at room temperature overnight and then methyl iodide (230 g) was added to the reaction solution. The reaction solution was further stirred at room temperature for six hours. The reaction solution was added to ice water and the precipitated solid was collected by filtration. The resulting solid was dried at 50°C overnight to provide 178 g of the title compound. The property values corresponded to the reported values (CAS #5081-37-8).

### 2) Synthesis of 4-amino-3-methoxybenzoic acid methyl ester

10% palladium-carbon (containing 50% water, 15 g) was added to a solution of 3-methoxy-4-nitrobenzoic acid methyl ester (150 g) in methanol (600 mL) and THF (300 mL) and the reaction solution was stirred at a hydrogen pressure of 0.9 MPa at 50°C to 64°C for 6.5 hours. The reaction solution was allowed to cool to room temperature and then filtered through a celite pad. The resulting filtrate was concentrated under reduced pressure to provide 134 g of the title compound. The property values corresponded to the reported values (CAS #41608-64-4).

### 3) Synthesis of 4-formylamino-3-methoxybenzoic acid methyl ester

Acetic anhydride (268 mL) was added dropwise to formic acid (401 mL) at room temperature and the reaction solution was stirred at room temperature for 40 minutes. A solution of 4-amino-3-methoxybenzoic acid methyl ester (134 g) in THF (600 mL) was added dropwise to the reaction solution at room temperature and the reaction solution was stirred for one hour. To the reaction solution was added 3.8 L of ice water, and the precipitated solid was collected by filtration and further washed with water (2 L). The resulting solid was dried at 50°C overnight to provide 111 g of the title compound. The property values corresponded to the reported values (CAS #700834-18-0).

### 4) Synthesis of 4-[formyl-(2-oxopropyl)amino]-3-methoxybenzoic acid methyl ester

Chloroacetone (84.5 mL) was added dropwise to a mixture of 4-formylamino-3-methoxybenzoic acid methyl ester (111 g), cesium carbonate (346 g) and potassium iodide (8.78 g) in DMF (497 mL) at room temperature and the reaction solution was stirred for three hours. Cesium carbonate (173 g) and chloroacetone (42.0 mL) were added to the reaction solution, which was then stirred at room temperature for two hours. Ice water and ethyl acetate were added to the reaction solution and the organic layer was separated. Ethyl acetate was added to the aqueous layer and the organic layer was separated. The organic layers were combined and washed with water and brine in this order. The resulting organic layers were dried over anhydrous magnesium sulfate and then concentrated under reduced pressure. The residue was diluted with toluene and the solution was concentrated under reduced pressure. *tert*-Butyl methyl ether and heptane were added to the resulting residue. The precipitated solid was collected by filtration and washed with a solution of 50% *tert*-butyl methyl ether in heptane. The resulting solid was air-dried overnight to provide 118 g of the title compound. ¹H-NMR (400 MHz, CDCl₃) δ (ppm): 2.19 (s, 3H), 3.91 (s, 3H), 3.94 (s, 3H), 4.49 (s, 2H), 7.31 (d, J = 8.0 Hz, 1H), 7.63 (d, J = 2.0 Hz, 1H), 7.69 (dd, J = 8.0, 2.0 Hz, 1H), 8.33 (s, 1H).

### 5) Synthesis of 3-methoxy-4-(4-methyl-1H-imidazol-1-yl)benzoic acid methyl ester

A solution of 4-[formyl-(2-oxopropyl)amino]-3-methoxybenzoic acid methyl ester (118 g) and ammonium acetate (172 g) in acetic acid (255 mL) was heated and stirred at 140°C for one hour. After completion of the reaction, the reaction solution was neutralized with aqueous ammonia under ice-cooling. Ethyl acetate was added to the reaction solution and the organic layer was separated. The resulting organic layer was dried over anhydrous magnesium sulfate and then filtered through a silica gel pad. The filtrate was concentrated under reduced pressure. *tert*-Butyl methyl ether and heptane were added to the residue. The precipitated solid was collected by filtration and washed with a solution of 50% *tert*-butyl methyl ether in heptane. The resulting solid was air-dried overnight to provide 68.4 g of the title compound. Further, the crystallization mother liquor was concentrated under reduced pressure. The residue was purified by silica gel column chromatography (elution solvent: heptane-ethyl acetate system) to provide 22.3 g of the title compound.
¹H-NMR (400 MHz, CDCl₃) δ (ppm): 2.30 (s, 3H), 3.94 (s, 3H), 3.96 (s, 3H), 6.98 (brs, 1H), 7.32 (d, J = 8.4 Hz, 1H), 7.71-7.73 (m, 2H), 7.79 (brs, 1H).

### Synthesis of 3-methoxy-4-(4-methyl-1H-imidazol-1-yl)benzaldehyde

A solution of pyrrolidine (18 mL) in THF (45 mL) was added dropwise to a solution of sodium bis(2-methoxyethoxy)aluminum hydride (65% solution in toluene, 56 mL) in THF (60 mL) at -5°C or less over 15 minutes. The reaction solution was stirred at room temperature for one hour. Then, a suspension of potassium tert-butoxide (2.10 g) in THF (15 mL) was added dropwise to the reaction solution at room temperature and the reaction solution was stirred for 15 minutes. The above reaction solution was added dropwise to a solution of 3-methoxy-4-(4-methyl-1*H-*imidazol-1-yl)benzoic acid methyl ester (20 g) in THF (50 mL) under ice-cooling over 30 minutes. The reaction solution was stirred at room temperature for two hours and then a 5 N sodium hydroxide solution (150 mL) was added dropwise to the reaction solution. Ethyl acetate was added to the reaction solution and the organic layer was separated. The organic layer was washed with a saturated ammonium chloride solution and brine in this order. The organic layer was dried over anhydrous magnesium sulfate and filtered through a silica gel pad. Then, the filtrate was concentrated under reduced pressure. The residue was diluted with ethyl acetate and the precipitated solid was collected by filtration. The resulting solid was air-dried overnight to provide 7.10 g of the title compound. Further, the crystallization mother liquor was concentrated under reduced pressure. The residue was purified by silica gel column chromatography (elution solvent: heptane-ethyl acetate-2-propanol system) to provide 2.65 g of the title compound.
¹H-NMR (600 MHz, CDCl₃) δ (ppm): 2.32 (s, 3H), 3.96 (s, 3H), 7.01 (s, 1H), 7.44 (d, J = 8 Hz, 1H), 7.55 (dd, J = 8, 2 Hz, 1H), 7.58 (d, J = 2 Hz, 1H), 7.86 (s, 1H), 10.00 (s, 1H).

### Example 1

### Synthesis of 1-[1-(4-fluorophenyl)ethyl]-3-{hydroxy[3-methoxy-4-(4-methylimidazol-1-yl)phenyl]methyl}piperidin-2-one

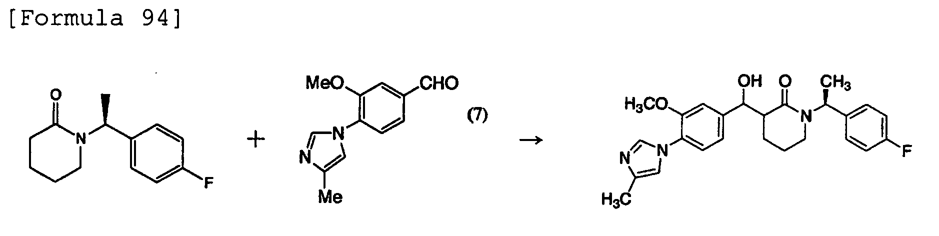

A mixed solution of toluene (86 mL) and tetrahydrofuran (5.8 mL) containing diisopropylamine (10.1 mL, 71 mol, 1.58 equivalents) was cooled in a dry ice bath in a nitrogen atmosphere. *n-*Butyllithium (2.67 M solution in cyclohexane, 25.4 mL, 68 mmol, 1.5 equivalents) was added dropwise to the diisopropylamine solution at -20°C. After completion of the dropwise addition, the reaction solution was cooled to -70°C or less and stirred for 30 minutes. Then, a solution of 1-[1-(4-fluorophenyl)ethyl]piperidin-2-one (10 g, 45.2 mol) in toluene (50 mL) was added dropwise to the reaction mixture over 30 minutes. A solution of 3-methoxy-4-(4-methylimidazol-1-yl)benzaldehyde (9.75 g, 45.1 mmol, 1.0 equivalents) in tetrahydrofuran (120 mL) was added dropwise to the reaction mixture over 30 minutes. After completion of the dropwise addition, the temperature of the cooling bath was raised to about -40°C and then water (20 mL) was added to quench the reaction. Then, the reaction solution was further heated to room temperature. Water (30 ml) was added to the reaction mixture which was then transferred to a separating device and the aqueous layer was discarded. 2 N hydrochloric acid was added to the organic layer to adjust the aqueous layer to pH 1.0 and the aqueous layer was separated. The aqueous layer was adjusted to pH 10.0 with tetrahydrofuran and a 2 N sodium hydroxide solution and then the aqueous layer was discarded. Toluene was added to the organic layer. The organic layer was sequentially washed with 5% saline and water and then dried over anhydrous magnesium sulfate. The solvent was concentrated under reduced pressure to provide 19.186 g of a crude product as a dark brown oil.
The property values (NMR) of the four isomers (1) to (4) are as follows.
(1) ¹H-NMR (400 MHz, CDCl₃) δ (ppm): 7.67 (d, J = 1.6 Hz, 1H), 7.32-7.28 (m, 2H), 7.20 (d, J = 7.6 Hz, 1H), 7.14 (d, J = 1.6 Hz, 1H), 7.06 (dd, J = 8.8, 8.8 Hz, 2H), 6.98 (dd, J = 8.0, 1.2 Hz, 1H), 6.89 (t, J = 1.2 Hz, 1H), 6.52 (brs, 1H), 6.12 (q, J = 7.2 Hz, 1H), 4.77 (d, J = 9.6 Hz, 1H), 3.86 (s, 3H), 3.15-3.09 (m, 1H), 2.76-2.69 (m, 1H), 2.60-2.53 (m, 1H), 2.29 (d, J = 1.6 Hz, 3H), 1.70-1.51 (m, 2H), 1.52 (d, J = 7.2 Hz, 3H), 1.42-1.34 (m, 1H), 1.28-1.18 (m, 1H).
(2) ¹H-NMR (400 MHz, CDCl₃) δ (ppm): 7.70 (d, J = 1.2 Hz, 1H), 7.20 (d, J = 8.0 Hz, 1H), 7.16-7.12 (m, 3H), 7.00-6.93 (m, 3H), 6.91 (t, J = 1.2 Hz, 1H), 6.07 (q, J = 7.2 Hz, 1H), 5.33 (d, J = 4.2 Hz, 1H), 4.67 (brs, 1H), 3.84 (s, 3H), 3.04-2.99 (m, 1H), 2.89-2.84 (m, 1H), 2.64-2.57 (m, 1H), 2.31 (d, J = 0.8 Hz, 3H), 1.66-1. 53 (m, 3H), 1. 51 (d, *J* = 7.2Hz, 3H).
(3) ¹H-NMR (400 MHz, CDCl₃) δ (ppm): 7.68 (d, J = 0.8 Hz, 1H), 7.30-7.25 (m, 2H), 7.23 (d, J = 8.0 Hz, 1H), 7.15 (d, J = 1.2 Hz, 1H), 7.07-7.02 (m, 2H), 6.99 (dd, J = 8.0, 1.2 Hz, 1H), 6.90 (t, J = 1.2 Hz, 1H), 6.65 (brs, 1H), 6.13 (q, J = 7.2 Hz, 1H), 4.79 (d, J = 9.6 Hz, 1H), 3.88 (s, 3H), 3.17-3.10 (m, 1H), 2.91-2.87 (m, 1H), 2.61-2.54 (m, 1H), 2.29 (s, 3H), 1.78-1.25 (m, 3H), 1.55 (d, J = 7.2 Hz, 3H).
(4) ¹H-NMR (400 MHz, CDCl₃) δ (ppm): 7.69 (d, J = 1.2 Hz, 1H), 7.28-7.25 (m, 2H), 7.21 (d, J = 8.0 Hz, 1H), 7.12 (d, J = 1.6 Hz, 1H), 7.03 (dd, J = 8.8, 8.8 Hz, 2H), 6.98 (dd, J = 8.0, 1.2 Hz, 1H), 6.91 (t, J = 1.2 Hz, 1H), 6.12 (q, J = 7.2 Hz, 1H), 5.34 (d, J = 3.6 Hz, 1H), 3.86 (s, 3H), 3.03-2.96 (m, 1H), 2.90-2.79 (m, 2H), 2.30 (d, J = 1.2 Hz, 3H), 1.76-1.43 (m, 4H), 1.46 (d, J = 7.2 Hz, 3H).

### Example 2

### Synthesis of 3-{chloro-[3-methoxy-4-(4-methylimidazol-1-yl)phenyl]methyl}-1-(4-fluorobenzyl)piperidin-2-one

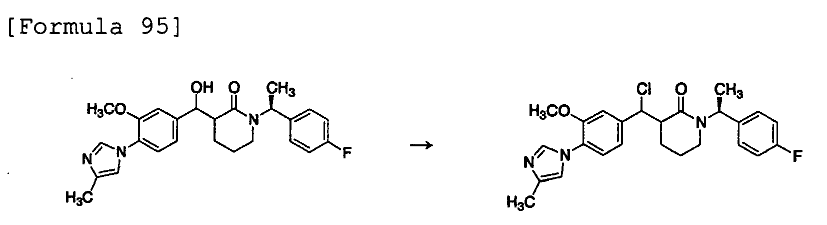

A solution of 1-[1-(4-fluorophenyl)ethyl]-3-{hydroxy[3-methoxy-4-(4-methylimidazol-1-yl)phenyl]methyl}piperidin-2-one (19.1 g) in dimethoxyethane (70 mL) was added dropwise to a solution of thionyl chloride (4.58 mL, 62.7 mol, 2 equivalents) in dimethoxyethane (70 mL) in a nitrogen atmosphere at 10°C to 23°C over 10 minutes. The reaction solution was stirred at 20°C for two hours and then cooled in an ice water bath and toluene (140 mL) was added to the reaction mixture. Subsequently, a 2 N sodium hydroxide solution (140 mL) was added to the reaction mixture. The reaction solution was transferred to a separating funnel and the aqueous layer was discarded. The organic layer was sequentially washed with 5% saline (66 mL x 2) and water (5 mL) and dried over anhydrous magnesium sulfate. The solvent was concentrated under reduced pressure to provide 22.1 g of a crude product as a brown oil.
Alternatively, the title compound was obtained as follows. Thionyl chloride (538 µL, 7.36 mol, 2 equivalents) was added dropwise to a solution of 1-[1-(4-fluorophenyl)ethyl]-3-{hydroxy[3-methoxy-4-(4-methylimidazol-1-yl)phenyl]methyl}piperidin-2-one (1.6 g, 3.68 mmol) in dimethoxyethane (16 mL) at 5°C to 10°C. The mixture was stirred for 1.5 hours and then poured into an ice-cooled saturated sodium bicarbonate solution, followed by extraction with ethyl acetate. Thereafter, the extract was washed with water and brine, dried over anhydrous magnesium sulfate and then filtered and concentrated under reduced pressure to provide 1.68 g of the title compound.
¹H-NMR (400 MHz, CDCl₃) δ (ppm): 1.30-2.10 (m, 7H), 2.23-2.35 (m, 3H), 2.60-3.30 (m, 3H), 3.80-3.90 (m, 3H), 5.90-6.28 (m, 2H), 6.80-7.38 (m, 8H), 7.66-7.76 (m, 1H).

### Example 3

### Synthesis of (3E)-1-[(1S)-1-(4-fluorophenyl)ethyl]-3-[3-methoxy-4-(4-methyl-1H-imidazol-1-yl)benzylidene]piperidin-2-one

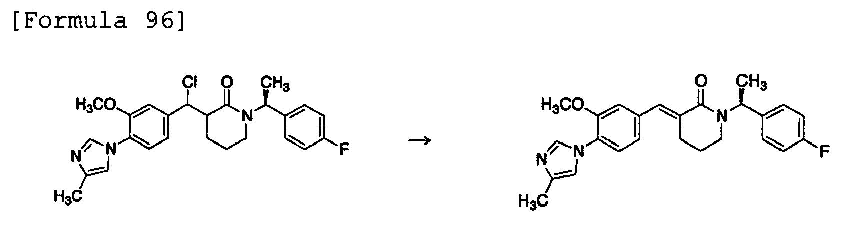

A solution of 3-{chloro-[3-methoxy-4-(4-methylimidazol-1-yl)phenyl]methyl}-1-(4-fluorobenzyl)piperidin-2-one (14.5 g) in tetrahydrofuran (145 mL) was cooled in an ice water bath in a nitrogen atmosphere. A solution of sodium methoxide in methanol (28% solution, 2.64 mL, 1.5 equivalents) was added dropwise to the mixed solution. The reaction mixture was stirred under ice-cooling for 25 minutes and then heated to room temperature. A solution of sodium methoxide in methanol (28% solution, 2.5 mL) was further added to the reaction mixture and the reaction solution was stirred for 10 minutes. After completion of the reaction, *tert*-butyl methyl ether and water were sequentially added to the reaction solution, followed by extraction with *tert*-butyl methyl ether. The resulting organic layer was sequentially washed with 5% saline (twice) and water and dried over anhydrous magnesium sulfate. The resulting organic layer was concentrated under reduced pressure and azeotropically distilled with ethyl acetate. The resulting crude product was purified using a column filled with NH-SiO₂ with ethyl acetate as an elution solvent to provide 12.1 g of the title compound. ¹H-NMR (600 MHz, CDCl₃) δ (ppm): 1.55 (d, J = 7 Hz, 3H), 1.69 (m, 1H), 1.82 (m, 1H), 2.32 (s, 3H), 2.76 (m, 1H), 2.82 (m, 1H), 2.94 (m, 1H), 3.24 (m, 1H), 3.86 (s, 3H), 6.23 (q, J = 7 Hz, 1H), 6.94 (s, 1H), 7.02 (overlapped, 2H), 7.04 (overlapped, 1H), 7.05 (overlapped, 1H), 7.25 (d, J = 5 Hz, 1H), 7.32 (dd, J = 8, 5 Hz, 2H), 7.76 (brs, 1H), 7.89 (brs, 1H).

### Example 4

### Synthesis of (3E)-1-[(1S)-1-(4-fluorophenyl)ethyl]-3-[3-methoxy-4-(4-methyl-1H-imidazol-1-yl)benzylidene]piperidin-2-one

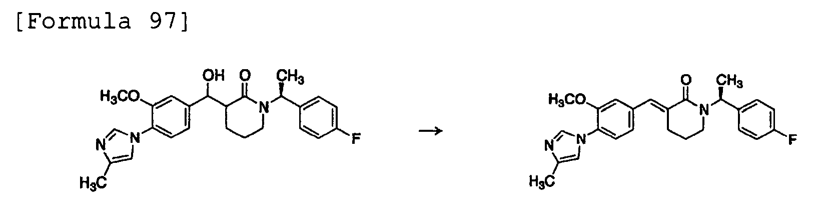

Methanesulfonyl chloride (31.5 mg, 0.275 mmol) was added to a solution containing 1-[(1S)-1-(4-fluorophenyl)ethyl]-3-{hydroxy-[3-methoxy-4-(4-methyl-1H-imidazol-1-yl)phenyllmethyllpiperidin-2-one (100 mg, 0.229 mmol) and triethylamine (34.8 mg, 0.344 mmol) in toluene (1 mL) at room temperature and the reaction mixture was stirred at the same temperature for 1.5 hours. 1,8-Diazabicyclo[5.4.0]unde-7-cene (105 mg, 0.687 mmol) was added to the reaction solution which was then heated to 100°C. The reaction solution was stirred for three hours and then quenched with water, followed by extraction with ethyl acetate. The organic layer was washed with water and then with brine and further dried over magnesium sulfate and concentrated under reduced pressure to provide about 66 mg of the title compound.
¹H-NMR (600 MHz, CDCl₃) δ (ppm): 1.55 (d, J = 7 Hz, 3H), 1.69 (m, 1H), 1.82 (m, 1H), 2.32 (s, 3H), 2.76 (m, 1H), 2.82 (m, 1H), 2.94 (m, 1H), 3.24 (m, 1H), 3.86 (s, 3H), 6.23 (q, *J* = 7 Hz, 1H), 6.94 (s, 1H), 7.02 (overlapped, 2H), 7.04 (overlapped, 1H), 7.05 (overlapped, 1H), 7.25 (d, J = 5 Hz, 1H), 7.32 (dd, *J* = 8, 5 Hz, 2H), 7.76 (brs, 1H), 7.89 (brs, 1H).

### Example 5

### Synthesis of 1-[1-(4-fluorophenyl)ethyl]piperidin-2-one

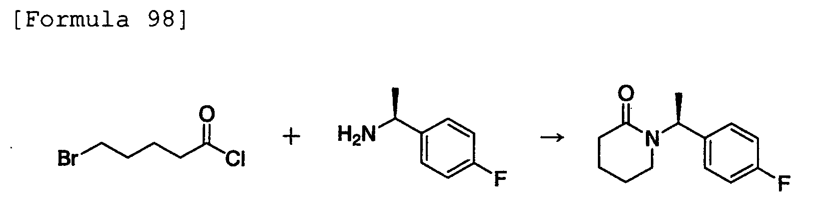

A solution of 5-bromovaleryl chloride (1.0 ml) in toluene (2 ml) was added dropwise to a two-layer mixed solution of a vigorously stirred solution of 1.0 g of (S)-1-(4-fluorophenyl)ethylamine (7.19 mmol) in 5 ml of toluene and a 50% sodium hydroxide solution (7 ml) under ice-cooling over 13 minutes. After stirring the reaction mixture at the same temperature for 15 minutes, benzyltriethylammonium chloride (164 mg) was added to the reaction solution. The reaction mixture was stirred at room temperature for four days. To the reaction solution was added 30 ml of ice water and the layers were separated. Then, the aqueous layer is reextracted with toluene (7 ml). The combined organic layers were sequentially washed with water, 1 N hydrochloric acid, water, a saturated sodium bicarbonate solution and brine and dried over anhydrous magnesium sulfate. Thereafter, the solvent was evaporated under reduced pressure to provide 1.38 g of the title compound as a colorless oil (yield: 87%).
¹H-NMR (600 MHz, CDCl₃) δ (ppm): 1.49 (d, J = 7 Hz, 3H), 1.60 (m, 1H), 1.74 (overlapped, 1H), 1.76 (overlapped, 2H), 2.48 (m, 2H), 2.77 (m, 1H), 3.10 (m, 1H), 6.13 (q, J = 7 Hz, 1H), 7.01 (dd, J = 9, 9 Hz, 2H), 7.26 (dd, J = 9, 6 Hz, 2H).

### Example 6

### Synthesis of 3-bromo-1-[1-(4-fluorophenyl)ethyl]piperidin-2-one

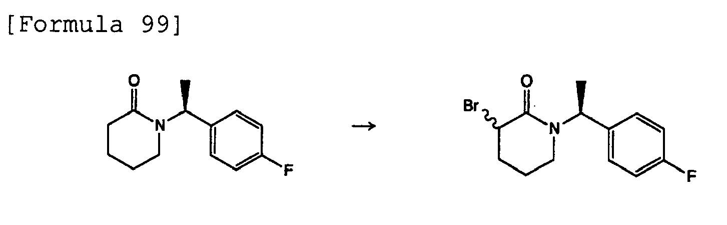

A solution of sec-butyl lithium in 1 M hexane-cyclohexane (8.4 ml) was added dropwise to a solution of 1-[1-(4-fluorophenyl)ethyl]piperidin-2-one (1.5 g) in THF (30 ml) in a nitrogen atmosphere at -78°C over 13 minutes. The reaction mixture was stirred at the same temperature for 20 minutes. Then, bromine (0.38 ml) was added dropwise to the reaction solution and the reaction mixture was stirred at the same temperature for 25 minutes. Thereafter, the reaction was quenched with a saturated ammonium chloride solution (8 ml). The reaction solution was returned to room temperature and then water was added, followed by extraction with ethyl acetate twice. The combined organic layers were sequentially washed with a saturated sodium bicarbonate solution, a 5% sodium thiosulfate solution, water and brine and dried over anhydrous magnesium sulfate. Then, the solvent was evaporated under reduced pressure. The resulting crude product was subjected to silica gel chromatography [Wakogel C-200, 20 g, toluene:ethyl acetate = 1:0 -> 1:1] and the fraction of toluene:ethyl acetate = 19:1 was concentrated to provide 211 mg of the less polar target product as pale yellow crystals (yield: 10%). A mixture (372 mg) of the target diastereomer mixture and a dimer was obtained as a pale yellow viscous oil.
The resulting mixture of the target diastereomer mixture and the dimer was subjected to silica gel chromatography [Wakogel C-200, methylene chloride:n-heptane = 9:1] to provide the target diastereomer mixture (315 mg) as a pale yellow viscous oil.
Then, the resulting target diastereomer mixture was subjected to silica gel chromatography [Wakogel C-200, 4 g, ethyl acetate:n-heptane = 1:5 -> 1:1] and the fraction of ethyl acetate:n-heptane = 1:5 was concentrated to provide the less polar target product (45 mg) as pale yellow crystals (yield: 2%). Further, the fraction of ethyl acetate:n-heptane = 1:5 to 1:1 was concentrated to provide the more polar target product (234 mg) as a pale yellow viscous oil (yield: 12%).
Less polar target product:
¹H-NMR (400 MHz, DMSO-d₆) δ (ppm): 1.44 (d, J = 6.8 Hz, 3H), 1.65-1.75 (m, 1H), 1.88-2.00 (m, 1H), 2.04-2.14 (m, 1H), 2.21-2.31 (m, 1H), 2.76 (ddd, J = 4.8, 9.2, 12.4 Hz, 1H), 3.26 (dt, J = 5.2, 12.4 Hz, 1H), 4.79 (t, J = 4.8 Hz, 1H), 5.75 (q, J = 6.8 Hz, 1H), 7.17 (dd, J = 8.8, 8.8 Hz, 2H), 7.26 (dd, *J* = 6.0, 8.8 Hz, 2H). More polar target product:
¹H-NMR (400 MHz, DMSO-d₆) δ (ppm): 1.45 (d, J = 7.2 Hz, 3H), 1.70-1.79 (m, 1H), 1.80-1.93 (m, 1H), 2.04-2.14 (m, 1H), 2.27-2.37 (m, 1H), 2.84 (td, J = 4.0, 12.4 Hz, 1H), 3.23-3.35 (m, 1H), 4.78 (t, J = 4.0 Hz, 1H), 5.75 (q, J = 7, 2 Hz, 1H), 7.21 (dd, J = 8.8, 8.8 Hz, 2H), 7.26 (dd, J = 5.6, 8.8 Hz, 2H).

### Example 7

### Synthesis of diethyl {1-[(1S)-1-(4-fluorophenyl)ethyl]-2-oxopiperidin-3-yl}phosphonate

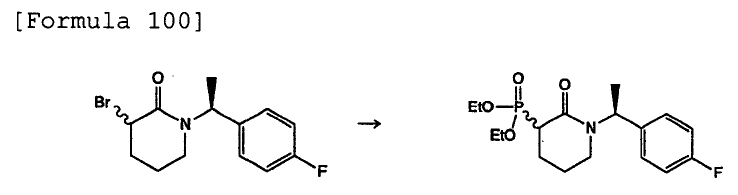

Triethyl phosphite (2.24 g, 13.5 mmol) was added to 1-[(1*S*)-1-(4-fluorophenyl)ethyl]-3-bromopiperidin-2-one (1.35 g, 4.5 mmol) with stirring at room temperature and the reaction solution was stirred at 120°C for five hours. The reaction solution was cooled to room temperature and then concentrated under reduced pressure. The resulting crude oil was purified by silica gel column chromatography to provide the title compound (1.61 g) as a colorless oil.
¹H-NMR (400 MHz, DMSO-d₆) δ (ppm): 1.17-1.27 (m, 6H), 1.42 and 1.44 (each d, *J* = 7.2 Hz, 3H), 1.45-2.01 (m, 4H), 2.65-2.82 (m, 1H), 3.08-3.28 (m, 2H), 3.98-4.12 (m, 4H), 5.79-5.89 (m, 1H), 7.17 (dd, J = 8.8, 8.8 Hz, 2H), 7.28-7.36 (m, 2H).
¹H-NMR (400 MHz, CDCl₃) δ (ppm): 1.30-1.40 (m, 6H), 1.50 (d, J = 6 Hz, 3H), 1.96 (m, 1H), 2.09 (m, 1H), 2.19 (m, 1H), 2.81 (m, 1H), 3.05 (m, 1H), 3.14 (m, 1H), 4.11 (m, 1H), 4.14-4.32 (m, 4H), 6.11 (m, 1H), 7.02 (m, 2H), 7.29 (m, 2H).

### Example 8

### Synthesis of 1-[1-(4-fluorophenyl)ethyl]-3-iodopiperidin-2-one

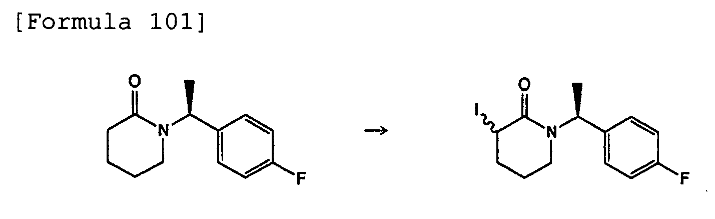

Chlorotrimethylsilane (12.5 ml) was added dropwise to a solution cooled to -20°C of 1-[1-(4-fluorophenyl)ethyl]piperidin-2-one (10 g) and N,N,N',N'-tetramethylethylenediamine (22.5 ml) in toluene (100 ml) in a nitrogen atmosphere. Then, iodine (18.6 g) was introduced in three portions. The reaction solution was gradually heated to 0°C and stirred under ice-cooling for one hour. Then, a mixed solution of a 10% sodium thiosulfate solution and 10% saline was added to the reaction solution. After separating the layers, the organic layer was sequentially washed with a 10% sodium thiosulfate solution, water (twice), 1 N hydrochloric acid, water, a saturated sodium bicarbonate solution and brine. After drying over anhydrous magnesium sulfate, the solvent was evaporated under reduced pressure. The resulting crude product was subjected to silica gel chromatography [Wakogel C-200, 150 g, n-heptane:ethyl acetate = 1:0 -> 2:1] and the fraction of n-heptane:ethyl acetate = 3:1 -> 2:1 was concentrated to provide the target product (15.1 g) as a brown oil (yield: 96%).
¹H-NMR (400 MHz, CDCl₃) δ (ppm): 1.48 and 1.50 (each d, J = 7.2 Hz, 3H), 1.65-1.81 (m, 1H), 1.96-2.25 (m, 3H), 2.82-3.00 (m, 1H), 3.20-3.35 (m, 1H), 4.88-4.96 (m, 1H), 6.04 (q, J = 7.2 Hz, 1H), 6.99-7.10 (m, 2H), 7.24-7.34 (m, 2H).

### Example 9

### Synthesis of diethyl {1-[(1S)-1-(4-fluorophenyl)ethyl]-2-oxopiperidin-3-yl}phosphonate

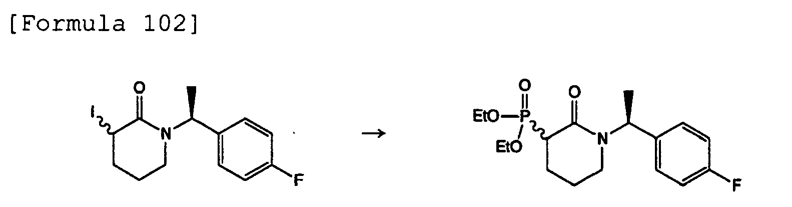

A solution of 1-[1-(4-fluorophenyl)ethyl]-3-iodopiperidin-2-one (10 g) in triethyl phosphite (14.8 ml) was stirred at an external temperature of 80°C for seven hours. Triethyl phosphite was evaporated under reduced pressure. Then, the residue was subjected to silica gel chromatography [Kieselgel 60, 70 to 230 mesh, 100 g, n-heptane:ethyl acetate = 1:0 -> ethyl acetate:ethanol = 19:1]. The fraction of n-heptane:ethyl acetate = 1:1 -> ethyl acetate:ethanol = 19:1 was concentrated to provide the target product (10.28 g) as a light yellow viscous oil (yield: 100%).

### Example 10

### Synthesis of (3E)-1-[(1S)-1-(4-fluorophenyl)ethyl]-3-[3-methoxy-4-(4-methyl-1H-imidazol-1-yl)benzylidene]piperidin-2-one

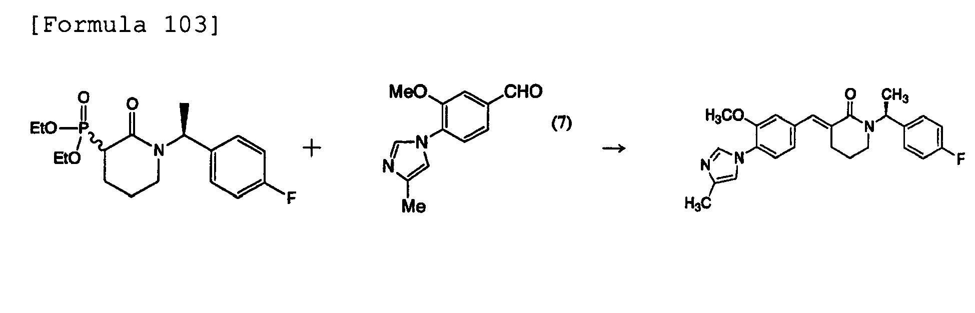

Lithium hydroxide monohydrate (38.8 mg, 0.924 mmol) was added to a mixed solution containing 3-methoxy-4-(4-methyl-1H-imidazol-1-yl)benzaldehyde (100 mg, 0.462 mmol) and diethyl {1-[(15)-1-(4-fluorophenyl)ethyl]-2-oxopiperidin-3-yl}phosphonate (257 mg, 0.693 mmol) in tetrahydrofuran (1 mL) and ethanol (0.1 mL) at room temperature and the reaction solution was stirred at the same temperature for 18 hours. The reaction solution was quenched with water (1 mL), followed by extraction with ethyl acetate. The organic layer was washed with water and then with brine and further dried over magnesium sulfate, filtered and concentrated.
Toluene (1 mL) was added to the resulting crude crystals which were then heated to 100°C and stirred. After confirming that the crystals were dissolved, the solution was cooled to room temperature. After confirming precipitation of the crystals, toluene (0.6 mL) and heptane (1 m) were added and the mixture was further stirred for 2.5 hours. The crystals were collected by filtration and dried to provide the title compound 1 (53 mg) as white crystals.
¹H-NMR (600 MHz, CDCl₃) δ (ppm): 1.55 (d, J = 7 Hz, 3H), 1.69 (m, 1H), 1.82 (m, 1H), 2.32 (s, 3H), 2.76 (m, 1H), 2.82 (m, 1H), 2.94 (m, 1H), 3.24 (m, 1H), 3.86 (s, 3H), 6.23 (q, J = 7 Hz, 1H), 6.94 (s, 1H), 7.02 (overlapped, 2H), 7.04 (overlapped, 1H), 7.05 (overlapped, 1H), 7.25 (d, J = 5 Hz, 1H), 7.32 (dd, J = 8, 5 Hz, 2H), 7.76 (brs, 1H), 7.89 (brs, 1H).

### Example 11

### Synthesis of 2-{3-[(S)-1-(4-fluorophenyl)ethylamino]propyl}malonic acid dimethyl ester

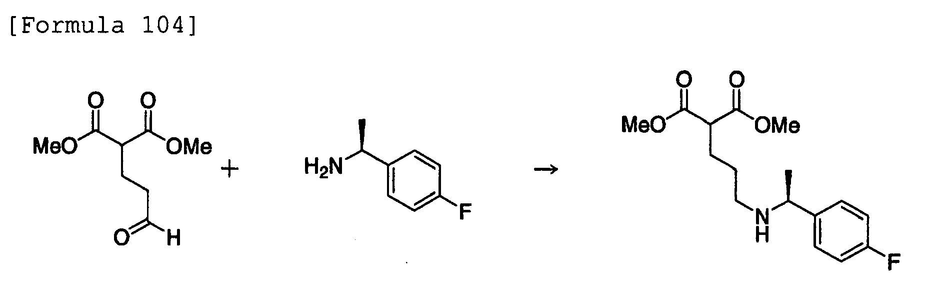

Acetic acid (1 mL), (S)-1-(4-fluorophenyl)ethylamine (4.31 mL) and sodium triacetoxyborohydride (10.2 g) were added to a solution containing 2-(3-oxo-propyl)malonic acid dimethyl ester (6 g) in tetrahydrofuran (120 mL). The reaction mixture was stirred at room temperature for two hours.
The reaction solution was neutralized with sodium bicarbonate. Water and ethyl acetate were added to the reaction solution and the organic layer was separated. The organic layer was dried over anhydrous magnesium sulfate and the solvent was evaporated under reduced pressure. The residue was purified by silica gel column chromatography (Chromatorex NH, heptane-ethyl acetate system) to provide 7.04 g of the title compound.
¹H-NMR (400 MHz, CDCl₃) δ (ppm): 7.29-7.25 (m, 2H), 7.03-6.97 (m, 2H), 3.73 (q, J = 6.8 Hz, 1H), 3.72 (d, J = 2.0 Hz, 6H), 3.34 (t, J = 7.2 Hz, 1H), 2.54-2.48 (m, 1H), 2.48-2.37 (m, 1H), 1.94-1.87 (m, 2H), 1.51-1.46 (m, 2H), 1.34 (d, J = 6.8 Hz, 3H).

### Example 12

### Synthesis of 1-[(S)-1-(4-fluorophenyl)ethyl]-2-oxo-piperidine-3-carboxylic acid

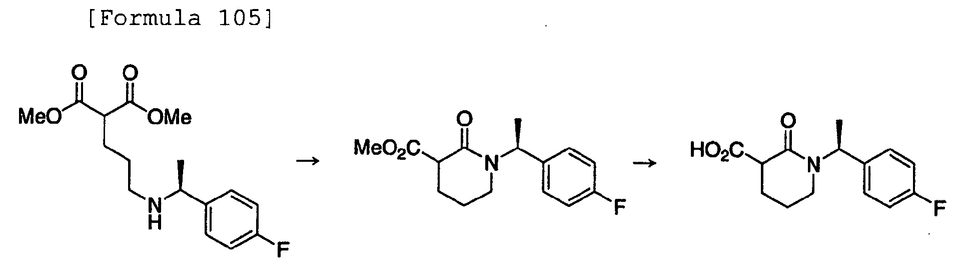

A solution containing 2-{3-[(*S*)-1-(4-fluorophenyl)ethylamino]propyl}malonic acid dimethyl ester (3 g) in acetic acid (30 mL) was heated under reflux for 15 hours. The reaction solution was returned to room temperature and the solvent was evaporated under reduced pressure. Ethyl acetate and a saturated sodium bicarbonate solution were added to the residue and the organic layer was separated. The organic layer was dried over anhydrous magnesium sulfate and the solvent was evaporated under reduced pressure. The residue was dissolved in methanol (20 mL). A 2 N sodium hydroxide solution was added and the reaction mixture was stirred at room temperature for two hours. Ethyl acetate and 2 N sodium hydroxide were added to the reaction solution and the aqueous layer was separated. The aqueous layer was adjusted to about pH 4 with 5 N hydrochloric acid, followed by extraction with ethyl acetate. The organic layer dried over magnesium sulfate and the solvent was evaporated under reduced pressure. The generated solid was washed with ethyl acetate-heptane (1:4) to provide 1.47 g of the title compound.
¹H-NMR (400 MHz, DMSO-d₆) δ (ppm): 12.6 (br, s), 7.32-7.27 (m, 2H), 7.19-7.12 (m, 2H), 5.85-5.76 (m, 1H), 3.37-3.28 (m, 1H), 3.20-3.10 (m, 1H), 2.78-2.65 (m, 1H), 1.99-1.51 (m, 4H), 1.42 (d, J = 7.2 Hz, 3H).

### Example 13

### Synthesis of (3E)-1-[(1S)-1-(4-fluorophenyl)ethyl]-3-[3-methoxy-4-(4-methyl-1H-imidazol-1-yl)benzylidene]piperidin-2-one

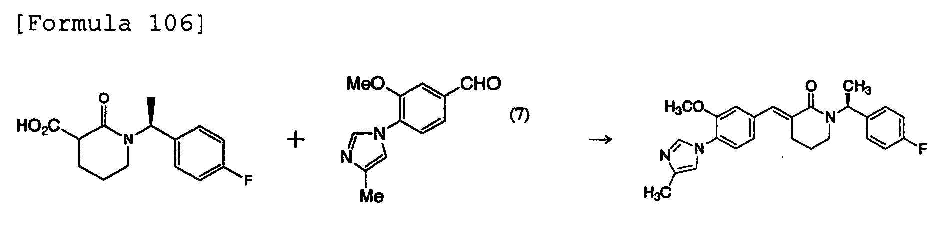

3-Methoxy-4-(4-methyl-imidazol-1-yl)benzaldehyde (815 mg) and piperidine (0.37 mL) were added to a solution containing 1 g of 1-[(S)-1-(4-fluorophenyl)ethyl]-2-oxo-piperidine-3-carboxylic acid in 1 mL of pyridine and the reaction solution was stirred at 65°C for 20 hours. The reaction solution was returned to room temperature. Water and ethyl acetate were added and the organic layer was separated. The organic layer was dried over anhydrous magnesium sulfate and the solvent was evaporated under reduced pressure. The residue was purified by silica gel column chromatography (Chromatorex NH, heptane-ethyl acetate system) to provide 1.12 g of the title compound.
¹H-NMR (600 MHz, CDCl₃) δ (ppm): 1.55 (d, J = 7 Hz, 3H), 1.69 (m, 1H), 1.82 (m, 1H), 2.32 (s, 3H), 2.76 (m, 1H), 2.82 (m, 1H), 2.94 (m, 1H), 3.24 (m, 1H), 3.86 (s, 3H), 6.23 (q, J = 7 Hz, 1H), 6.94 (s, 1H), 7.02 (overlapped, 2H), 7.04 (overlapped, 1H), 7.05 (overlapped, 1H), 7.25 (d, J = 5 Hz, 1H), 7.32 (dd, J = 8, 5 Hz, 2H), 7.76 (brs, 1H), 7.89 (brs, 1H).

### Example 14

### Synthesis of (3E)-1-[(1S)-1-(4-fluorophenyl)ethyl]-3-[3-methoxy-4-(4-methyl-1H-imidazol-1-yl)benzylidene]piperidin-2-one

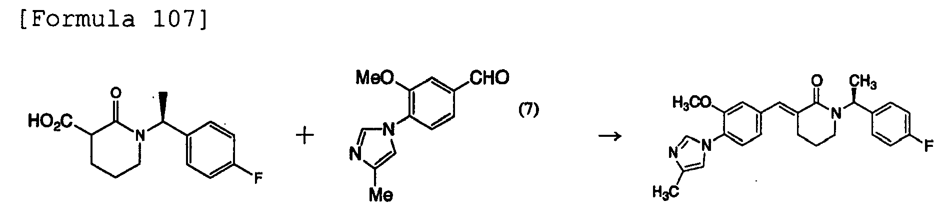

3-Methoxy-4-(4-methylimidazol-1-yl)benzaldehyde (1 g) and piperidine (0.45 mL) were added to a solution containing 1-[(S)-1-(4-fluorophenyl)ethyl]-2-oxo-piperidine-3-carboxylic acid (4.91 g) in pyridine (2.5 mL) and the reaction solution was stirred at 65°C for 20 hours. Further, 1-[(S)-1-(4-fluorophenyl)ethyl]-2-oxo-piperidine-3-carboxylic acid (1.23 g) was added to the reaction solution which was then stirred at 65°C for five hours. The reaction solution was returned to room temperature. Water and ethyl acetate were added and the organic layer was separated. The organic layer was dried over anhydrous magnesium sulfate and the solvent was evaporated under reduced pressure. The residue was purified by silica gel column chromatography (Chromatorex NH, heptane-ethyl acetate system) to provide 1.55 g of the title compound.
¹H-NMR (600 MHz, CDCl₃) δ (ppm): 1.55 (d, J = 7 Hz, 3H), 1.69 (m, 1H), 1.82 (m, 1H), 2.32 (s, 3H), 2.76 (m, 1H), 2.82 (m, 1H), 2.94 (m, 1H), 3.24 (m, 1H), 3.86 (s, 3H), 6.23 (q, J = 7 Hz, 1H), 6.94 (s, 1H), 7.02 (overlapped, 2H), 7.04 (overlapped, 1H), 7.05 (overlapped, 1H), 7.25 (d, J = 5 Hz, 1H), 7.32 (dd, J = 8, 5 Hz, 2H), 7.76 (brs, 1H), 7.89 (brs, 1H).

### Example 15

### Synthesis of 1-[1-(4-fluorophenyl)ethyl]piperidin-2-one

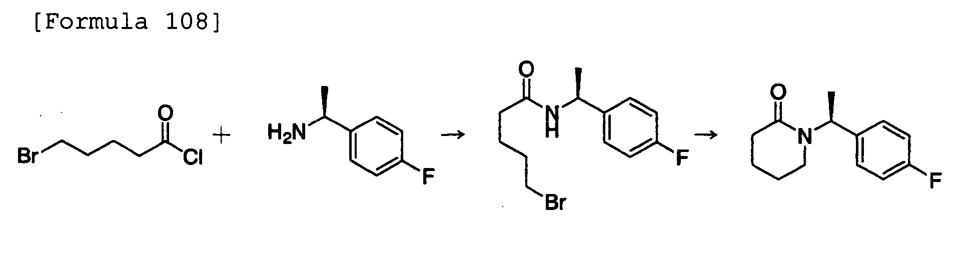

(S)-1-(4-Fluorophenyl)ethylamine (75 g) and *tert*-butyl methyl ether (375 mL) were added to a 2 L four-necked flask in a nitrogen atmosphere at room temperature. Subsequently, a solution of potassium carbonate (111.7 g) in water (525 mL) was added and the mixture was cooled to 0 to 10°C. A previously prepared solution of 5-bromovaleryl chloride (112.9 g) in *tert-*butyl methyl ether (225 mL) was added dropwise at 0°C to 5°C and the reaction was carried out for 30 to 90 minutes. After confirming completion of the reaction using HPLC, the layers were separated and the organic layer was cooled to 0°C to 5°C. *tert*-Butoxy potassium (121.0 g) was added portionwise at 0°C to 25°C and then the reaction was carried out for 10 minutes. After confirming the reaction using HPLC, water (375 mL) was added and the layers were separated. The organic layer was washed with dilute hydrochloric acid (obtained by dissolving 17.9 g of concentrated hydrochloric acid in water (375 mL)) and a sodium bicarbonate solution (obtained by dissolving sodium bicarbonate (22.5 g) in water (375 mL)) and further with water (375 mL) and then concentrated. Toluene (375 mL) was further added. Concentration and then distillation under reduced pressure (119°C to 135°C/0.4 mmHg) were carried out to provide 113.5 g (yield: 95.2%) of the title compound.

### Example 16

### Synthesis of (3E)-1-[(1S)-1-(4-fluorophenyl)ethyl]-3-[3-methoxy-4-(4-methyl-1H-imidazol-1-yl)benzylidene]piperidin-2-one

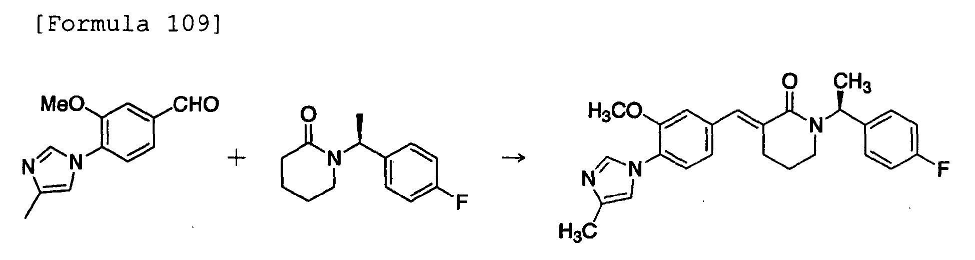

*n*-Butyl lithium (2.44 M solution in cyclohexane, 2.05 mL, 5 mmol) was added to a solution (ice-cooled) of diisopropylamine (1.46 mL, 10.4 mmol) in tetrahydrofuran (0.55 mL) in a nitrogen atmosphere to prepare an LDA solution. Tetrahydrofuran (0.66 mL) was further added to the LDA solution which was then cooled with an ethanol-dry ice bath (-70°C or less). A solution of 1-[1-(4-fluorophenyl)ethyl]piperidin-2-one (884 mg, 4 mmol) in tetrahydrofuran (2 mL) was added dropwise to the solution and the mixture was stirred at the same temperature for one hour. A solution of diethyl chlorophosphate (1.52 g, 8.8 mmol) in tetrahydrofuran (2 mL) was added dropwise to the reaction solution at the same temperature and the mixture was stirred for one hour. A separately adjusted LDA solution [obtained by adding *n*-Butyl lithium (2.44 M solution in cyclohexane, 2.05 mL, 5 mmol) to a solution of diisopropylamine (1.46 mL, 10.4 mmol) in tetrahydrofuran (0.55 mL) in a nitrogen atmosphere] was added dropwise to the reaction solution at the same temperature and the mixture was stirred for one hour. A solution of 3-methoxy-4-(4-methylimidazol-1-yl)benzaldehyde (864 mg, 4 mmol) in tetrahydrofuran (10 mL) was added dropwise to the reaction solution at the same temperature and the mixture was stirred for one hour. Water (4 mL) was added to the reaction solution to complete the reaction, followed by extraction with *tert*-butyl methyl ether (15 mL) and ethyl acetate (5 mL). The organic layer was washed with water and then with 5% saline and further dried over anhydrous magnesium sulfate, filtered and concentrated. The resulting crude product (2.3488 g) was analyzed by HPLC to confirm that the crude product contained 1.01 g (yield: 60%) of the title compound.
Ethyl acetate (6 mL) was added to a crude product (1.866 g, containing 1.19 g of the title compound) obtained by the same operation and the mixture was heated to 60°C and stirred. After confirming that the crude product was dissolved, the solution was cooled to 50°C. A small amount of seed crystals were added and then the mixture was cooled to room temperature. Heptane (9.6 mL) was added and the mixture was stirred for one hour. The crystals were collected by filtration and dried to provide the title compound 967 mg (yield: 77.6%) as flesh-colored crystals.

### Example 17

### Synthesis of (3E)-1-[(1S)-1-(4-fluorophenyl)ethyl]-3-[3-methoxy-4-(4-methyl-1H-imidazol-1-yl)benzylidene]piperidin-2-one

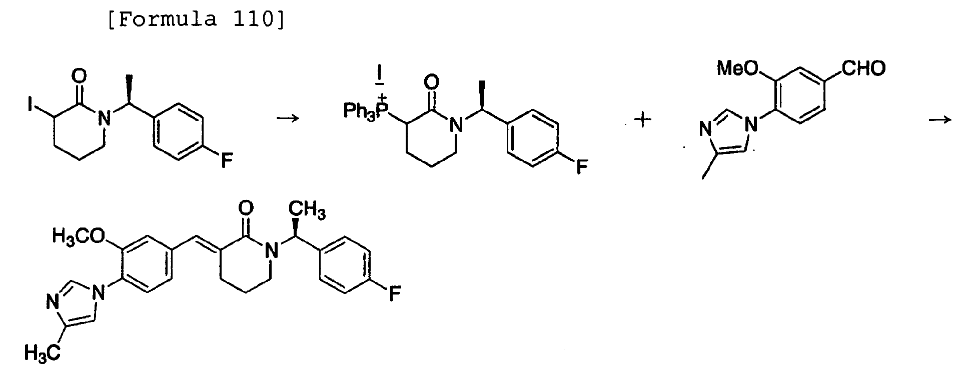

1-[(1S)-1-(4-Fluorophenyl)ethyl]-3-iodopiperidin-2-one (506 mg, 1.46 mmol) was weighed in a 20 mL flask and dissolved in 4 mL of THF. When triphenylphosphine (383 mg, 1.46 mmol) was added, the brown solution was immediately turned yellow and triphenylphosphine was gradually dissolved. The solution was stirred for one hour and heated in an oil bath at 65°C for eight hours. Further, 35.5 mg (0.135 mmol) of triphenylphosphine was added and the mixture was stirred at the same temperature for about two hours.
Next, 0.869 mmol of the concentrate of the synthesized phosphonium salt was weighed and dissolved in EtOH (4 mL). Further, 3-methoxy-4-(4-methylimidazol-1-yl)benzaldehyde (200 mg, 0.925 mmol) and then triethylamine (109 mg, 1.2 equivalents) were added and the mixture was heated at 65°C for 1.5 hours, at 50°C for 18 hours and further at 70°C for 1.5 hours. Triethylamine (212 mg, 2.4 equivalents) was added and the mixture was stirred at 70°C for two hours. Thereafter, the reaction was completed. According to quantitative determination using HPLC, it was confirmed that 274 mg of the title compound was generated (yield: 44.7%)

### Example 18

### Synthesis of 1-[(1S)-1-(4-fluorophenyl)ethyl]-3-{hydroxy[3-methoxy-4-(4-methyl-1H-imidazol-1-yl)phenyl]methyl}piperidin-2-one

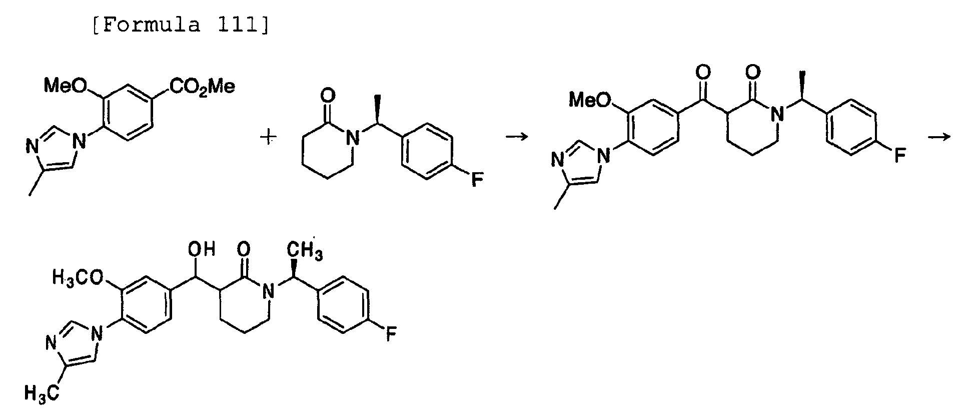

### Synthesis of 1-[(S)-1-(4-fluorophenyl)ethyl]-3-[3-methoxy-4-(4-methyl-1H-imidazol-1-yl)benzoyl]piperidin-2-one

Diisopropylamine (120 µL, 0.84 mmol) in THF (1.2 mL) was cooled to -30°C and *n*-butyllithium (2.44 M in cyclohexane, 330 µL, 0.8 mmol) was added dropwise. After once heating to 0°C, the reaction solution was put in an ethanol-dry ice bath and cooled. A solution of 1-[1-(4-fluorophenyl)ethyl]piperidin-2-one (136 mg, 0.62 mmol) in THF (0.7 mL) was added dropwise at the same temperature over nine minutes and this solution was referred to as a lithium anion solution. A solution containing 3-methoxy-4-(4-methyl-1H-imidazol-1-yl)phenylbenzoic acid methyl ester (100 mg, 0.41 mmol) and N,N,N',N'-tetramethylethylenediamine (0.12 mL, 0.8 mmol) in THF (1.4 mL) was put in an ethanol-dry ice bath and cooled and the lithium anion solution was added dropwise over 16 minutes. One hour and 20 minutes later, the reaction solution was heated to -40°C from the ethanol-dry ice bath and acetic acid (140 µL) was added dropwise over four minutes. Thereafter, the mixture was heated to room temperature and water (2 mL) and toluene (4 mL) were added. The organic layer was washed with brine and water to provide a solution containing 1-[(*S*)-1-(4-fluorophenyl)ethyl]-3-[3-methoxy-4-(4-methyl-1*H*-imidazol-1-yl)benzoyl]piperidin-2-one (141 mg, 0.33 mmol, yield: 80%) in toluene.

### Synthesis of 1-[(1S)-1-(4-fluorophenyl)ethyl]-3-{hydroxy[3-methoxy-4-(4-methyl-1H-imidazol-1-yl)phenyl]methyl}piperidin-2-ol

Sodium borohydride (1.97 mg, 0.05 mmol) was added to a solution containing 1-[(S)-1-(4-fluorophenyl)ethyl]-3-[3-methoxy-4-(4-methyl-1*H-*imidazol-1-yl)benzoyl]piperidin-2-one (17.9 mg, 0.04 mmol) in isopropyl alcohol (0.18 mL) and the mixture was stirred at room temperature for 12 hours. Water (2 mL) and toluene (4 mL) were added and the layers were separated. The organic layer was further washed with brine and water then the solvent was evaporated under reduced pressure. The residue was azeotropically distilled with toluene twice to provide a solution containing 1-[(1S)-1-(4-fluorophenyl)ethyl]-3-{hydroxy[3-methoxy-4-(4-methyl-1H-imidazol-1-yl)phenyl]methyl}piperidin-2-ol (14.5 mg, 0.033 mmol, yield: 81%) in toluene.

### Example 19

### Synthesis of 1-[(1S)-1-(4-fluorophenyl)ethyl]-3-[3-methoxy-4-(4-methyl-1H-imidazol-1-yl)benzoyl]piperidin-2-one

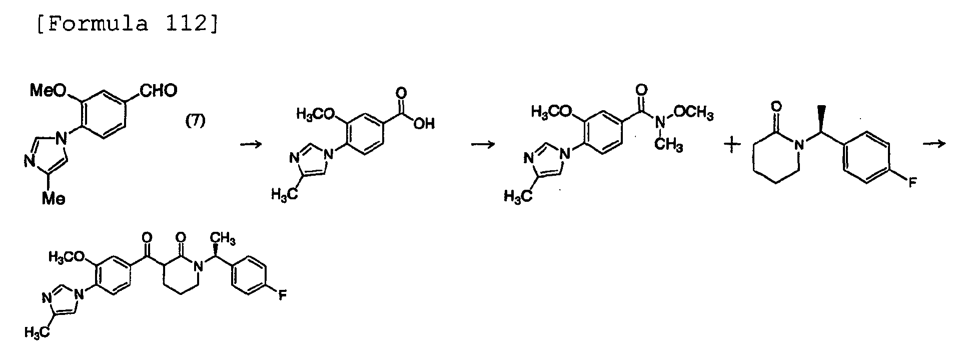

### Synthesis of 3-methoxy-4-(4-methyl-1H-imidazol-1-yl)benzoic acid

To 3-methoxy-4-(4-methyl-1H-imidazol-1-yl)benzaldehyde (10 g, 46.2 mmol) were added a hydrogen peroxide solution (31.0%, 60 mL) at room temperature and a sodium hydroxide solution (24 mL, 0.12 mol) at room temperature. The reaction mixture was stirred at room temperature for 5.3 hours. Sodium thiosulfate (4 g) was added and the mixture was stirred at room temperature for one hour. Ethyl acetate (100 mL) and water (100 mL) were added to the reaction mixture. The separated aqueous layer was further washed with ethyl acetate (100 mL) twice. Hydrochloric acid (5 N, 9 mL) was added to the aqueous layer at room temperature and the precipitated white solid was collected by filtration. The resulting white solid was washed with water (8 mL) twice to provide 8.90 g (yield: 83%) of the title compound.
¹H-NMR (400 MHz, d₆-DMSO) δ = 2.15 (3H, d, J = 1.2 Hz), 3.89 (3H, s), 7.20-7.21 (1H, m), 7.49 (1H, d, J = 8.2 Hz), 7.61 (1H, dd, J = 8.2 and 17.2 Hz), 7.67 (1H, d, J = 17.2 Hz), 7.87 (1H, d, J = 1.2 Hz).

### Synthesis of N,3-dimethoxy-N-methyl-4-(4-methyl-1H-imidazol-1-yl)benzamide

N,N-Dimethylformamide (10 mL) was added to a mixture of 3-methoxy-4-(4-methyl-1*H*-imidazol-1-yl)benzoic acid (1.0 g, 4.31 mmol) and *N*,*O-*dimethylhydroxyamine hydrochloride (441 mg, 4.52 mmol), followed by addition of triethylamine (2.10 mL, 15.1 mmol) at room temperature. Diethyl cyanophosphonate (719 µL, 4.74 mmol) was added to the mixture at room temperature and the reaction mixture was stirred for 25.9 hours. Ethyl acetate (40 mL) and water (20 mL) were added to the reaction mixture and then a sodium hydroxide solution (2 N, 2 mL) was added. The separated organic layer was washed with water (10 mL) twice and the combined aqueous layers were extracted with ethyl acetate (100 mL). The combined organic layers were washed with water (50 mL) three times, dried over anhydrous magnesium sulfate and then concentrated under reduced pressure. The resulting residue was purified by silica gel column chromatography (NH-SiO₂, elution solvent: ethyl acetate) to provide 857 mg (yield: 72%) of the title compound. ¹H-NMR (400 MHz, CDCl₃) δ = 2.31 (3H, bs), 3.40 (3H, s), 3.60 (3H, s), 3.90 (3H, s), 6.93-6.96 (1H, m), 7.27 (1H, d, J = 8.0 Hz), 7.38-7.41 (2H, m), 7.78 (1H, d, J = 1.2 Hz).

### Synthesis of 1-[(1S)-1-(4-fluorophenyl)ethyl]-3-[3-methoxy-4-(4-methyl-1H-imidazol-1-yl)benzoyl]piperidin-2-one

A solution of diisopropylamine (414 µL, 2.95 mmol) in tetrahydrofuran (4.14 mL) was cooled to -40°C and *n*-butyllithium (2.44 M in cyclohexane, 0.96 mL, 2.34 mmol) was added dropwise. The reaction mixture was stirred at -40°C for 0.4 hours. Then, a solution of 1-[1-(4-fluorophenyl)ethyl]piperidin-2-one (398 mg, 1.80 mmol) in tetrahydrofuran (1.99 mL) was added dropwise over five minutes. (This solution is referred to as a lithium anion solution.) The lithium anion solution was added dropwise to a solution of N,3-dimethoxy-*N*-methyl-4-(4-methyl-1*H*-imidazol-1-yl)benzamide (413 mg, 1.50 mmol) in tetrahydrofuran (5.62 mL) at -40°C over 0.3 hours and then the mixture was stirred for 1.5 hours. Water (5.62 mL) was added dropwise to the reaction mixture at -40°C over 0.3 hours and then the mixture was stirred at room temperature for 0.8 hours. Toluene (36 mL), water (5 mL) and brine (2 mL) were added to the mixture and the organic layer was separated. The organic layer was quantitatively determined by HPLC analysis to provide 480 mg (yield: 74%) of the title compound.

### Example 20

### Synthesis of (3E)-1-[(1S)-1-(4-fluorophenyl)ethyl]-3-[3-methoxy-4-(4-methyl-1H-imidazol-1-yl)benzylidene]piperidin-2-one

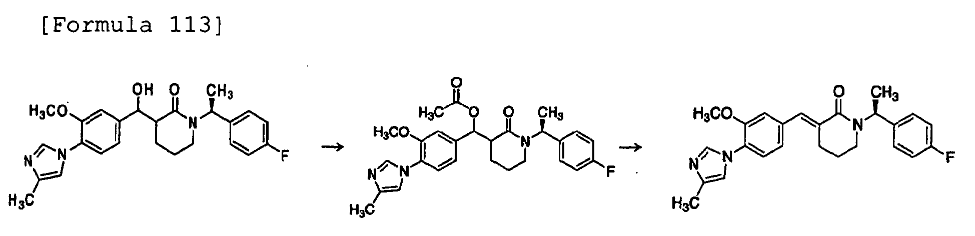

### Synthesis of acetic acid {1-[(1S)-1-(4-fluorophenyl)ethyl]-2-oxopiperidin-3-yl}[3-methoxy-4-(4-methyl-1H-imidazol-1-yl)phenyl]methyl ester

To 49.2 g (net. 47.1 g, 0.11 mol) of the crude product of 1-[1-(4-fluorophenyl)ethyl]-3-{hydroxy[3-methoxy-4-(4-methylimidazol-1-yl)phenyl]methyl}piperidin-2-one were added toluene (236 mL), tetrahydrofuran (236 mL) and 4-dimethylaminopyridine (658 mg, 5.38 mmol) at room temperature and then acetic anhydride (15.3 mL, 0.16 mol) at room temperature. The reaction mixture was stirred for seven minutes and then stirred at 40°C for three hours. Methanol (23.6 mL) was added at room temperature over six minutes. After stirring the mixture at room temperature for 30 minutes, water (236 mL) and a sodium hydroxide solution (2 N, 53.8 mL) were sequentially added at room temperature and the organic layer was separated. The organic layer was sequentially washed with a sodium carbonate solution (5%, 236 mL), a sodium chloride solution (5%, 236 mL) and water (236 mL). The organic layer was concentrated under reduced pressure and the concentrated with toluene (94.2 mL) three times to provide 53.0 g (net. 49.0 g, yield: 95%) of the title compound as a crude product.
¹H-NMR (400 MHz, CDCl₃) δ = 1.36, 1.47, 1.51 and 1.52 (3H, d, J = 7.2 Hz), 1.40-2.00 (5H, m), 2.12, 2.15, 2.16 and 2.17 (3H, S), 2.28-2.32 (3H, m), 2.40-3.25 (3H, m), 3.78-3.86 (3H, m), 5.96-6.20 (1H, m), 6.60-6.70 (1H, m), 6.85-7.32 (7H, m), 7.62-7.70 (1H, m).

### Synthesis of (3E)-1-[(1S)-1-(4-fluorophenyl)ethyl]-3-[3-methoxy-4-(4-methyl-1H-imidazol-1-yl)benzylidene]piperidin-2-one

To a suspension of sodium tert-butoxide (4.21 g) in toluene (150 mL) was added at room temperature was added a solution of 16.3 g (net. 15.0 g, 31.3 mmol) of the crude product of acetic acid {1-[(1*S*)-1-(4-fluorophenyl)ethyl]-2-oxopiperidin-3-yl}[3-methoxy-4-(4-methyl-1*H*-imidazol-1-yl)phenyl]methyl ester in toluene (75 mL) at -13°C over 35 minutes. The mixture was stirred at the same temperature for 2.4 hours. Water (75 mL) was added dropwise to the reaction mixture at -13°C over six minutes and then the mixture was stirred at room temperature for 1.6 hours. The mixture was filtered and toluene (15 mL) was added to the filtrate. The separated organic layer was washed with water (75 mL) three times and then concentrated under reduced pressure. The concentrate was azeotropically distilled with toluene (30 mL) to provide 13.4 g (net. 11.8 g, yield: 90%) of the title compound as a crude product.

### Recrystallization of (3E)-1-[(1S)-1-(4-fluorophenyl)ethyl]-3-[3-methoxy-4-(4-methyl-1H-imidazol-1-yl)benzylidene]piperidin-2-one

Isobutyl acetate (29 mL) was added to 11.2 g (net. 9.61 g, 22.9 mmol) of the crude product of (3E)-1-[(1S)-1-(4-fluorophenyl)ethyl]-3-[3-methoxy-4-(4-methyl-1*H*-imidazol-1-yl)benzylidene]piperidin-2-one.
The mixture was concentrated under reduced pressure at 50°C and then isobutyl acetate (96.1 mL) was added to the concentrate. The mixture was stirred at 95°C for 11 minutes and then stirred at 65°C for 30 minutes. After stirring the mixture at 70°C for six minutes, seed crystals of the title compound (20 mg) were added and the mixture was stirred for 19 minutes. The mixture was stirred at 53°C for nine minutes and then stirred at 54°C for 52 minutes. Heating was stopped and the mixture was stirred for 33 minutes. After stirring the mixture at 60°C for 22 minutes, heating was stopped. The mixture was stirred for 40 minutes and stirred at 7°C for 20 hours. The precipitated solid was collected by filtration. The resulting solid was washed with a mixed solution of isobutyl acetate (19.2 mL) and heptane (19.2 mL) and then dried under reduced pressure at 60°C for 12 hours to provide 7.87 g (yield: 90%) of the title compound.

### Example 21

### Synthesis of (3E)-1-[(1S)-1-(4-fluorophenyl)ethyl]-3-[3-methoxy-4-(4-methyl-1H-imidazol-1-yl)benzylidene]piperidin-2-one

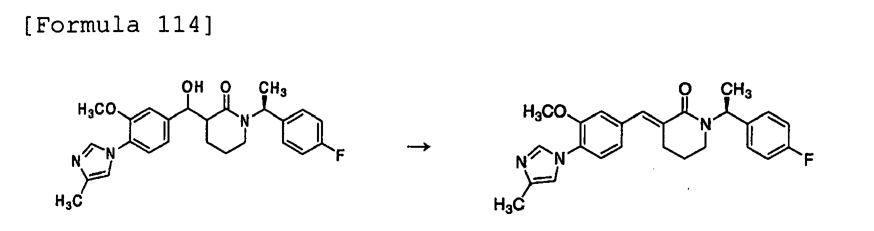

*p*-Toluenesulfonic anhydride (194 mg, 0.59 mmol) and sodium *p*-toluenesulfonate (89 mg, 0.46 mmol) were sequentially added to 1-[1-(4-fluorophenyl)ethyl]-3-{hydroxy[3-methoxy-4-(4-methylimidazol-1-yl)phenyl]methyl}piperidin-2-one (200 mg, 0.46 mmol). Then, toluene (4.0 mL) was added and the mixture was stirred at 100°C for 36 hours. Water (4 mL), toluene (4 mL), a sodium hydroxide solution (2 N, 4 mL) and tetrahydrofuran (6 mL) were sequentially added to the reaction mixture. After stirring at room temperature for three minutes, toluene (4 mL) and tetrahydrofuran (2 mL) were added. A sodium hydroxide solution (2 N, 3 mL) was added to the separated organic layer which was then filtered through a celite pad. Then, toluene (6 mL) was added to the filtrate and the layers were separated. The organic layer was quantitatively determined by HPLC analysis to provide 147 mg (yield: 77%) of the title compound.

### Example 22

### Synthesis method of 1-[(1S)-1-(4-fluorophenyl)ethyl]-3-{hydroxy[3-methoxy-4-(4-methyl-1H-imidazol-1-yl)phenyl]piperidin-2-one

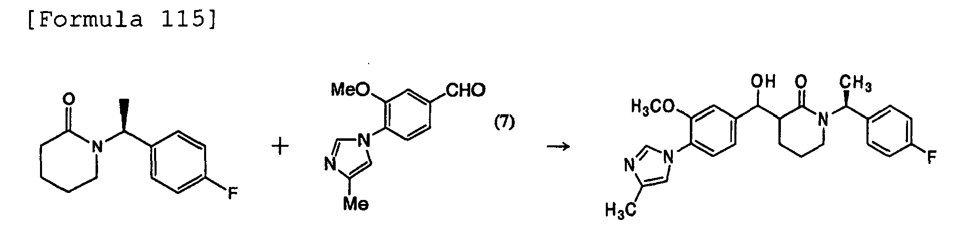

A solution of diisopropylamine (21.2 mL, 151 mmol) in toluene (208 mL) was cooled to -10°C and *n-*butyllithium (20% in cyclohexane, 59.1 mL, 144 mmol) was added dropwise over 0.5 hours to prepare a diisopropylamide solution. The diisopropylamide solution was added dropwise to a solution of 1-[1-(4-fluorophenyl)ethyl]piperidin-2-one (29.3 g, 132 mmol) in toluene (130 mL) at -10°C for 1.4 hours and the mixture was stirred at the same temperature for 0.5 hours. A solution of 3-methoxy-4-(4-methylimidazol-1-yl)benzaldehyde (26.0 g, 120 mmol) in tetrahydrofuran (351 mL) was added dropwise to the reaction mixture at -10°C over 1.2 hours and the mixture was stirred at - 10°C for 0.5 hours. Methanol (13.0 mL) was added dropwise to the reaction mixture at -10°C for five minutes and then the mixture was stirred at -10°C for 0.5 hours. Further, water (130 mL) was added dropwise over three minutes and then the mixture was stirred at room temperature for 50 minutes. The mixture was transferred to a separating device and the aqueous layer was discarded. Thereafter, 2 N hydrochloric acid (260 mL) was added to the organic layer and the aqueous layer was separated. Tetrahydrofuran (390 mL) and a 5 N sodium hydroxide solution (130 mL) were added to the aqueous layer and the aqueous layer was discarded. Toluene (260 mL) was added to the organic layer which was then sequentially washed with 5% saline (78 mL) and water (78 mL) and then concentrated under reduced pressure. The concentrate was concentrated under reduced pressure with toluene (52 mL) twice to provide 55.47 g (net. 52.6 g, yield: >99%) of the title compound as a crude product.

### INDUSTRIAL APPLICABILITY

According to the present invention, a cinnamide derivative not yet described in any document, in particular, (3*E*)-1-[(1*S*)-1-(4-fluorophenyl)ethyl]-3-[3-methoxy-4-(4-methyl-1H-imidazol-1-yl)benzylidene]piperidin-2-one can be efficiently produced.

## Claims

1. A process for producing a compound (8) represented by the formula (8): 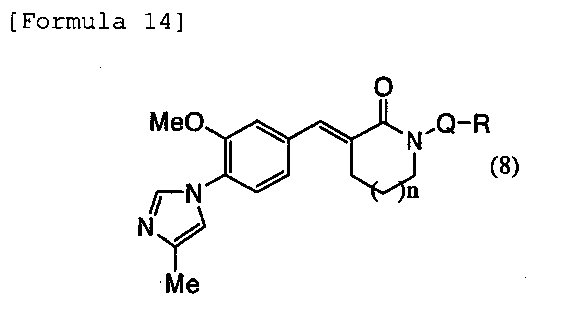 wherein Q represents a single bond or -CH(Y)- (wherein Y represents a hydrogen atom or a C1-6 alkyl group), R represents a 6- to 14-membered aromatic hydrocarbon ring group which may have a substituent or a 5- to 14-membered aromatic heterocyclic group which may have a substituent and n represents 0 to 2, the process comprising the steps of:
reacting, in the presence of a base, a compound (1) represented by the formula (1):
[Formula 1] R₁-Q-NH₂ (1)
wherein Q is as defined above and R₁ represents a 6- to 14-membered aromatic hydrocarbon ring group which may have a substituent (wherein the substituent may have a protecting group) or a 5- to 14-membered aromatic heterocyclic group which may have a substituent (wherein the substituent may have a protecting group), with a compound (2) represented by the formula (2):
[Formula 2] X₁- (CH₂)₂ - (CH₂)ₙ-CH₂-CO-X₂ (2)
wherein X₁ and X₂ are the same or different and each represent a halogen atom and n is as defined above, to convert the compound (1) into a compound (3) represented by the formula (3): 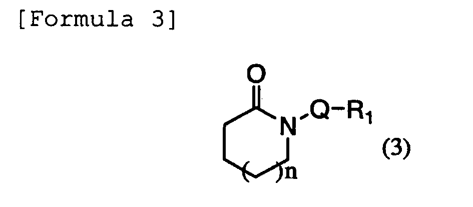 wherein Q, R₁ and n are as defined above;
treating the compound (3) represented by the formula (3): 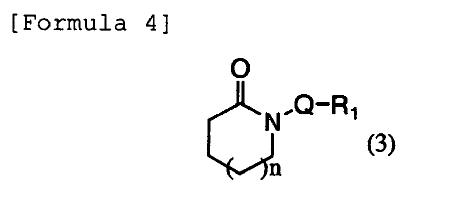 wherein Q, R₁ and n are as defined above, with a base and then reacting the treated compound with a halogenating reagent, or reacting the compound (3) with a halogenating reagent in the presence of a base to convert the compound (3) into a compound (4) represented by the formula (4): 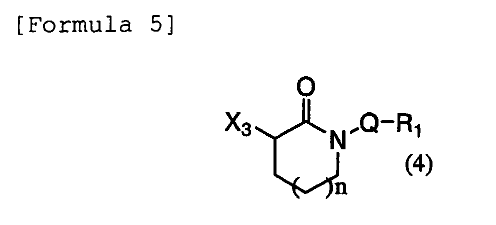 wherein X₃ represents a halogen atom and Q, R₁ and n are as defined above;
reacting the compound (4) represented by the formula (4): 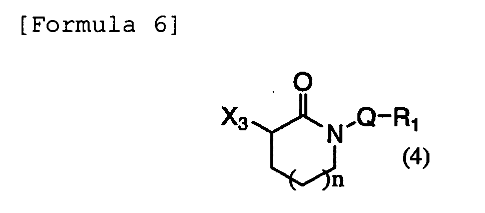 wherein Q, R₁, X₃ and n are as defined above, with a phosphorous acid compound (5-a) represented by the formula (5-a):
[Formula 7] P (OR₂ₐ) ₃ (5 -a)
wherein R₂ₐ represents a C1-6 alkyl group which may have a substituent or a phenyl group which may have a substituent, or a phosphorus compound (5-b) represented by the formula (5-b):
[Formula 8] P (R_{2b}) ₃ (5-b)
wherein R_{2b} represents a C1-6 alkyl group which may have a substituent or a phenyl group which may have a substituent, to respectively convert the compound (4) into a compound (6-a) represented by the formula (6-a): 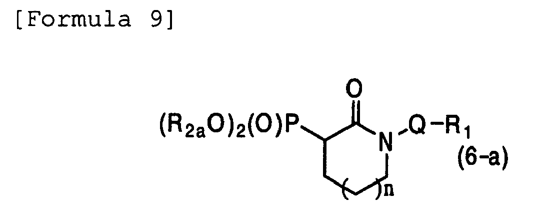 wherein Q, R₁, R₂ₐ and n are as defined above, or a compound (6-b) represented by the formula (6-b): 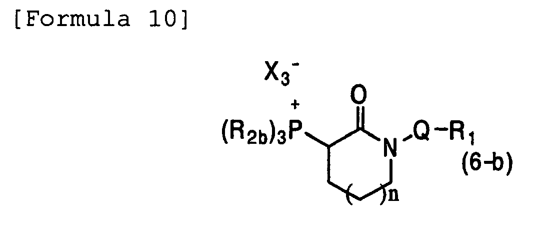 wherein Q, R₁, R_{2b}, X₃ and n are as defined above; and
reacting, in the presence of a base, the compound (6-a) represented by the formula (6-a): 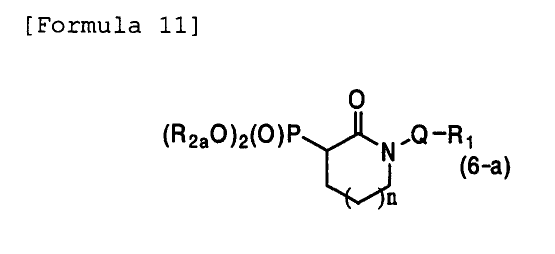 wherein Q, R₁, R₂ₐ and n are as defined above, or the compound (6-b) represented by the formula (6-b): 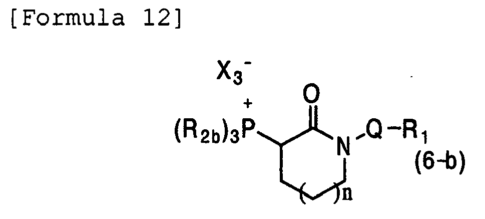 wherein Q, R₁, R_{2b}, X₃ and n are as defined above, with 3-methoxy-4-(4-methyl-1H-imidazol-1-yl)benzaldehyde represented by the formula (7): 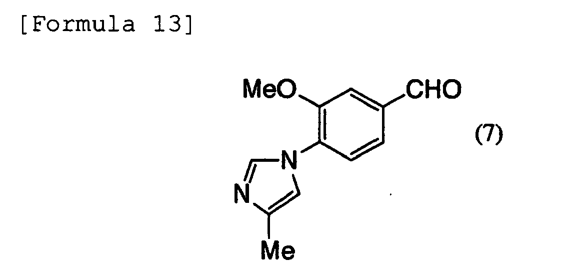 and removing a protecting group when R₁ has the protecting group.

2. The process for producing a compound (8) according to claim 1, comprising the step of reacting a compound (4) with a phosphorous acid compound (5-a) to convert the compound (4) into a compound (6-a).

3. A process for producing a compound (8) represented by the formula (8): 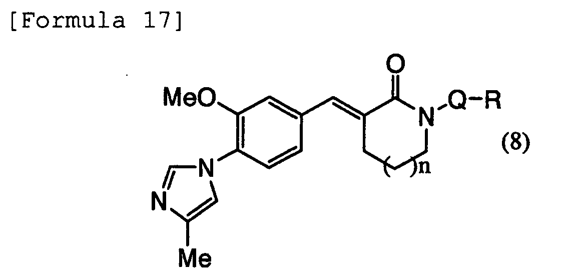 wherein Q, R and n are as defined in claim 1, the process comprising the steps of:
reacting, in the presence of a base, a compound (6-a) represented by the formula (6-a): 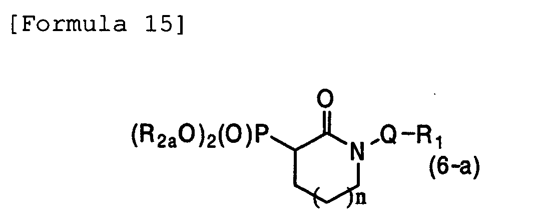 wherein R₁ and R₂ₐ are as defined in claim 1 and Q and n are as defined above, with 3-methoxy-4-(4-methyl-1*H-*imidazol-1-yl)benzaldehyde represented by the formula (7) : 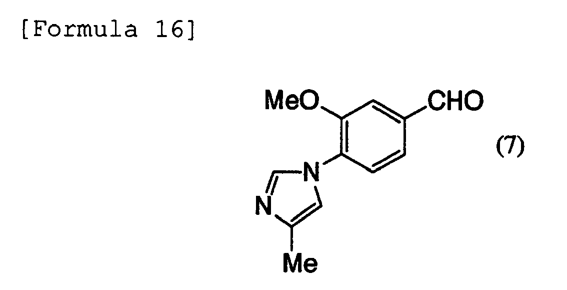 and removing a protecting group when R₁ has the protecting group.

4. A process for producing a compound (6-a) represented by the formula (6-a): 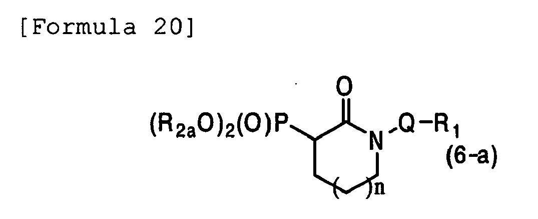 wherein Q, R₁, R₂ₐ and n are as defined in claim 1, the process comprising:
reacting a compound (4) represented by the formula (4): 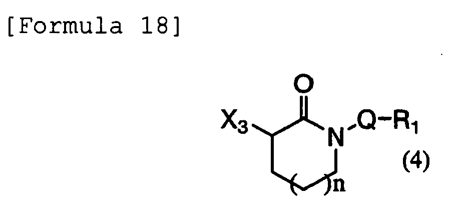 wherein X₃ is as defined in claim 1 and Q, R₁ and n are as defined above, with a phosphorous acid compound (5-a) represented by the formula (5-a):
[Formula 19] P (OR₂ₐ) ₃ (5-a)
wherein R₂ₐ is as defined above.

5. A process for producing a compound (4) represented by the formula (4): 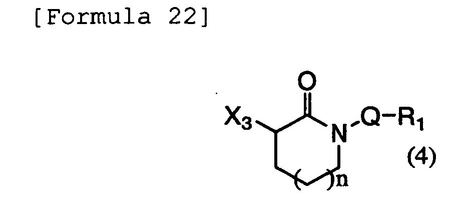 wherein Q, R₁, X₃ and n are as defined in claim 1, the process comprising:
treating a compound (3) represented by the formula (3): 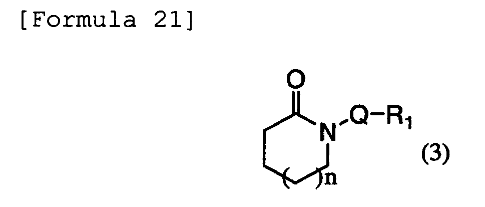 wherein Q, R₁ and n are as defined above, with a base and reacting the treated compound with a halogenating reagent.

6. A process for producing a compound (3) represented by the formula (3): 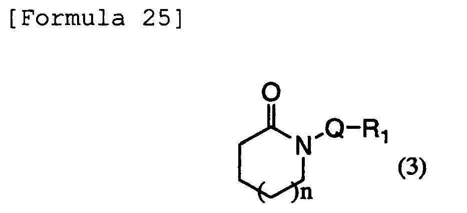 wherein Q represents a single bond or -CH(Y)- (wherein Y represents a hydrogen atom or a C1-6 alkyl group), R₁ represents a 6- to 14-membered aromatic hydrocarbon ring group which may have a substituent (wherein the substituent may have a protecting group) or a 5- to 14-membered aromatic heterocyclic group which may have a substituent (wherein the substituent may have a protecting group) and n represents 0 to 2, the process comprising:
reacting, in the presence of a base, a compound (1) represented by the formula (1):
[Formula 23] R₁-Q-NH₂ (1)
wherein Q and R₁ are as defined above, with a compound (2) represented by the formula (2):
[Formula 24] X₁- (CH₂)₂- (CH₂) ₙ-CH₂-CO-X₂ (2)
wherein X₁ and X₂ are the same or different and each represent a halogen atom and n is as defined above.

7. A compound (15) represented by the formula (15) : 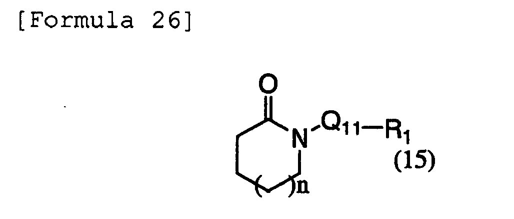 wherein Q₁₁ represents -CH(Y₁₁)- (wherein Y₁₁ represents a C1-6 alkyl group) and R₁ and n are as defined in claim 1, or a salt thereof, or a hydrate thereof.

8. A compound (16) represented by the formula (16): 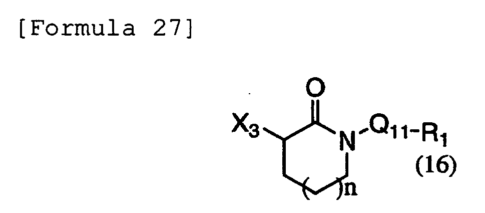 wherein R₁, X₃ and n are as defined in claim 1 and Q₁₁ is as defined in claim 7, or a salt thereof, or a hydrate thereof.

9. A compound (17) represented by the formula (17): 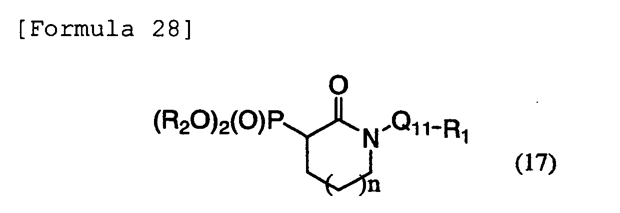 wherein R₁, R₂ and n are as defined in claim 1 and Q₁₁ is as defined in claim 7, or a salt thereof, or a hydrate thereof.

10. A process for producing a compound (8) represented by the formula (8): 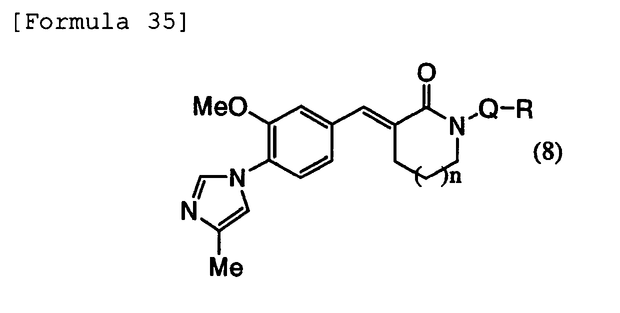 wherein Q, R and n are as defined in claim 1, the process comprising the steps of:
reacting, in the presence of a base, a compound (3) represented by the formula (3): 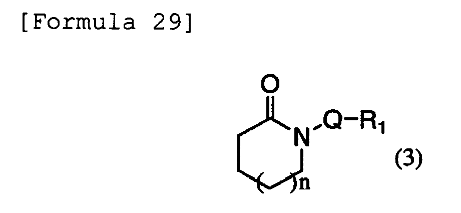 wherein R₁ is as defined in claim 1 and Q and n are as defined above, with 3-methoxy-4-(4-methyl-1H-imidazol-1-yl)benzaldehyde represented by the formula (7): 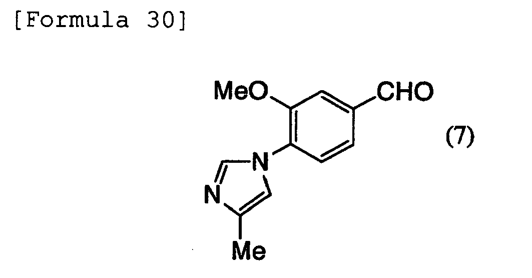 to convert the compound (3) into a compound (9) represented by the formula (9): 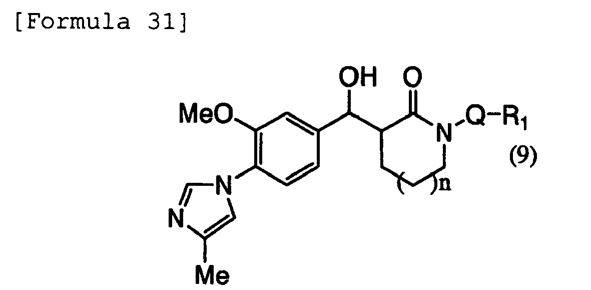 wherein Q, R₁ and n are as defined above;
reacting the compound (9) represented by the formula (9): 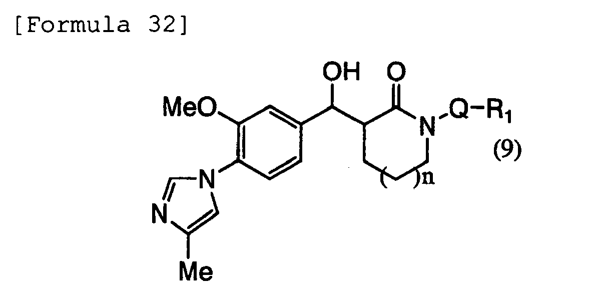 wherein Q, R₁ and n are as defined above, with a leaving group introduction reagent in the presence of a base if necessary to convert the compound (9) into a compound (10) represented by the formula (10): 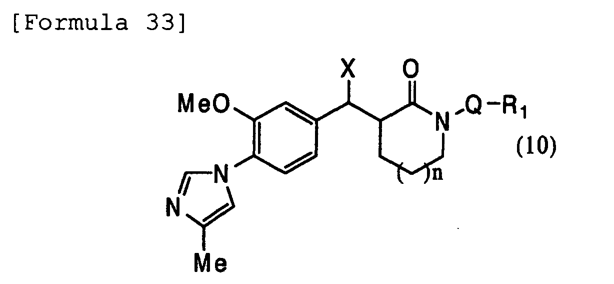 wherein X represents a leaving group and Q, R₁ and n are as defined above; and
treating the compound (10) represented by the formula (10): 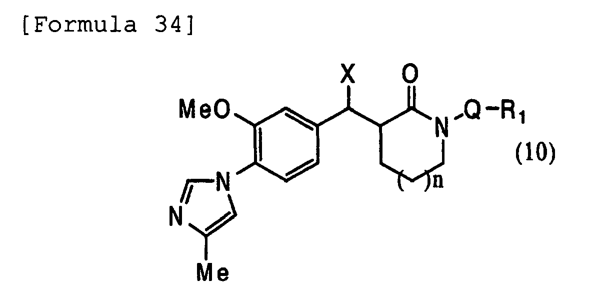 wherein Q, R₁, X and n are as defined above, with a base and removing a protecting group when R₁ has the protecting group.

11. The process for producing a compound (8) according to claim 10, comprising the steps of:
reacting a compound (9) with a halogenating reagent or a sulfonylating reagent in the presence of a base if necessary to convert the compound (9) into a compound (10) represented by the formula (10-a): 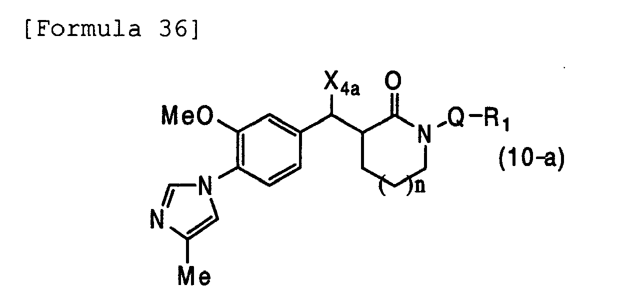 wherein X₄ₐ represents a halogen atom or a sulfonyloxy group and Q, R₁ and n are as defined above; and
treating the compound represented by the formula (10-a): 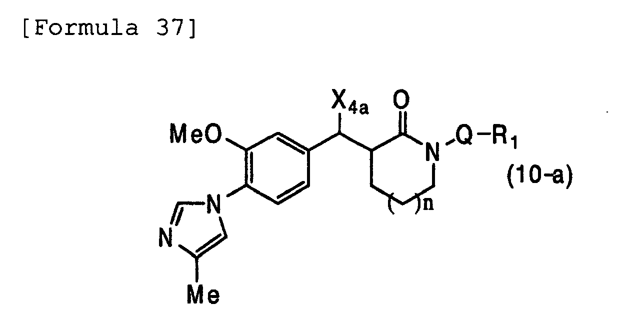 wherein Q, R₁, X₄ₐ and n are as defined above, with a base and removing a protecting group when R₁ has the protecting group.

12. A process for producing a compound (8) represented by the formula (8): 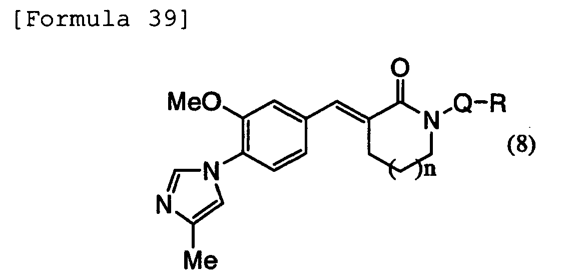 wherein Q, R and n are as defined in claim 1, the process comprising the steps of:
treating a compound represented by the formula (10-a): 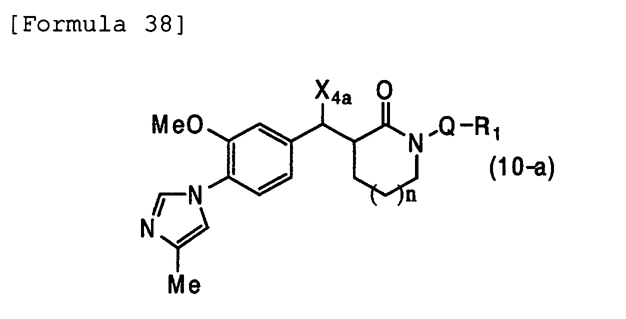 wherein R₁ is as defined in claim 1, X₄ₐ is as defined in claim 11 and Q and n are as defined above, with a base and removing a protecting group when R₁ has the protecting group.

13. A process for producing a compound (10) represented by the formula (10-a): 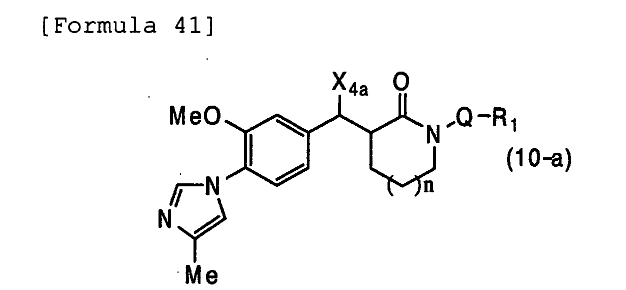 wherein Q, R₁ and n are as defined in claim 1 and X₄ₐ is as defined in claim 11, the process comprising:
reacting a compound (9) represented by the formula (9): 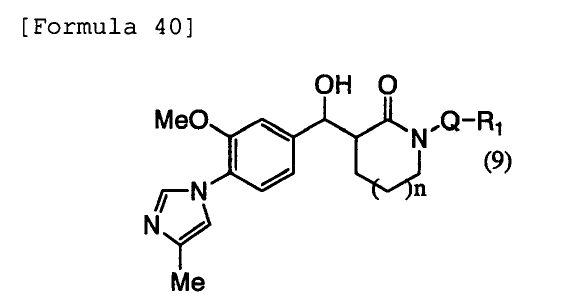 wherein Q, R₁ and n are as defined in claim 1, with a halogenating reagent or a sulfonylating reagent in the presence of a base if necessary.
(clearly presented as an independent claim)

14. A process for producing a compound (9) represented by the formula (9): 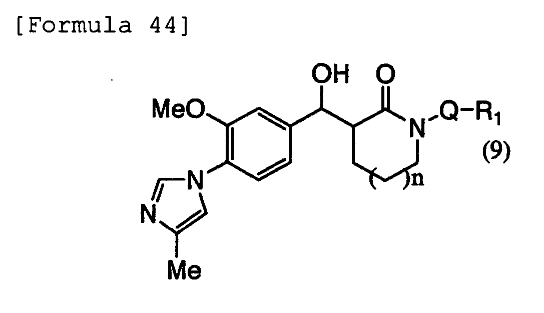 wherein Q, R₁ and n are as defined in claim 1, the process comprising:
reacting, in the presence of a base, a compound (3) represented by the formula (3): 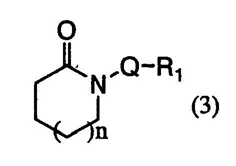 wherein Q, R₁ and n are as defined above, with 3-methoxy-4-(4-methyl-1H-imidazol-1-yl)benzaldehyde represented by the formula (7):

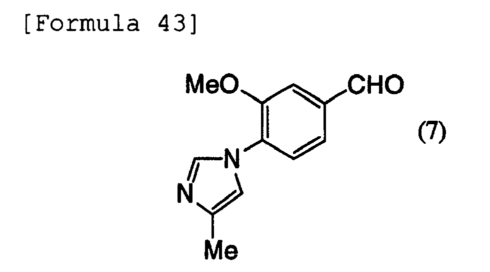

15. The process for producing a compound (8) according to claim 10, comprising the steps of:
reacting a compound (9) with an acylating reagent in the presence of a base if necessary to convert the compound (9) into a compound (10-b) represented by the formula (10-b): 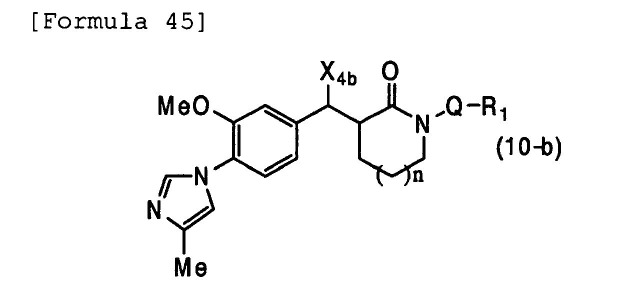 wherein X_{4b} represents an acyloxy group and Q, R₁ and n are as defined above; and
treating the compound represented by the formula (10-b): 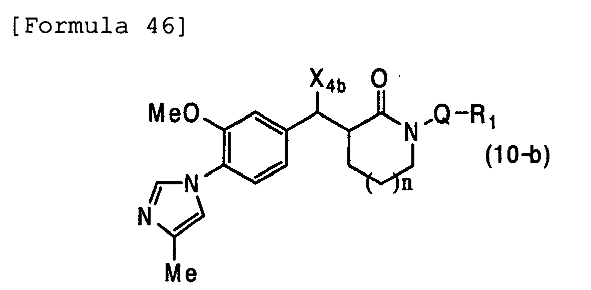 wherein Q, R₁, X_{4b} and n are as defined above, with a base and removing a protecting group when R₁ has the protecting group.

16. A process for producing a compound (8) represented by the formula (8): 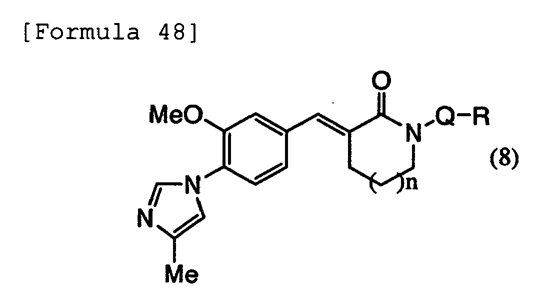 wherein Q, R and n are as defined in claim 1, the process comprising the steps of:
treating a compound represented by the formula (10-b): 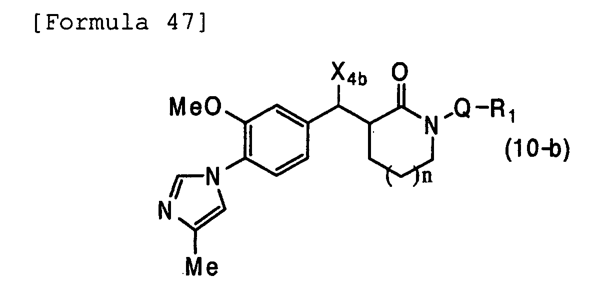 wherein R₁ is as defined in claim 1, X_{4b} is as defined in claim 16 and Q and n are as defined above, with a base and removing a protecting group when R₁ has the protecting group.

17. A process for producing a compound (10-b) represented by the formula (10-b): 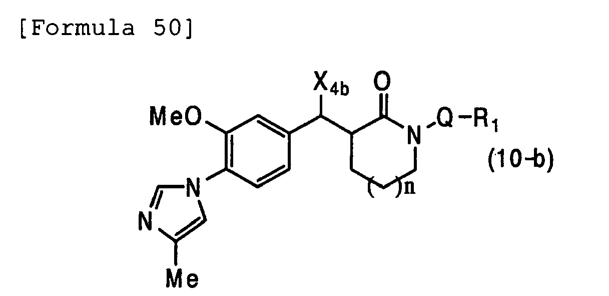 wherein Q, R₁ and n are as defined in claim 1 and X_{4b} is as defined in claim 16, the process comprising:
reacting a compound (9) represented by the formula (9): 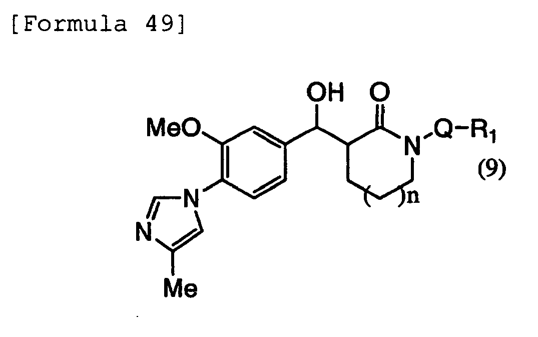 wherein Q, R₁ and n are as defined in claim 1, with an acylating reagent in the presence of a base if necessary.

18. A compound (9) represented by the formula (9) : 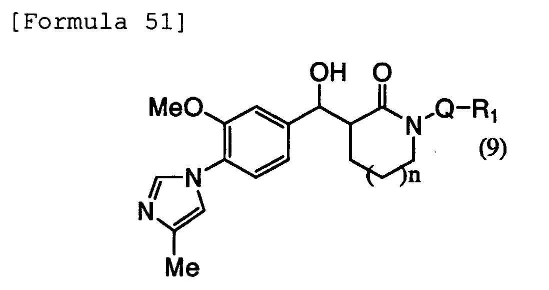 wherein Q, R₁ and n are as defined in claim 1, or a salt thereof, or a hydrate thereof.

19. A compound (10-a) represented by the formula (10-a): 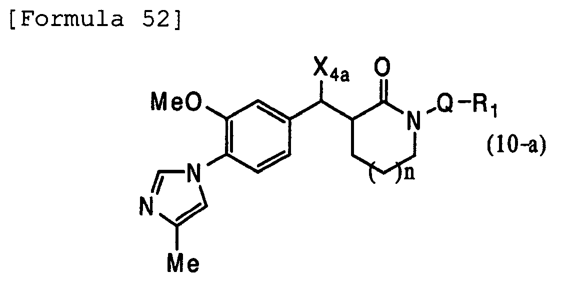 wherein Q, R₁ and n are as defined in claim 1 and X₄ₐ is as defined in claim 11, or a salt thereof, or a hydrate thereof.

20. A compound (10-b) represented by the formula (10-b): 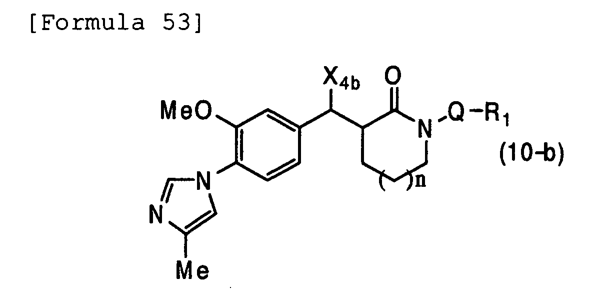 wherein Q, R₁ and n are as defined in claim 1 and X_{4b} is as defined in claim 16, or a salt thereof, or a hydrate thereof.

21. A process for producing a compound (8) represented by the formula (8): 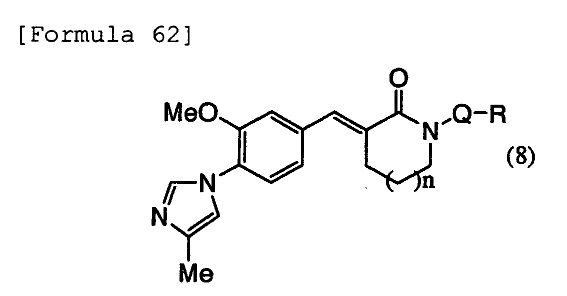 wherein Q, R and n are as defined in claim 1, the process comprising the steps of:
reacting, in the presence of a base, a compound (3) represented by the formula (3): 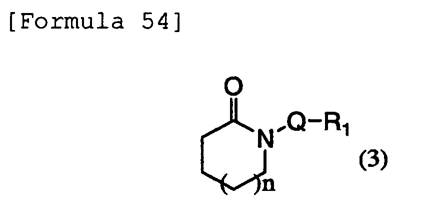 wherein R₁ is as defined in claim 1 and Q and n are as defined above, with a compound (20) represented by the formula (20): 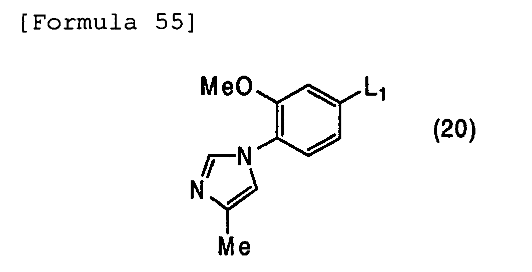 wherein L₁ represents an ester group or -CO-NR₂₀ₐ(-OR₂₀ₐ) (wherein R₂₀ₐ represents a C1-6 alkyl group), to convert the compound (3) into a compound (21) represented by the formula (21): 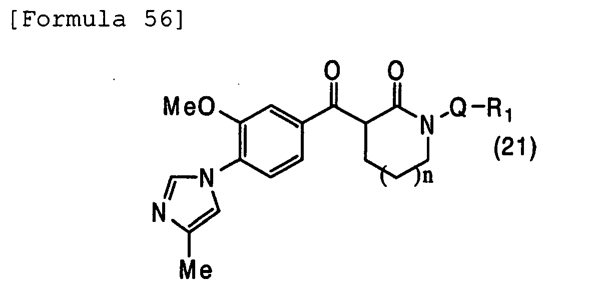 wherein Q, R₁ and n are as defined above;
treating the compound (21) represented by the formula (21): 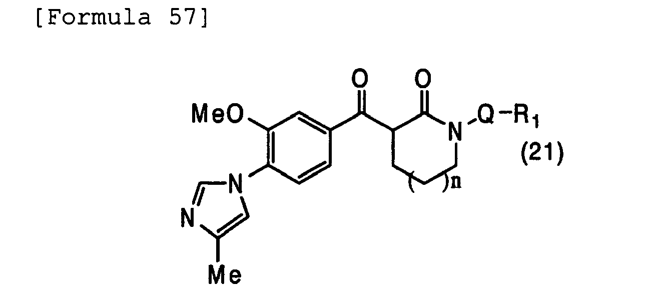 wherein Q, R₁ and n are as defined above, with a reducing agent to convert the compound (21) into a compound (9) represented by the formula (9): 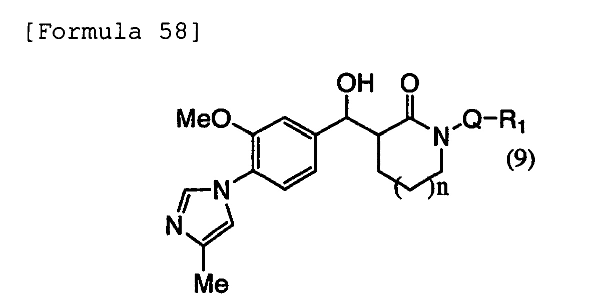 wherein Q, R₁ and n are as defined above;
reacting the compound (9) represented by the formula (9): 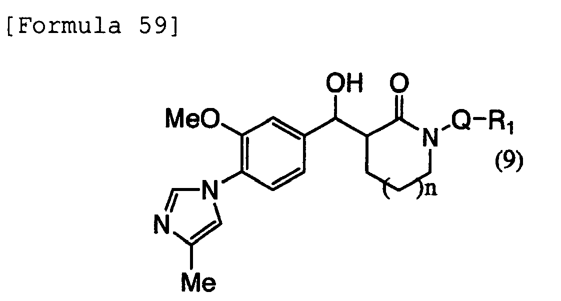 wherein Q, R₁ and n are as defined above, with a leaving group introduction reagent in the presence of a base if necessary to convert the compound (9) into a compound (10) represented by the formula (10): 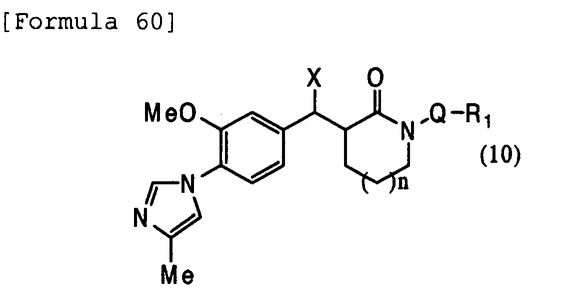 wherein X represents a leaving group and Q, R₁ and n are as defined above; and
treating the compound represented by the formula (10): 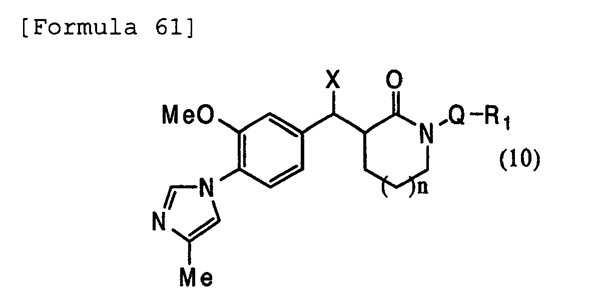 wherein Q, R₁, X and n are as defined above, with a base and removing a protecting group when R₁ has the protecting group.

22. A compound (21) represented by the formula (21): 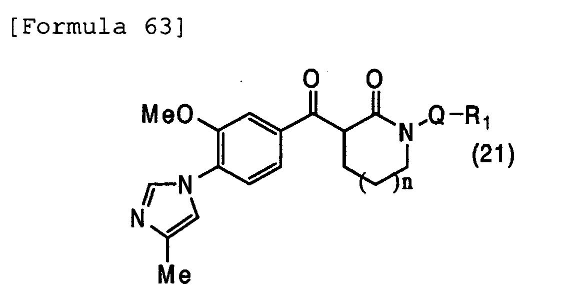 wherein Q, R₁ and n are as defined above, or a salt thereof, or a hydrate thereof.

23. A compound (20-a) represented by the formula (20-a): 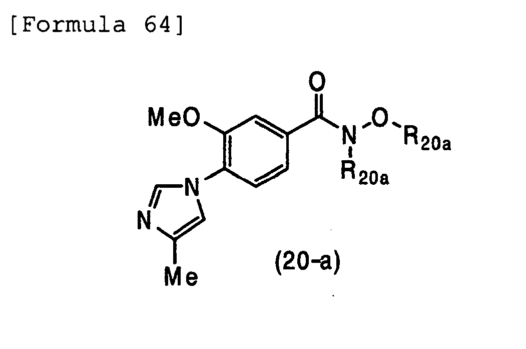 wherein R₂₀ₐ represents a C1-6 alkyl group, or a salt thereof, or a hydrate thereof.

24. A process for producing a compound (8) represented by the formula (8): 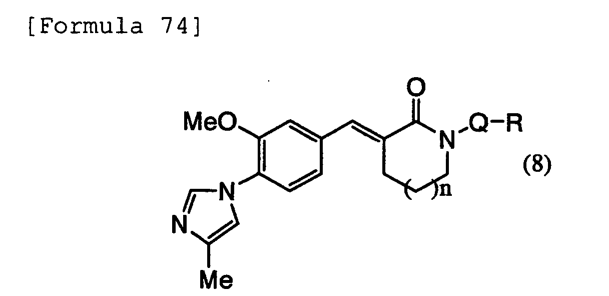 wherein Q, R and n are as defined in claim 1, the process comprising the steps of:
reacting, in the presence of a reducing agent, a compound (11) represented by the formula (11): 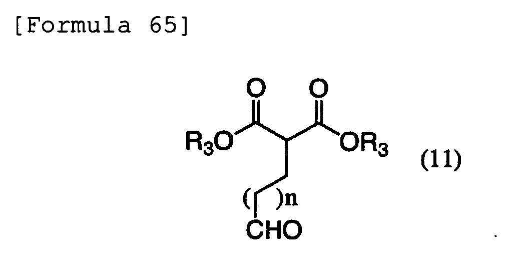 wherein R₃ is a protecting group for a carboxyl group and n is as defined above, with a compound (1) represented by the formula (1):
[Formula 66] R₁-Q-NH₂ (1)
wherein R₁ is as defined in claim 1 and Q is as defined above, to convert the compound (11) into a compound (12) represented by the formula (12): 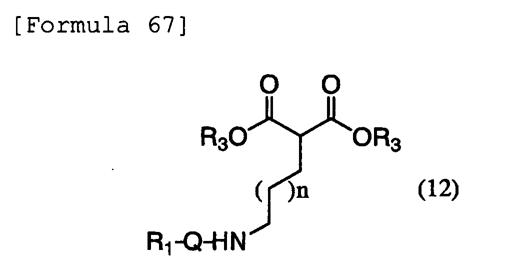 wherein Q, R₁, R₃ and n are as defined above;
heating, in the coexistence of an acid, the compound (12) represented by the formula (12): 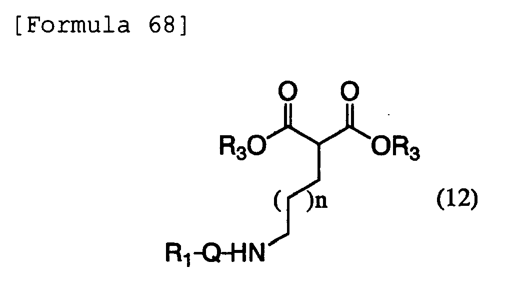 wherein Q, R₁, R₃ and n are as defined above, to convert the compound (12) into a compound (13) represented by the formula (13): 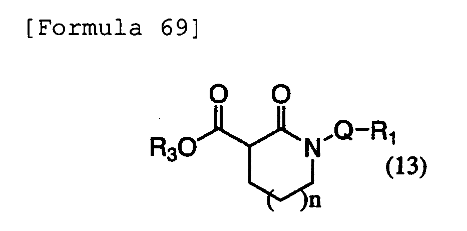 wherein Q, R₁, R₃ and n are as defined above;
hydrolyzing, in the presence of a base, the compound (13) represented by the formula (13): 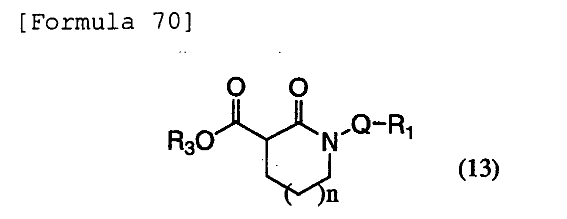 wherein Q, R₁, R₃ and n are as defined above, to convert the compound (13) into a compound (14) represented by the formula (14): 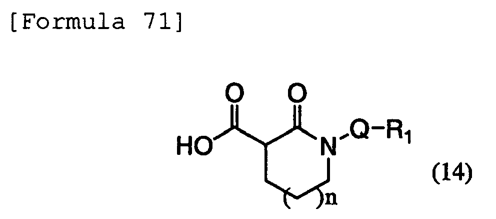 wherein Q, R₁ and n are as defined above; and
reacting, in the presence of a base, the compound (14) represented by the formula (14): 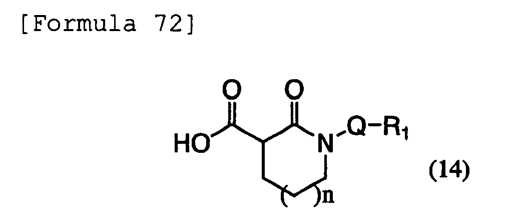 wherein Q, R₁ and n are as defined above, with 3-methoxy-4-(4-methyl-1*H*-imidazol-1-yl)benzaldehyde represented by the formula (7): 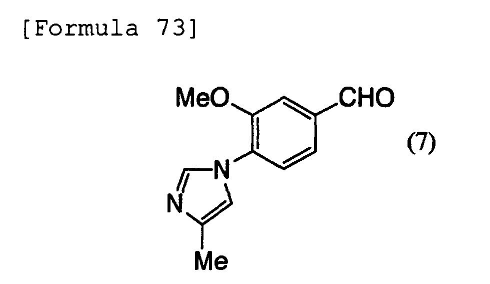 and removing a protecting group when R₁ has the protecting group.

25. A process for producing a compound (8) represented by the formula (8): 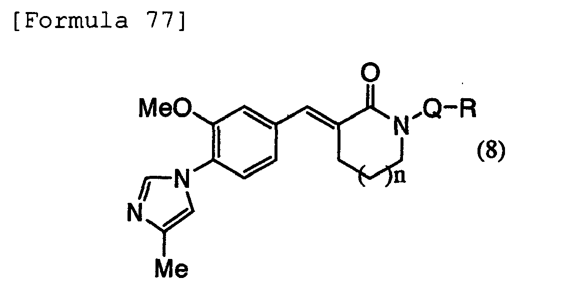 wherein Q, R and n are as defined in claim 1, the process comprising the steps of:
reacting, in the presence of a base, a compound (14) represented by the formula (14): 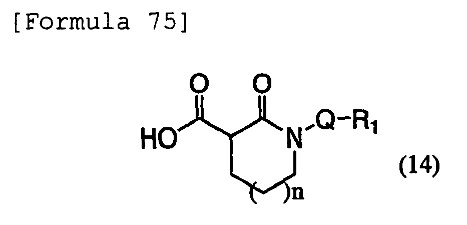 wherein R₁ is as defined in claim 1 and Q and n are as defined above, with 3-methoxy-4-(4-methyl-1H-imidazol-1-yl)benzaldehyde represented by the formula (7): 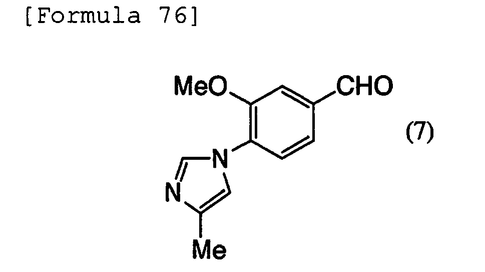 and removing a protecting group when R₁ has the protecting group.

26. A process for producing a compound (14) represented by the formula (14): 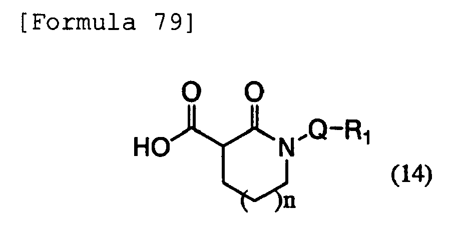 wherein Q, R₁ and n are as defined in claim 1, the process comprising:
hydrolyzing, in the presence of a base, a compound (13) represented by the formula (13): 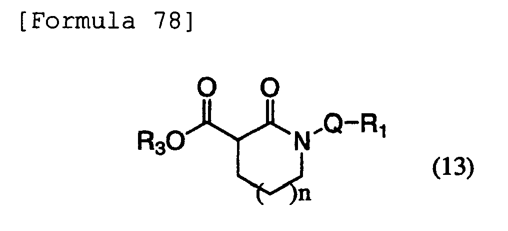 wherein R₃ is as defined in claim 1 and Q, R₁ and n are as defined above.

27. A process for producing a compound (13) represented by the formula (13): 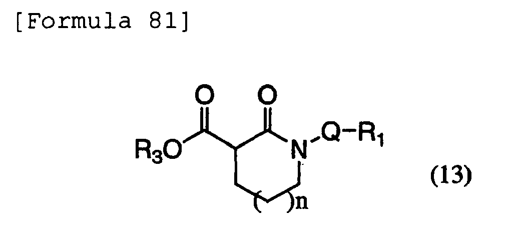 wherein Q, R₁ and n are as defined in claim 1 and R₃ is as defined in claim 24, the process comprising:
heating, in the coexistence of an acid, a compound (12) represented by the formula (12): 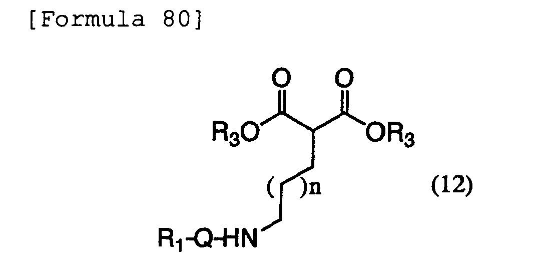 wherein Q, R₁, R₃ and n are as defined above.

28. A process for producing a compound (12) represented by the formula (12): 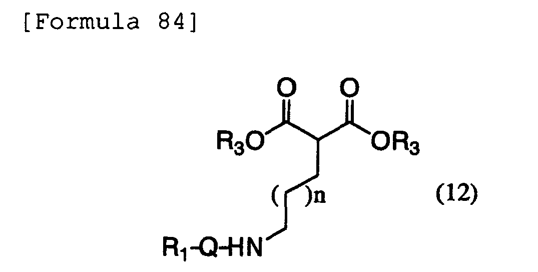 wherein Q, R₁ and n are as defined in claim 1 and R₃ is as defined in claim 24, the process comprising:
reacting, in the presence of a reducing agent, a compound (11) represented by the formula (11): 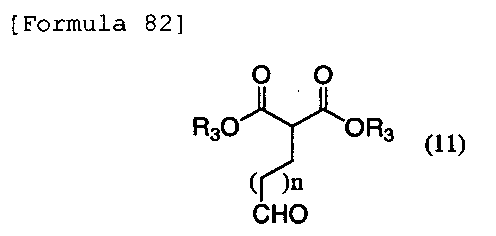 wherein R₃ and n are as defined above, with a compound (1) represented by the formula (1):
[Formula 83] R₁-Q-NH₂ (1)
wherein Q and R₁ are as defined above.

29. A compound (18) represented by the formula (18): 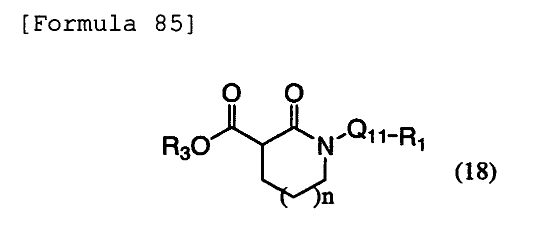 wherein R₁ and n are as defined in claim 1, Q₁₁ is as defined in claim 7 and R₃ is as defined in claim 24, or a salt thereof, or a hydrate thereof.

30. A compound (19) represented by the formula (19): 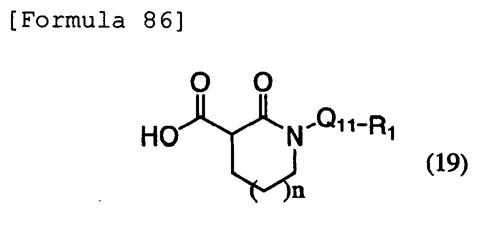 wherein R₁ and n are as defined in claim 1 and Q₁₁ is as defined in claim 7, or a salt thereof, or a hydrate thereof.
